(19) Europäisches Patentamt
European Patent Office
Office européen des brevets

(11) Publication number: **0 352 538 B1**

(12) **EUROPEAN PATENT SPECIFICATION**

(45) Date of publication of patent specification: **01.12.93**

(51) Int. Cl.5: **C07K 9/00**, C07K 1/00, A61K 37/02, //C12P21/04, (C12P21/04,C12R1:045)

(21) Application number: **89112608.8**

(22) Date of filing: **10.07.89**

(54) **Amides of N15-alkyl and N15,N15-dialkyl teicoplanin derivatives.**

(30) Priority: **26.07.88 GB 8817736**

(43) Date of publication of application:
**31.01.90 Bulletin 90/05**

(45) Publication of the grant of the patent:
**01.12.93 Bulletin 93/48**

(84) Designated Contracting States:
**AT BE CH DE ES FR GB GR IT LI LU NL SE**

(56) References cited:
**EP-A- 0 218 099**

(73) Proprietor: **GRUPPO LEPETIT S.p.A.**
**Via Roberto Lepetit, 8**
**I-20020 Lainate (MI)(IT)**

(72) Inventor: **Malabarba, Adriano**
**5/A, Via Roma**
**I-20082 Binasco (Milano)(IT)**
Inventor: **Trani, Aldo**
**11, Via Longhi**
**I-20137 Milano(IT)**
Inventor: **Kettenring, Jürgen Kurt**
**27, Via Oronco**
**I-21100 Varese(IT)**

(74) Representative: **Macchetta, Francesco et al**
**GRUPPO LEPETIT S.p.A.**
**Patent and Trademark Department**
**Via Roberto Lepetit, 34**
**I-21040 Gerenzano (Varese) (IT)**

EP 0 352 538 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention is directed to amides of $N^{15}$ alkyl and $N^{15},N^{15}$-dialkyl derivatives of teicoplanin compounds having the following formula I

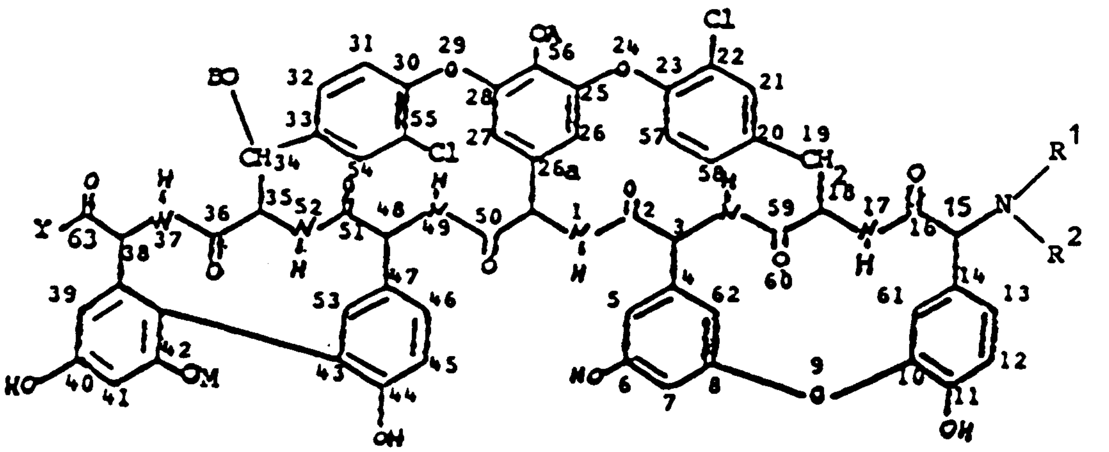

I

wherein:

$R^1$ is H or a $(C_1-C_3)$alkyl

$R^2$ represents a group $[CHR^3(CR^4R^5)_mX]_p-(CH_2)_n-R^6$

wherein:

$R^3$ and $R^4$ independently represent H or a $(C_1-C_6)$alkyl;

$R^5$ represents H, a $(C_1-C_6)$alkyl or OH;

$R^6$ represents H, a $(C_1-C_3)$alkyl, $COOR^7$, $OR^7$, $SR^7$, $NR^7R^8$ or halogen;

$R^7$ and $R^8$ independently represent H or a $(C_1-C_3)$alkyl;

m is zero or 1, n is an integer comprised between zero and 6, p is an integer comprised between 1 and 6, extremes included.

X is O, NH or a bond with the proviso that when X is O or NH, then n is different from zero, p ranges between 1 and 3, and $R^5$ is different from OH

Y represents a group

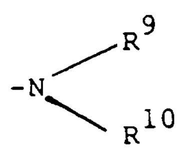

wherein:

$R^9$ represents H or a $(C_1-C_4)$alkyl; hydroxy$(C_2-C_4)$alkyl; amino$(C_2-C_4)$alkyl;$(C_1-C_3)$alkylamino$(C_2-C_4)$-alkyl;

$R^{10}$ represents H, $(C_1-C_6)$alkyl, hydroxy$(C_2-C_4)$alkyl, halogen$(C_2-C_4)$alkyl, carboxy$(C_1-C_3)$alkyl, carboxamino$(C_1-C_3)$alkyl, $(C_1-C_3)$alkylcarboxy$(C_1-C_3)$-alkylamido, $(C_1-C_3)$alkoxycarbonyl$(C_1-C_3)$-alkyl, phenyl$(C_1-C_3)$alkoxycarbonyl$(C_1-C_3)$alkyl, a group

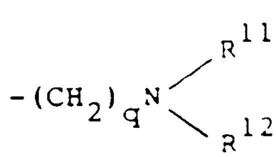

or a group

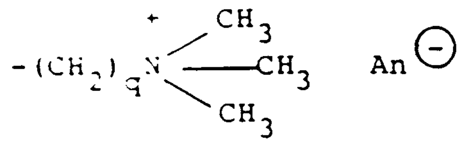

in which q is an integer ranging from 2 to 8, and one of the hydrogen atoms of the methylene unit is optionally substituted with a substituent selected from $(C_1-C_3)$alkyl, carboxy, $(C_1-C_4)$-alkoxycarbonyl, and carboxamido and $An^{\ominus}$ is a pharmaceutically acceptable anion;

$R^{11}$ represents H or a $(C_1-C_4)$alkyl, hydroxy$(C_2-C_4)$alkyl, halogen$(C_2-C_4)$alkyl,

$R^{12}$ represents H, $(C_1-C_6)$alkyl, hydroxy$(C_2-C_4)$alkyl, halogen$(C_2-C_4)$alkyl, $(C_1-C_3)$alkoxy$(C_1-C_4)$alkyl, phenyl$(C_1-C_4)$alkoxycarbonyl, $(C_1-C_4)$alkylcarbonyl

or $R^{11}$ and $R^{12}$ taken together with the adjacent nitrogen atom represent a saturated 5-7 membered heterocyclic ring which may optionally bear one to two $(C_1-C_4)$alkyl substituents on the ring carbons and may contain a further heterogroup selected from -O-, -S-, and $-NR^{13}$-wherein $R^{13}$ is H or a $(C_1-C_4)$alkyl

or $R^9$ and $R^{10}$ taken together with the adjacent nitrogen atom represent a saturated 5-7 membered heterocyclic ring which may optionally bear one to two $(C_1-C_4)$alkyl substituents on the ring carbons and may contain a further heterogroup selected from -O-, -S-, and $-NR^{14}$-wherein $R^{14}$ is H or a $(C_1-C_4)$alkyl

A represents hydrogen or $-N[(C_9-C_{12})$aliphatic acyl]-beta-D-2-deoxy-2-amino-glucopyranosyl,

B represents hydrogen or N-acetyl-beta-D-2-deoxy-2-amino-glucopyranosyl,

M represents hydrogen or alpha-D-mannopyranosyl;

with the proviso that B represents hydrogen only when A and M are simultaneously hydrogen and the addition salts thereof.

Teicoplanin is the international non-proprietary name (INN) of the antibiotic substance formerly named teichomycin which is obtained by cultivating the strain Actinoplanes teichomyceticus nov. sp. ATCC 31121 in a culture medium containing assimilable sources of carbon, nitrogen and inorganic salts (see U.S. Patent No. 4,239,751). According to the procedure described in the above cited patent an antibiotic complex containing Teichomycin $A_1$, $A_2$ and $A_3$ is recovered from the separated fermentation broth by extraction with a suitable water insoluble organic solvent and precipitation from the extracting solvent according to common procedures. Teichomycin $A_2$, which is the major factor of the isolated antibiotic complex, is then separated from the other factors by means of column chromatography on Sephadex$^R$. British Patent No. 2121401 discloses that antibiotic Teichomycin $A_2$ actually is a mixture of five closely related co-produced main components.

It is possible to represent teicoplanin $A_2$ (formerly Teichomycin $A_2$) main components 1, 2, 3, 4 and 5 by the above formula I wherein R is hydrogen, Y is hydroxy, A represents $-N[(C_{10}-C_{11})$aliphatic acyl]-beta-D-2-deoxy-2-amino-glucopyranosyl, B represents N-acetyl-beta-D-2--deoxy-2-amino-glucopyranosyl, M represents alpha-D-mannopyranosyl.

More particularly, in teicoplanin $A_2$ component 1, the $[(C_{10}-C_{11})$-aliphatic acyl] substituent represents Z-decenoyl, in teicoplanin $A_2$ component 2 represents 8-methyl-nonanoyl, in teicoplanin $A_2$ component 3 represents decanoyl, in teicoplanin $A_2$ component 4 represents 8-methyldecanoyl, in teicoplanin $A_2$ component 5 represents 9-methyldecanoyl.

European Patent Application Ser. No. 88110194.3 describes production of teicoplanin components where the aliphatic acyl group of the beta-D-2-deoxy-2-aminoglucopyranosyl moiety is a n-nonanoyl (compound B or RS3) or a 6-methyl octanoyl group (compound A or RS4).

In the paper entitled: "Isolation by HPLC and structural determination of minor components of teicoplanin" given by Zanol et al., at the 17th International Symposium on chromatography, Wien, September 25-30, 1988, other two teicoplanin compounds (RS1 and RS2) are described.

Said compounds are characterized in that the aliphatic acyl moieties of the beta-D-2-deoxy-2-aminoglucopyranosyl moiety are respectively methyl-undecanoyl and dodecanoyl.

All the sugar moieties, when present, are linked to the teicoplanin nucleus through O-glycosidic bonds.

In addition, it has been found that it is possible to transform teicoplanin, a pure factor thereof or a mixture of any of said factors in any proportion, into unitary antibiotic products by means of selective hydrolysis of one or two sugar moieties. They are named antibiotic L 17054 and antibiotic L 17046 and are described in European Patent Application Publication No. 119575 and European Patent Application Publication No. 119574, respectively.

Preferred hydrolysis conditions for the production of antibiotic L 17054 are: 0.5 N hydrochloric acid at a temperature between 70 °C and 90 °C and for a time which is generally between 15 and 90 min.

Antibiotic L 17054 is represented by the above formula I wherein Y is hydroxy, R and A represent hydrogen,
B represents N-acetyl-beta-D-2-deoxy-2-amino-glucopyranosyl,
M represents alpha-D-mannopyranosyl wherein the sugar moieties are linked to the peptidic nucleus through an O-glycosidic bond.
Preferred hydrolysis conditions for the preparation of antibiotics L 17046 are: 1-3 N hydrochloric acid, at a temperature between 50° and 90°C and for a time which is generally between 30 and 60 min.

Antibiotic L 17046 is represented by the above formula I wherein Y is hydroxy, R, A and M represent hydrogen atoms, and B is N-acetyl-beta-D-2-deoxy-2-amino-glucopyranosyl wherein the sugar moiety is linked to the peptidic nucleus through an O-glycosidic bond.

European Patent Application Publication No. 301247 describes de-mannosyl teicoplanin derivatives, i.e. compounds of the formula I above wherein A and B are different from hydrogen and M is hydrogen.

The complete selective cleavage of all the sugar moieties of the teicoplanin compounds gives an aglycone molecule which is called antibiotic L 17392, or deglucoteicoplanin, and is represented by the above formula I wherein Y is hydroxy, and R, A, B, and M each individually represents a hydrogen atom. This selective hydrolysis process is described in European Patent Application Publication No. 146053.

A substance having the same structural formula is disclosed in European Patent Application Publication No. 0090578 and is named antibiotic A 41030 factor B.

This substance is obtained by means of microbiological process which involves the fermentation of the strain Streptomyces virginiae NRRL 12525 or Streptomyces virginiae NRRL 15156 in a suitable medium, the isolation, purification and separation into its components of antibiotic A 41030, an antibiotic complex of at least seven factors, antibiotic A 41030 factor B, included.

All the above named compounds, i.e. teicoplanin, teicoplanin $A_2$ complex, teicoplanin $A_2$ component 1, teicoplanin $A_2$ component 2, teicoplanin $A_2$ component 3, teicoplanin $A_2$ component 4, teicoplanin $A_2$ component 5, antibiotic L 17054, antibiotic L 17046, antibiotic L 17392, compound A or RS4, compound B or RS3, the de-mannosyl derivatives of European Patent Application Publication No. 301247 and any mixture thereof in any proportion, are suitable starting materials for the preparation of the amide derivatives of the invention.

In the present specification "teicoplanin compound" or "teicoplanin starting material" is used to indicate any one of the above starting materials, i.e. teicoplanin as obtained according to U.S. patent 4,239,751, any further purification thereof, teicoplanin $A_2$ complex, a compound of the above formula I wherein R is hydrogen, Y is hydroxy, A represents hydrogen or
-N[($C_9$-$C_{12}$)aliphatic acyl]-beta-D-2-deoxy-2-amino-glucopyranosyl, B represents hydrogen or N-acetyl-beta-D-2-deoxy-2-amino-glucopyranosyl,
M represents hydrogen or alpha-D-mannopyranosyl, with the proviso that B may represent hydrogen only when A and M are simultaneously hydrogen, a salt thereof, or a mixture thereof in any proportion.

As used herein the term "alkyl", either alone or in combination with other substituents, includes both straight and branched hydrocarbon groups; more particularly, "($C_1$-$C_6$)alkyl" represents a straight or branched aliphatic hydrocarbon chain of 1 to 6 carbon atoms such as methyl, ethyl, propyl, 1-methylethyl, butyl, 1-methylpropyl, 1,1-dimethylethyl, pentyl, 1-methylbutyl, 2-methylbutyl, 1-hexanyl, 2-hexanyl, 3-hexanyl, 3,3-dimethyl-1-butyl, 4-methyl-1-pentyl and 3-methyl-1-pentyl;
likewise, "($C_1$-$C_4$)alkyl" represents a straight or branched hydrocarbon chain of 1 to 4 carbon atoms such as those alkyl of 1 to 4 carbons exemplified above.

The term "halogen" represents an halogen atom selected from fluorine, chlorine, bromine an iodine.

The expression "a saturated 5-7 membered heterocyclic ring which may optionally bear one to two ($C_1$-$C_4$)alkyl substituents on the ring carbons and may optionally contain a further heterogroup selected form -O-, -S- and -$NR^{13}$ (or-$NR^{14}$)" include, for instance, the following heterocyclic groups: morpholinyl, piperidinyl, piperazinyl, thiomorpholinyl, pyrazolidinyl, 1,3-oxazolidinyl, 1,3-thiazolidinyl and hexahydroazepinyl, which may optionally be substituted by one or two ($C_1$-$C_4$)alkyl group on the carbon skeleton.

A preferred group of compounds of the invention is represented by those compounds of formula I wherein $R^1$ represents a hydrogen atom or a methyl and the other substituents are as defined above.

A further preferred group of compounds of the invention is represented by those compounds of formula I wherein:

$R^1$ is H or a ($C_1$-$C_3$)alkyl;

$R^2$ represents a group $[CHR^3(CR^4R^5{}_mX]_p$-$(CH_2)_n$-$R^6$

wherein:

$R^3$ and $R^4$ independently represent H or a ($C_1$-$C_4$)alkyl;

$R^5$ represents H, a $(C_1\text{-}C_4)$alkyl or OH;

$R^6$ represents H, a $(C_1\text{-}C_3)$alkyl, $OR^7$, or $NR^7R^8$;

$R^7$ and $R^8$ independently represent H or a $(C_1\text{-}C_3)$alkyl,

m is zero or 1, n is an integer comprised between zero and 3, p is an integer comprised between 1 and 3

X is O, NH or a bond with the proviso that when X is O or NH, then n is different from zero, p is 1 or 2, and $R^5$ is different from OH

Y represents a group

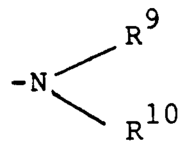

wherein:

$R^9$ represents H or a $(C_1\text{-}C_4)$alkyl

$R^{10}$ represents H, $(C_1\text{-}C_4)$alkyl, hydroxy$(C_2\text{-}C_4)$alkyl or a group

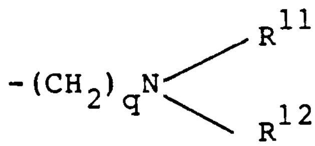

in which q is an integer ranging from 2 to 8, and one of the hydrogen atoms of the methylene unit is optionally substituted with a substituent selected from carboxy, and $(C_1\text{-}C_3)$-alkoxycarbonyl,

$R^{11}$ represents H or a $(C_1\text{-}C_4)$alkyl

$R^{12}$ represents H, $(C_1\text{-}C_6)$alkyl, hydroxy$(C_2\text{-}C_4)$alkyl, $(C_1\text{-}C_3)$alkoxy$(C_1\text{-}C_4)$alkyl, phenyl$(C_1\text{-}C_4)$-alkoxycarbonyl, $(C_1\text{-}C_4)$alkyloxycarbonyl

or $R^9$ and $R^{10}$ taken together with the adjacent nitrogen atom represent a saturated 5-7 membered heterocyclic ring which may optionally bear one to two $(C_1\text{-}C_4)$alkyl substituents on the ring carbons and may contain a further heterogroup selected from -O-, -S-, and $-NR^{14}$-wherein $R^{14}$ is H or a $(C_1\text{-}C_4)$alkyl

A represents hydrogen or -N[$(C_9\text{-}C_{12})$aliphatic acyl]-beta-D-2-deoxy-2-amino-glucopyranosyl,

B represents hydrogen or N-acetyl-beta-D-2-deoxy-2-amino-glucopyranosyl,

M represents hydrogen or alpha-D-mannopyranosyl;

with the proviso that B represents hydrogen only when A and M are simultaneously hydrogen and the addition salts thereof.

Another further preferred group of compounds of the invention is represented by those compounds of formula I wherein:

$R^1$ is H or $CH_3$, $R^2$ represents a group as defined wherein $R^3$ is H or $CH_3$, $R^4$ is H or $CH_3$; $R^5$ is H or OH; m is zero or 1, n ranges from 0 to 2, p is 1 or 2, $R^6$ is H, $OR^7$ or $NR^7R^8$ and $R^7$ and $R^8$ are independently H or $CH_3$

Y represents a group

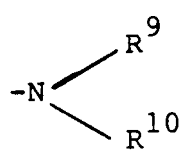

wherein:

$R^9$ represents H or a $(C_1\text{-}C_4)$alkyl

$R^{10}$ represents H, $(C_1\text{-}C_4)$alkyl, hydroxy$(C_2\text{-}C_4)$alkyl or a group

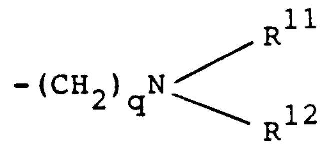

in which q is an integer ranging from 2 to 8, and one of the hydrogen atoms of the methylene unit is optionally substituted with a substituent selected from carboxy, and $(C_1-C_3)$-alkoxycarbonyl,

$R^{11}$ represents H or a $(C_1-C_4)$alkyl

$R^{12}$ represents H, $(C_1-C_6)$alkyl, hydroxy$(C_2-C_4)$alkyl, halogeno$(C_2-C_4)$alkyl, $(C_1-C_3)$alkoxy$(C_1-C_4)$alkyl, phenyl$(C_1-C_4)$alkoxycarbonyl, $(C_1-C_4)$alkyloxycarbonyl

or $R^{11}$ and $R^{12}$ taken together with the adjacent nitrogen atom represent a saturated 5-7 membered heterocyclic ring which may optionally bear one to two $(C_1-C_4)$alkyl substituents on the ring carbons and may contain a further heterogroup selected from -O-, -S-, and $-NR^{13}$-wherein $R^{13}$ is H or a $(C_1-C_4)$alkyl

or $R^9$ and $R^{10}$ taken together with the adjacent nitrogen atom represent a saturated 5-7 membered heterocyclic ring which may optionally bear one to two $(C_1-C_4)$alkyl substituents on the ring carbons and may contain a further heterogroup selected from -O-, -S-, and $-NR^{14}$-wherein $R^{14}$ is H or a $(C_1-C_4)$alkyl

A represents hydrogen or $-N[(C_9-C_{12})$aliphatic acyl]-beta-D-2-deoxy-2-amino-glucopyranosyl,

B represents hydrogen or N-acetyl-beta-D-2-deoxy-2-amino-glucopyranosyl,

M represents hydrogen or alpha-D-mannopyranosyl;

with the proviso that B represents hydrogen only when A and M are simultaneously hydrogen and the addition salts thereof.

Another group of preferred compounds of the invention is represented by those compounds of formula I wherein:

$R^1$ is H or a $(C_1-C_3)$alkyl,

$R^2$ represents a group $[CHR^3(CR^4R^5{}_mX]_p-(CH_2)_n-R^6$

wherein:

$R^3$ and $R^4$ independently represent H or a $(C_1-C_6)$alkyl

$R^5$ represents H, a $(C_1-C_6)$alkyl or OH

$R^6$ represents H, a $(C_1-C_3)$alkyl, $COOR^7$, $OR^7$, $SR^7$, $NR^7R^8$ or halogen

$R^7$ and $R^8$ independently represent H or a $(C_1-C_3)$alkyl,

m is zero or 1, n is an integer comprised between zero and 6, p is an integer comprised between 1 and 6

X is O, NH or a bond with the proviso that when X is O or NH, then n is different from zero, p ranges between 1 and 3, and $R^5$ is different from OH

Y represents a group selected from:

$-NH_2$, $-NHC_4H_9$, $-NH(CH_2)_4-OH$, $-NH(CH_2)_3NH(CH_2)_2-OH$,
$-NHCH_2COOH$, $-NHCH_2COOCH_2C_6H_5$, $-NHCH_2COOC_2H_5$,
$-NH-CH_2CONH_2$, $-NH-CH_2-CON(C_2H_5)_2$,
$-NH-CH-(COOCH_3)-(CH_2)_4NHCOOCH_2C_6H_5$,
$-NH-CH-(COOCH_3)-(CH_2)_4NH_2$, $-NH-CH-(CONH_2)CH_2CONH_2$,
$-NH-(CH_2)_q-NH_2$, $-NH-(CH_{2q}NHCH_3$, $-NH(CH_2)_q-N(CH_3)_2$,
$-NH-(CH_2)_qN(C_2H_5)_2$, $-HN(CH_2)_qN(CH_3)(C_2H_5)$, $-NH(CH_2)_qN(C_4H_9)_2$
wherein q represents 2, 3, 4, 5, 6, 7 or 8
$-NH-(CH_2)_2N(C_2H_4OH)(C_2H_4Cl)$;
$-NH(CH_2)_2N[(CH_2)_4OH]_2$, $-NH(CH_2)_4N(C_2H_4Cl)_2$,
$-NH-(CH_2)_3N(C_4H_9)_2$,

$$-NH-(CH_2)_3\overset{\oplus}{N}(CH_3)_3\ An^{\ominus},$$

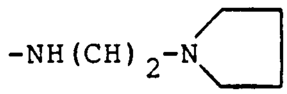 , 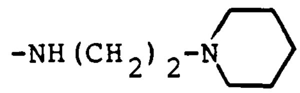 , 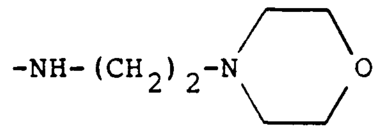

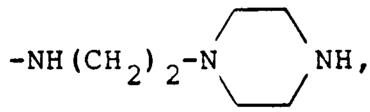 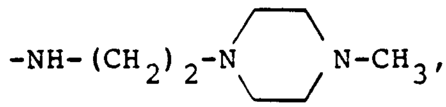

$-NH-CH_2CH(CH_3)CH_2-N(C_2H_5)_2$

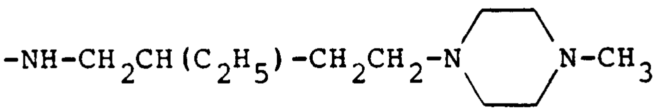

$-N(CH_3)_2$, $N(CH_2CH_2OH)_2$, $-N(CH_2CH_2NH_2)_2$, $-N(CH_2CH_2NHCH_3)_2$,
$-N[CH_2CH_2N(CH_3)_2]_2$, $-N(CH_3)(CH_2CH_2NH_2)$,
$-N(CH_3)[(CH_2)NHCH_3]$, $-N(CH_3)[(CH_2)_2N(CH_3)_2]$,
$-N(CH_3)[(CH_2)_3NH(CH_3)]$,
$-N(C_2H_5)[(CH_2)_2-NHCH_3]$, $-N[CH_2CH_2CH_2N(C_2H_5)_2]_2$,

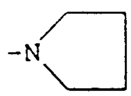 , 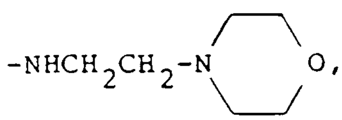 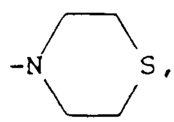 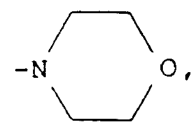

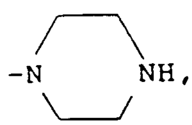 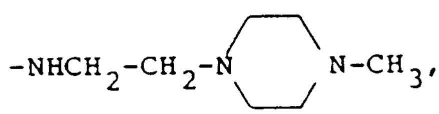 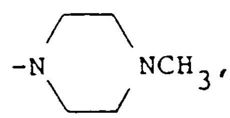

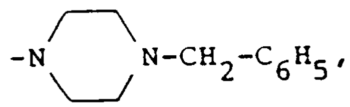 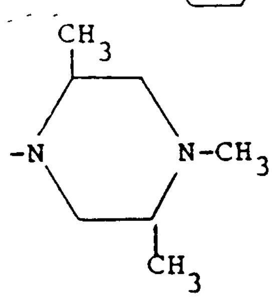

The compounds of the invention can form salts according to conventional procedures.

In particular, those compounds of formula I wherein $R^1$ represents hydrogen as well as those compounds of formula I wherein the group $-NR^9R^{10}$ contains further amine functions from acid addition salts.

In addition, those compounds of the invention which contain acid functions in the $-NR^9R^{10}$ moiety may also form base addition salts.

In general, those compounds of the invention which contain acid and basic functions can form internal salts. For the scope of the present invention the "internal salts" are encompassed by the definition of the "non-salt" form.

Preferred addition salts of the compounds of this invention are the pharmaceutically acceptable acid and/or base addition salts.

With the term "pharmaceutically acceptable acid and/or base addition salts" are intended those salts with

acids and/or bases which from biological, manufacturing and formulation standpoint are compatible with the pharmaceutical practice as well as with the use in the animal growth promotion.
Representative and suitable acid addition salts of the compounds of formula I include those salts formed by standard reaction with both organic and inorganic acids such as, for example, hydrochloric, hydrobromic, sulfuric, phosphoric, acetic, trifluoroacetic, trichloroacetic, succinic, citric, ascorbic, lactic, maleic, fumaric, palmitic, cholic, pamoic, mucic, glutamic, camphoric, glutaric, glycolic, phthalic, tartaric, lauric, stearic, salicylic, methanesulfonic, benzenesulfonic, sorbic, picric, benzoic, cinnamic and the like acids.
Representative examples of these bases are:
alkali metal or alkaline-earth metal hydroxide such sodium, potassium, and calcium hydroxide; ammonia and organic aliphatic, alicyclic or aromatic amines such as methylamine, dimethylamine, trimethylamine, and picoline.

The transformation of the free amino or non-salt compounds of the invention into the corresponding addition salts, and the reverse, i.e. the transformation of an addition salt of a compound of the invention into the non-salt or free amino form, are within the ordinary technical skill and are encompassed by the present invention.
For instance, a compound of formula I can be transformed into the corresponding acid or base addition-salt by dissolving the non-salt form in an aqueous solvent and adding a slight molar excess of the selected acid or base. The resulting solution or suspension is then lyophilized to recover the desired salt. Instead of lyophilizing, in some instances, it is possible to recover the final salt by extraction with an organic solvent, concentration
to a small volume of the separated organic phase and precipitation by adding a non-solvent.
In case the final salt is unsoluble in an organic solvent where the non-salt form is soluble it is recovered by filtration from the organic solution of the non-salt form after addition of the stoichiometric amount or a slight molar excess of the selected acid or base.
The non-salt form can be prepared from a corresponding acid or base salt dissolved in an aqueous solvent which is then neutralized to free the non-salt form. This is then recovered for instance by extraction with an organic solvent or is transformed into another base or acid addition salt by adding the selected acid or base and working up as above.
When following the neutralization desalting is necessary, a common desalting procedure may be employed.
For example, column chromatography on controlled pore polydextrane resins (such as Sephadex L H 20) or silanized silica gel may be conveniently used. After eluting the undesired salts with an aqueous solution, the desired product is eluted by means of linear gradient or step-gradient of a mixture of water and a polar or apolar organic solvent, such as acetonitrile/water from 50:50 to about 100% acetonitrile.

As is known in the art, the salt formation either with pharmaceutically acceptable acids (bases) or non-pharmaceutically acceptable acids (bases) may be used as a convenient purification technique. After formation and isolation, the salt form of a compound of formula I can be transformed into the corresponding non-salt or into a pharmaceutically acceptable salt.

In some instances the acid addition salt of a compound of formula I is more soluble in water and hydrophilic solvents and has an increased chemical stability.

However, in view of the similarity of the properties of the compounds of formula I and their salts, what is said in the present application when dealing with the biological activities of the compounds of formula I applies also to their pharmaceutically acceptable salts, and viceversa.

Amides of teicoplanin compounds are described in European Patent Application Publication No. 218099.

In particular, this application describes $N^{63}$carboxyamides of formula I wherein Y has the meanings reported above and the groups linked to the $N^{15}$ atom are either two hydrogen atoms or a hydrogen atom and a protecting group of the amino function.

These $N^{63}$carboxyamides shown an interesting degree of activity as semi-synthetic antibacterial agents mainly active against gram positive bacteria.

The compounds of the invention, which in addition to the amidic substituent (Y in formula I) have also alkylic substituents at the $N^{15}$amino function ($R^1$ and $R^2$ in formula I) possess a higher water solubility and consequently allow an easier pharmaceutical formulation without substantially affecting the degree of activity of the antibiotic substances.

In particular, the compounds of the present invention, due to this improved water solubility, can easily be formulated as injectables and can be administered by intramuscular route at high concentrations thus using a smaller amount of vehicle without any local tolerability problem.

Due to the particular characteristics of the different final products there are many procedures which can be employed for preparing the compounds according to the present invention.

Some general procedures are described hereinbelow:

Procedure A: Reaction of a $N^{63}$amide derivative of a teicoplanin compound of formula I wherein Y is as defined and $R^1$ and $R^2$ are replaced by two hydrogen atoms, with an alkyl halide having formula (halo)$R^2$ wherein $R^2$ is as defined and (halo) represents an halogen atom, in a polar aprotic solvent, in the presence of a base in order to prepare the $N^{15}$ monoalkyl derivative of said teicoplanin amide.

Procedure B: Reductive alkylation of a $N^{63}$ amide derivative of a teicoplanin compound of formula I wherein Y is as defined and $R^1$ and $R^2$ are substituted by two hydrogen atoms with a carbonyl compound, in an organic polar solvent, in the presence of a suitable reducing agent in order to prepare the $N^{15}$monoalkyl derivative of said teicoplanin amide.

Procedure C: Reaction of a $N^{15}$alkyl (or $N^{15}$,$N^{15}$-di-alkyl) derivative of a teicoplanin compound of formula I wherein $R^1$ and $R^2$ are as defined, and Y is substituted by an -OH group, with a proper amine in a polar aprotic solvent in the presence of a condensing agent.

Procedure D: Reductive alkylation of the $N^{15}$monoalkyl derivative of teicoplanin amide of formula I wherein $R^2$ and Y are as defined, with a carbonyl compound in the presence of a suitable organic acid and a reducing agent in order to obtain the $N^{15}$,$N^{15}$dialkyl derivative of said teicoplanin amide.

Procedure E: Selective hydrolysis of the corresponding amide of $N^{15}$alkyl (or $N^{15}$,$N^{15}$di-alkyl) derivative of teicoplanin in order to remove (partially or totally) the sugar moieties A, B, and M stated above.

Procedure F: Catalytic hydrogenolysis of a compound of the invention wherein $R^{12}$ is a phenyl methyloxycarbonyl to give the corresponding compound wherein $R^{12}$ is H.

The starting material of the procedures A and B is a $N^{63}$ amide derived from a teicoplanin compound i.e. a compound of formula I wherein Y is as defined and $R^1$ and $R^2$ are substituted by two hydrogen atoms.

A general procedure for preparing said starting material is described in European Patent Application Publication No. 218099. According to this procedure "a teicoplanin starting material" as defined above is reacted with a proper amine $NHR^9R^{10}$ (in which $R^9$ and $R^{10}$ are as defined above) in an inert organic solvent in the presence of a condensing agent such as diphenylphosphorylazide (DPPA).

According to a preferred embodiment of procedure A of the present invention for preparing a $N^{15}$ mono-alkyl derivative of a $N^{63}$ teicoplanin amide, the proper amide used as starting material is reacted with an alkyl halide $R^2$(halo), wherein the alkyl portion is represented by an "alkyl" group which corresponds to the desired meaning of $R^2$ in the final product and the halide is preferably a chloride, bromide or iodide and most preferably bromide or chloride.

The reaction occurs at about room temperature in the presence of an organic or inorganic base of medium strength such as triethylamine, trimethylamine, tri-butylamine, sodium or potassium bicarbonate and the like.

Preferably, the reaction medium includes also an organic solvent capable of at least partially solubilizing the starting materials such as organic amides, alkyl ethers, ethers of glycols and polyols, phosphoramides, sulfoxides and aromatic compounds e.g., dimethylformamide, dimethoxyethane, hexamethylphosphoramide, dimethylsulfoxide, benzene, toluene and mixtures thereof.

This solvent should have a low water content (generally below about 5%).

The alkyl halide and the starting teicoplanin derivative are preferably employed in about equimolecular proportion or in a slight molar excess of the alkyl halide so that the mono-alkyl derivative is obtained in good yields.

The reaction occurs at about room temperature so that external heating is not in general necessary, even if the reaction can be carried out at temperatures of 40-70 °C.

A preferred embodiment of the procedure B for preparing a $N^{63}$ amide derivative of $N^{15}$ alkyl teicoplanin compound is a process which comprises:

a) adding to a suspension of an amide of teicoplanin compound (prepared e.g. as defined in European patent Application Publication No. 218099) in an organic polar protic solvent a suitable amount of an alkali metal borohydride at a temperature comprised between 0 °C and 30 °C, then

b) adding a molar excess of a carbonyl compound of at least 20 times with respect to teicoplanin starting material, and

c) reacting the resulting solution with a reducing agent, preferably a alkali metal borohydride.

From among the organic polar protic solvents lower alkanols having from 1 to 4 carbon atoms are preferred. In particular, methanol is the preferred solvent used.

Sometimes, in particular cases, a small amount of a polar aprotic co-solvent can be added to completely dissolve teicoplanin starting material during the course of the reaction, e.g. N, N-dimethylformamide, 1,3-dimethyl-3,4,5,6-tetrahydro 2(1H)-pyramidone (DMPU), dimethylsulfoxide.

The temperature at which the reaction is carried out is comprised between 0 °C and 30 °C, preferably it ranges from 10 °C to 15 °C.

It is important to use a large molar excess of carbonyl compound, generally from 20 to 100 times with respect to teicoplanin starting material. The carbonyl compound used has the same carbon skeleton of substituent $R^2$ and whose oxo group is on the carbon atom which links to the $N^{15}$-amino group of teicoplanin moiety.

For example, if

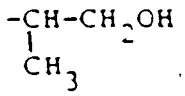

is the desired $N^{15}$ monoalkyl group $R^2$,
then

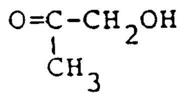

is the useful carbonyl compound which has to be employed as starting reactant.

Sodium borohydride is the preferred alkali metal borohydride used in step a).

As metal alkali borohydride used in the step c) the sodium borohydride is the preferred one, but also the potassium and the sodium cyano-borohydride can be used.

According to a preferred embodiment of this procedure B it is important to maintain the reactants addition order as indicated above in steps a), b) and c).

In a further preferred embodiment of the procedure B before adding the excess of carbonyl compound to the reaction mixture, the alkali metal borohydride is allowed to completely decompose under stirring. Generally it takes a time comprised between 30 minutes and 120 minutes.

In particular, the carbonyl compound can be preferably added when the reaction mixture is a clear solution.

The suitable amount of alkali metal borohydride added can be different depending on the particular amide used as the starting material, on the solvent used and on the temperature of the reaction, but it is advisable to add an amount of alkali metal borohydride such that the pH of the reaction mixture is alkaline, preferably between pH 8 and 10, as determined after diluting a sample of the non-aqueous reaction mixture with 4 volumes of water.

The reaction times of the process of this procedure B depend on the factors reported above such as the starting compound used, the solvent, the amount of alkali metal borohydride used and the like, and on the structure of reactant carbonyl compounds but, in general, it is preferred to leave the reaction mixture under stirring from 1 to 10 h after the addition of the reactants and before treating with the reducing agent.

The general procedure C for preparing a compound of the invention is represented by the reaction (amidation) of a suitable teicoplanin starting material wherein $R^1$ and $R^2$ are as above defined and Y is an OH group with the selected amine of formula $HNR^9R^{10}$ wherein $R^9$ and $R^{10}$ have the same meaning as above, in an inert organic solvent in the presence of a condensing agent.

The particular starting material of this procedure C (i.e. the teicoplanin compound wherein $R^1$ and/or $R^2$ are alkyl groups as defined) can be prepared with the same methods A or B defined before, but starting from a compound of formula I wherein Y, $R^1$ and $R^2$ are substituted by hydrogen atoms.

In carrying out the amidation for preparing the compounds of this invention, sometimes, and especially when at least one of A, B, and M in the teicoplanin starting material represent hydrogen, could be convenient to protect the primary amino function of the teicoplanin starting material in order to reduce possible undesired side-reactions.

Also, when the amine $HNR^9R^{10}$ contains further reactive functions such as amino or carboxy groups, which may unfavourably interfere with the course of the amidation they are protected by methods known per se in the art such as those described in reference books like T.W. Greene, "Protective Groups in Organic Synthesis", John Wiley and Sons, New York, 1981, and M. Mc. Omie "Protecting Groups in Organic Chemistry" Plenum Press, New York, 1973. These protecting groups must be stable at the conditions of the reaction process, must not unfavorably interfere with the main amidation reaction, and must be easily cleavable and removable from the reaction medium at the end of the reaction without altering the newly formed amide bond.

Representative examples of N-protecting groups which may be advantageously used in the process of the invention for protecting an amino function both in the teicoplanin starting material and, when appropriate, in the $R^9$ and $R^{10}$ moiety of the amine $HNR^9R^{10}$ are carbamate forming reagents characterized by the following oxycarbonyl groups: 1,1-dimethylpropynyloxycarbonyl, t-butyloxycarbonyl, vinyloxycarbonyl, aryloxycarbonyl, cinnamyloxycarbonyl, benzyloxycarbonyl, p-nitrobenzyloxycarbonyl, 3,4-dimethoxy-6-nitrobenzyloxycarbonyl, 2,4-dichlorobenzyloxycarbonyl, 5-benzisoxazolylmethyloxycarbonyl, 9-anthranylmethyloxycarbonyl, diphenylmethyloxycarbonyl, isonicotinyloxycarbonyl, S-benzyloxycarbonyl, and the like.

A convenient means of protection in the case the amine reactant $HNR^9R^{10}$ contains a primary amino function as substituent for $R^9$ and/or $R^{10}$, is, in some instances, the formation of a benzyliden derivative which may be prepared by reacting the amine $HNR^9R^{10}$ with benzaldehyde in a lower alkanol, such as ethanol, preferably at room temperature. After the reaction with the selected teicoplanin starting material has been completed, the benzylidene protecting group may be removed as known in the art, e.g. by treating with diluted mineral acid, preferably hydrochloric acid, at room temperature.

Obviously, when the final compound of formula I contains groups which are labile under acidic conditions, e.g. when A, B or M represent sugar moieties as above defined which may be hydrolized in an acidic medium, other removal conditions must be used, such as catalytic hydrogenation using for instance Palladium on carbon as the catalyst to remove the proper protecting group.

In this case, however, attention should be paid to the presence of groups which may be modified by catalytic hydrogenation. A typical consequence of the catalytic hydrogenation of a derivative of formula I wherein A represents a group as above defined whose acyl portion is Z-decenoyl (i.e. a teicoplanin $A_2$ component 1 derivative or a mixture containing it) is that it is at least partially transformed into the corresponding decanoyl derivative (i.e. a derivative of teicoplanin $A_2$ component 3).

The man skilled in the art is capable, also on the basis of the present disclosure, of deciding which functions of the amine $HNR^9R^{10}$ need to be protected, how they must be protected and the proper deprotection reaction which is necessary to free the final compound.

For instance, a suitable protection for reactive carboxylic acid function is by forming an ester function.

As it is appreciated by the skilled technician, the ultimate choice of the specific protecting group depends on the characteristics of the particular amide derivative which is desired. In fact, this amide function of the final compound should be stable at the condition of removal of the protecting group(s).

Inert organic solvents useful for the condensation reaction are those organic aprotic solvents which do not unfavorably interfere with the reaction course and are capable of at least partially solubilizing the teicoplanin starting material.

Examples of said inert organic solvents are organic amides, alkyl ethers, ethers of glycols and polyols, phosphoramides, sulfoxides and aromatic compounds.

Preferred examples of inert organic solvents are: dimethylformamide, dimethoxyethane, hexamethylphosphoramide, dimethylsulfoxide, benzene, toluene and mixtures thereof.

The condensing agent in the process of the invention is one suitable for forming amide bonds in organic compounds and in particular in peptide synthesis.

Representative and preferred examples of condensing agents are $(C_1-C_4)$alkyl, phenyl or heterocyclic phosphorazidates such as, diphenyl phosphorazidate (DPPA), diethyl phosphorazidate, di(4-nitrophenyl)-phosphorazidate, dimorpholylphosphorazidate and diphenylphosphorochloridate. The preferred condensing agent is diphenyl phosphorazidate (DPPA).

In procedure C, the amine reactant $HNR^9R^{10}$ is normally used in a molar excess.

In general, a 2- to 6-fold molar excess is used while a 3- to 4-fold molar excess is preferred.

For the amidation to proceed, it is necessary that the amine $HNR^9R^{10}$ be capable of forming a salt with the carboxy function of the teicoplanin starting material. In case the amine $HNR^9R^{10}$ is not strong enough to form such a salt in the selected reaction medium, it is necessary to add a salt-forming base to the reaction mixture at least in an equimolecular amount with the teicoplanin starting material.

Examples of said salt-forming bases are tertiary organic aliphatic or alicyclic amines such as trimethylamine, triethylamine, N-methyl pyrrolidine or heterocyclic bases such as picoline, and the like.

The condensing agent is generally employed in a slight molar excess such as from 1.2 to 1.7 and preferably is 1.5 times the teicoplanin starting compound.

In addition, the amine reactant $HNR^9R^{10}$ may also conveniently be introduced in the reaction medium as a corresponding acid addition salt, e.g. the hydrochloride. In this case, at least a double molar proportion and preferably a 2 to 4 fold molar excess of a strong base capable of freeing the $HNR^9R^{10}$ amine from its salts, is used. Also in this case, the suitable base is a tertiary organic aliphatic or alicyclic amine like those exemplified above. In fact, at least in some instances, the use of salt of the amine $HNR^9R^{10}$, which is then freed in situ with the above mentioned bases, is greatly preferred especially when the salt is more stable

than the corresponding free amine.

The reaction temperature will vary considerably depending on the specific starting materials and reaction conditions. In general, it is preferred to conduct the reaction at temperatures between 0-20 °C.
Also the reaction time vary considerably depending on the other reaction parameters. In general the condensation reaction is completed in about 24-48 h.
In any case, the reaction course is monitored by TLC or preferably by HPLC according to methods known in the art.
On the basis of the results of these assays a man skilled in the art will be able to evaluate the reaction course and decide when to stop the reaction and start working up the reaction mass according to known per se techniques which include for instance extraction with solvents, precipitation by addition of non-solvents, etc., in conjunction with further separations and purifications by column chromatography.
As already said, when protection of the $HNR^9R^{10}$ reactant or of the teicoplanin starting material, or of both of them, is necessary, the protected final compound is then de-protected according to procedures which are known per se and mainly depends on the protecting group involved. In case both the amine $HNR^9R^{10}$ and the teicoplanin starting material are protected, it might be convenient to use a similar type of protection which may be removed under the same conditions, so that only one de-protection step is needed to free both functions.

It is also evident that in many instances a compound of the invention may be prepared in more than one way and that a compound of the invention may be transformed into another by means of known per se reactions.
For instance, when the $HNR^9R^{10}$ amine is a diamine compound such as $HN(R^9)-(CH_2)_4-NR^{11}R^{12}$ defined above, the desired amide compound of formula I may be prepared either directly by condensing said amine, conveniently protected if necessary, with the selected starting material or it can be prepared by reacting an amide of formula I wherein the substituent $R^2$ is $(CH_2)_4$-(halo),wherein halo is preferably a chlorine or bromine atom with an amino of formula $HNR^{11}R^{12}$.
Moreover, an amide compound of formula I, bearing a carboxy function on the $-NR^9R^{10}$ moiety may be transformed into the corresponding acyl ester function by usual techniques. More particularly, an ester function is in general formed by reacting the carboxy containing product with a preparation of an alcohol in the presence of an acid catalyst at a temperature varying between room temperature and the boiling point of the reaction mixture. The acid is preferably a mineral acid and the alcohol contains the moiety that is to be linked to the carboxylic function in the ester derivative. An inert solvent may also by used. Obviously, a compound of formula I bearing a carboxylic ester function on the $-NR^9R^{10}$ substituent may be transformed into the corresponding carboxylic compound by hydrolysis.
A preferred hydrolysis technique involves an aqueous solution of an alkali metal carbonate, like sodium or potassium carbonate, at a temperature from room temperature to the boiling point of the reaction mixture.
A compound of formula I bearing an $-NH_2$ function on the $-NR^1R^2$ moiety may be transformed into the corresponding monoalkylamino derivative by means of a "reductive alkylation" which involves reacting it with the selected carbonyl derivative (which is capable of giving the desired alkyl substituent upon reduction) to form the corresponding Schiff base intermediate which is then reduced in the presence of a suitable reducing agent such as sodium or potassium borohydride.
When a free amino group is present in the $-NR^9R^{10}$ moiety of formula I, it may be alkylated as known in the art, e.g. by reacting it, or possibly the corresponding compound wherein the primary amino group of the teicoplanin moiety has been protected in case it is unsubstituted with an alkyl halide (bromide, chloride or iodide). Likewise, a secondary amino function may be transformed into a tertiary one or a tertiary amino function may be quaternized.

The procedure D, which permits obtaining the $N^{15}$, $N^{15}$-dialkyl compounds derived from the teicoplanin amide, comprises adding to a suspension of a monoalkyl derivative of teicoplanin amide, in an organic polar protic solvent a molar excess of a carbonyl compound having the same carbon skeleton of the substituents $R^1$ above and whose oxo group is on the carbon atom which is to link the $N^{15}$-amino group of the teicoplanin moiety, said molar excess being of at least 30 times with respect to the teicoplanin starting material, in the presence of an organic acid of such strength and in such amount that a sample of the obtained mixture diluted with four volumes of water shows a pH between 2.5 and 4, said acid being further characterized in that, under the reaction conditions, it cannot yield a reduction product which is a competitor of the carbonyl compound reactant in the reaction with the $N^{15}$ amino group of the teicoplanin moiety, and reacting the resulting mixture with a metal alkali borohydride at a temperature between 0 °C and 60 °C.

Among the organic polar protic solvents, alkyl alcohols having from 1 to 4 carbon atoms are preferred. Examples of alcohols which can be used are methanol, ethanol, propanol and butanol. In particular, methanol is preferred.

Sometimes in particular cases, a small amount of a polar co-solvent can be added to completely dissolve teicoplanin starting material during the course of the reaction, e.g. N,N-dimethylformamide, 1,3-dimethyl-3,4,5,6-tetrahydro-2(1H)-pyramidone (DMPU), dimethylsulfoxide.

As the reducing agent, alkali metal borohydrides are used, among which sodium borohydride is the preferred one, but also potassium and sodium cyano-borohydrides can be conveniently used.

The temperature can be preferably maintained between 30 ° C and 40 ° C.

The molar excess of the reactants with respect to teicoplanin ranges preferably from 30 to 300 times for aldehydes or ketones.

The organic acid which is used in the present invention is characterized by having such a strength to show a pH between 2.5 and 4 when a sample of the reaction mixture is added to 4 volumes of water before adding the reducing agent. Furthermore, said acid must not negatively interfere in the reaction course. It means that it preferably has not to be reduced by the reducing agent employed ($NaBH_4$ and the like) or, if a reaction occurs, the product of this reaction is not a competitor with the carbonyl compound chosen as starting material.

The term "competitor" as used in specification means any group which can be linked to an amino group with a bond like a "Schiff base" and subsequently reduced to an alkyl moiety, being said group different from the carbonyl compound used as starting material. Thus, in a preferred embodiment of the present invention when an aldehyde is used as the carbonyl compound the suitable acid to be preferably used is the carboxylic acid corresponding to said aldehyde; for example, formic acid with formaldehyde, acetic acid with acetaldehyde and so on. In the case that a keto compound is used as the starting material, a preferred acid can be choosen among a ($C_1$-$C_6$)alkyl sulfonic acid and a ($C_6$-$C_{10}$)aryl sulfonic acid such as for example paratoluensulfonic acid, methanesulfonic acid and the like. The amount of acid used depends on several factors such as the teicoplanin starting material and the carbonyl compound but usually the desired pH is obtained when a molar excess of acid is used from 5 to 20 times with respect to teicoplanin.

In addition, the sugar moiety of an amide compound of formula I having a $N^{15}$ alkyl or a $N^{15}$,$N^{15}$dialkyl substituted may be selectively removed to transform it into another amide compound of formula I (procedure E). For example, an amide compound of formula I wherein A, B, and M represent a sugar moiety as above defined can be transformed into the corresponding compound wherein B and M are as above and A is hydrogen by means of controlled acid hydrolysis in a strong concentrated aqueous organic acid. The concentrated organic acid in this case is preferably aqueous trifluoroacetic acid at a concentration between 75% and 95%, and the reaction temperature is preferably between 10 ° and 50 ° C. The preferred hydrolysis conditions are represented by about 90% trifluoroacetic acid at room temperature. The reaction time varies depending on the other specific reaction parameters but, in any case, the reaction may be monitored by TLC or preferably HPLC techniques. An analogous selective hydrolysis is reported in European Patent Application Publication No. 146822.

Similarly, amide compounds of formula I wherein A, B, and M represent a sugar moiety as above defined or A represents hydrogen and B and M represent sugar moieties as above defined can be transformed into the corresponding amide compounds of formula I wherein A and M represents hydrogen and B represent a sugar moiety as defined by means of a selective hydrolysis with a strong acid in the presence of a polar aprotic solvent selected from ethers, ketones, and mixture thereof which are liquid at room temperature. Preferred hydrolysis conditions are in this case represented by the use of a concentrated mineral acid in the presence of an ether such as dimethoxyethane at room temperature. Also in this case, the reaction course may be monitored by TLC or preferably HPLC. An analogous selective hydrolysis is reported in European Patent Application Publication No. 175100.

According to another embodiment of the present invention, an amide compound of formula I wherein A, B and M represent sugar moieties as defined above, an amide compound of formula I wherein A represents hydrogen and B and M represent the above defined sugar moieties, or an amide compound of formula I wherein A and M represent hydrogen, and B represents a sugar moiety as above defined may be transformed into the corresponding amide compound of formula I wherein A, B and M represent hydrogen atoms by means of a selective hydrolysis in an organic protic solvent selected from aliphatic acids and alpha-halogenated aliphatic acids which at the reaction temperature are liquids, aliphatic and cycloaliphatic alkanols which at the reaction temperature are liquids slightly mixable with water, phenylsubstituted lower alkanols wherein the phenyl moiety may optionally carry ($C_1$-$C_4$)alkyl, ($C_1$-$C_4$)alkoxy or halo rests which at the reaction temperature are liquids slightly mixable with water, and beta-polyhalogenated lower alkanols, which at the reaction temperature are liquids; in the presence of a strong acid, compatible with the solvent, selected from strong mineral acids, strong organic acids and strong acid cation exchange resins in the hydrogen form and at a temperature between 20 ° C and 100 ° C.

In this case, the preferred hydrolysis conditions are represented by the use of a mineral acid, such as hydrochloric acid, in an haloalkanol such as trifluoroethanol, at a temperature between 65 ° C and 85 ° C. Analogous selective hydrolysis conditions on a similar substrate are described in European Patent Application Publication No. 146053.

The procedure G comprises the catalytic hydrogenolysis of a compound of the invention wherein $R^{12}$ is a phenyl methyloxycarbonyl to give the corresponding compound in which $R^{12}$ is H.

The amide derivatives where $R^{12}$ is as defined can be prepared according to one of the above mentioned procedures and the conditions of the catalytic hydrogenolysis are the same conditions described above which permit de-protecting the amino group.

In the following table (Table I) the structure formulas of representative examples of compounds of the invention are reported.

TABLE I

| Compound | A | B | M | $R^1$ | $R^2$ | $Y=-NR^9R^{10}$ |
|---|---|---|---|---|---|---|
| 1 | GA | GAC | M | H | $CH_3$ | $-NH(CH_2)_3-N(CH_3)_2$ |
| 2 | $GA_2$ | do | do | do | do | do |
| 3 | H | H | H | do | do | do |
| 4 | GA | GAC | M | $CH_3$ | do | do |
| 5 | $GA_2$ | do | do | do | do | do |

LEGENDA

GA stands for N/(C$_{10}$-C$_{11}$)aliphatic acyl/-beta-D-2-deoxy-2-aminoglucopyranosyl

$GA_{2-5}$ stands for N/(C$_{10}$-C$_{11}$)aliphatic acyl/-beta-D-2-deoxy-2-aminoglucopyranosyl (components 2 to 5)

$GA_2$ stands for N-(8-methylnonanoyl)-beta-D-2-deoxy-2-aminoglucopyranosyl (component 2)

GAC stands for N-acetyl-beta-D-2-deoxy-2-aminoglucopyranosyl

M stands for alpha-D-mannopyranosyl

H stands for a hydrogen atom

TABLE I (continued)

| Compound | A | B | M | $R^1$ | $R^2$ | $Y=-NR^9R^{10}$ |
|---|---|---|---|---|---|---|
| 6 | H | H | H | $CH_3$ | $CH_3$ | $-NH(CH_2)_3N(CH_3)_2$ |
| 7 | GA | GAC | M | H | $CH(CH_3)-CH_2OH$ | do |
| 8 | H | H | H | do | do | do |
| 9 | GA | GAC | M | $CH_3$ | do | do |
| 10 | do | do | do | H | $CH_2-CH(OH)-CH_2OH$ | do |
| 11 | H | H | H | do | do | do |

TABLE I (continued)

| Compound | A | B | M | $R^1$ | $R^2$ | $Y=-NR^9R^{10}$ |
|---|---|---|---|---|---|---|
| 12 | GA | GAC | M | H | $CH(CH_3)-CH(CH_3)OH$ | $-NH(CH_2)_3N(CH_3)_2$ |
| 13 | H | H | H | do | do | do |
| 14 | GA | GAC | M | do | $CH_2O(CH_2)_2OCH_3$ | $-NH-(CH_2)_3-N(CH_3)_2$ |
| 15 | H | H | H | do | do | do |
| 16 | do | do | do | do | $[(CH_2)_2O]_2(CH_2)_2OH$ | do |
| 17 | GA | GAC | M | do | $CH_3$ | $-NH-(CH_2)_3-N(C_2H_5)_2$ |

TABLE I (continued)

| Compound | A | B | M | $R^1$ | $R^2$ | $Y=-NR^9R^{10}$ |
|---|---|---|---|---|---|---|
| 18 | $GA_2$ | GAC | M | H | $CH_3$ | $-NH(CH_2)_3N(C_2H_5)_2$ |
| 19 | H | H | H | do | do | do |
| 20 | do | do | do | $CH_3$ | do | do |
| 21 | GA | GAC | M | H | $CH(CH_3)CH_2OH$ | do |
| 22 | H | H | H | do | do | do |
| 23 | GA | GAC | M | $CH_3$ | do | do |
| 24 | do | do | do | H | $CH_2CH(OH)CH_2OH$ | do |

TABLE I (continued)

| Compound | A | B | M | $R^1$ | $R^2$ | $Y=-NR^9R^{10}$ |
|---|---|---|---|---|---|---|
| 25 | H | H | H | H | $CH_2-CH(OH)-CH_2OH$ | $NH-(CH_2)_3N(C_2H_5)_2$ |
| 26 | GA | GAC | M | H | $CH(CH_3)CH(CH_3)OH$ | do |
| 27 | H | H | H | H | do | do |
| 28 | GA | GAC | M | H | $CH_2O(CH_2)_2OCH_3$ | do |
| 29 | H | H | H | do | do | do |
| 30 | do | do | do | do | $[(CH_2)_2O]_2(CH_2)_2OH$ | do |
| 31 | GA | GAC | M | H | $CH_3$ | $NH-(CH_2)_3N(C_4H_9)_2$ |

TABLE I (continued)

| Compound | A | B | M | $R^1$ | $R^2$ | $Y=-NR^9R^{10}$ |
|---|---|---|---|---|---|---|
| 32 | GA | GAC | M | H | $CH(CH_3)CH_2OH$ | $-NH-(CH_2)_3-N(C_4H_9)_2$ |
| 33 | H | H | H | do | do | do |
| 34 | GA | GAC | M | do | $CH_2-CH(OH)CH_2OH$ | do |
| 35 | H | H | H | do | do | do |
| 36 | GA | GAC | M | do | $CH(CH_3)CH(CH_3)OH$ | do |
| 37 | H | H | H | do | do | do |

TABLE I (continued)

| Compound | A | B | M | $R^1$ | $R^2$ | $Y=-NR^9R^{10}$ |
|---|---|---|---|---|---|---|
| 38 | GA | GAC | M | $CH_3$ | $CH_3$ | $-NH(CH_2)_2-N(CH_3)_2$ |
| 39 | do | do | do | H | $CH_2CH_2-N(CH_3)_2$ | do |
| 40 | $GA_2$ | do | do | do | do | do |
| 41 | GA | do | do | do | do | $-NH(CH_2)_3-N(CH_3)_2$ |
| 42 | $GA_2$ | do | do | do | do | do |
| 43 | H | H | H | do | do | do |
| 44 | GA | GAC | M | do | do | $-NH-(CH_2)_3N(C_2H_5)_2$ |
| 45 | H | H | H | do | do | do |
| 46 | $GA_2$ | GAC | M | do | do | $-NH(CH_2)_3N(C_4H_9)_2$ |

TABLE I (continued)

| Compound | A | B | M | $R^1$ | $R^2$ | $Y=-NR^9R^{10}$ |
|---|---|---|---|---|---|---|
| 47 | H | H | H | H | $CH_2CH_2N(CH_3)_2$ | $-NH-(CH_2)_3N(C_4H_9)_2$ |
| 48 | $GA_{2-5}$ | GAC | M | do | do | $-NH(CH_2)_2NH_2$ |
| 49 | H | H | H | do | do | do |
| 50 | GA | GAC | M | do | do | $-NH(CH_2)_3-NH_2$ |
| 51 | H | H | H | do | do | do |
| 52 | GA | GAC | M | do | do | $-NH(CH_2)_4-NH_2$ |
| 53 | GA | GAC | do | do | $CH_3$ | $-NH(CH_2)_3-NH_2$ |
| 54 | H | H | H | do | do | do |

TABLE I (continued)

| Compound | A | B | M | $R^1$ | $R^2$ | $Y=-NR^9R^{10}$ |
|---|---|---|---|---|---|---|
| 55 | GA | GAC | M | $CH_3$ | $CH_3$ | $-NH(CH_2)_3-NH_2$ |
| 56 | do | do | do | H | $CH(CH_3)CH_2OH$ | do |
| 57 | H | H | H | do | do | do |
| 58 | GA | GAC | M | do | $CH_2-CH(OH)CH_2OH$ | do |
| 59 | do | do | do | do | $CH(CH_3)-CH(CH_3)OH$ | do |
| 60 | do | do | do | do | do | $-N(CH_3)-(CH_2)_2-NH(CH_3)$ |
| 61 | do | do | do | do | do | $-N(CH_3)-(CH_2)_3-NH(CH_3)$ |

TABLE I (continued)

| Compound | A | B | M | $R^1$ | $R^2$ | $Y=-NR^9R^{10}$ |
|---|---|---|---|---|---|---|
| 62 | H | H | H | H | $CH(CH_3)-CH(CH_3)OH$ | $-N(CH_3)-(CH_2)_3-N(CH_3)H$ |
| 63 | do | do | do | do | $CH_2-CH(OH)-CH_2OH$ | do |
| 64 | GA | GAC | M | do | $CH_3$ | $-NH-(CH_2)_3-NH(CH_2)_2OH$ |
| 65 | H | H | H | do | do | do |
| 66 | $GA_2$ | GAC | M | do | $CH(CH_3)-CH_2OH$ | do |
| 67 | H | H | H | do | do | do |
| 68 | GA | GAC | M | do | $CH(CH_3)-CH(CH_3)OH$ | do |
| 69 | H | H | H | do | do | do |
| 70 | GA | GAC | M | do | $CH_2O(CH_2)_2OCH_3$ | do |

TABLE I (continued)

| Compound | A | B | M | $R^1$ | $R^2$ | $Y=-NR^9R^{10}$ |
|---|---|---|---|---|---|---|
| 71 | H | H | H | H | $CH_2O(CH_2)_2OCH_3$ | $-NH-(CH_2)_3-NH(CH_2)_2-OH$ |
| 72 | do | do | do | do | $CH_2CH(OH)CH_2OH$ | do |
| 73 | do | do | do | do | do | 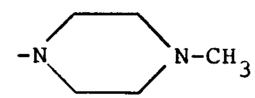 |
| 74 | do | do | do | do | $CH(CH_3)-CH_2OH$ | do |
| 75 | GA | GAC | M | do | do | do |
| 76 | do | do | do | do | $CH(CH_3)-CH(CH_3)-OH$ | do |
| 77 | H | H | H | do | do | do |
| 78 | GA | GAC | M | do | $CH_2CH_2-N(CH_3)_2$ | 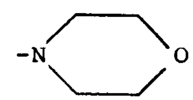 |
| 79 | do | do | do | do | $CH(CH_3)CH_2OH$ | do |
| 80 | H | H | H | do | do | do |

TABLE I (continued)

| Compound | A | B | M | $R^1$ | $R^2$ | $Y=-NR^9R^{10}$ |
|---|---|---|---|---|---|---|
| 81 | H | H | H | H | $CH(CH_3)-CH(CH_3)OH$ | 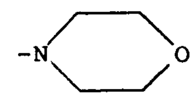 |
| 82 | GA | GAC | M | do | do | do |
| 83 | do | do | do | do | $CH_2-CH(OH)-CH_2OH$ | do |
| 84 | H | H | H | do | do | do |
| 85 | $GA_2$ | GAC | M | do | do | $-NH-CH(COOCH_3)(CH_2)_4-NHCOOCH_2C_6H_5$ |
| 86 | H | H | H | do | do | do |
| 87 | do | do | do | do | $-CH(CH_3)-CH_2OH$ | do |
| 88 | GA | GAC | M | do | do | do |
| 89 | $GA_2$ | do | do | do | do | do |
| 90 | do | do | do | do | $-CH(CH_3)-CH(CH_3)OH$ | do |

TABLE I (continued)

| Compound | A | B | M | $R^1$ | $R^2$ | $Y{=}-NR^9R^{10}$ |
|---|---|---|---|---|---|---|
| 91 | H | H | H | H | $CH(CH_3)-CH(CH_3)OH$ | $-NH-CH(COOCH_3)-(CH_2)_4-NHCOOCH_2C_6H_5$ |
| 92 | $GA_2$ | GAC | M | do | $CH_2-CH(OH)-CH_2OH$ | $-NH-CH(COOCH_3)-(CH_2)_4-NH_2$ |
| 93 | H | H | H | do | do | do |
| 94 | do | do | do | do | $CH(CH_3)-CH_2OH$ | do |
| 95 | $GA_{2-5}$ | GAC | M | do | do | do |
| 96 | $GA_2$ | do | do | do | do | do |
| 97 | do | do | do | do | $CH(CH_3)-CH(CH_3)OH$ | do |
| 98 | H | H | H | do | do | do |
| 99 | GA | GAC | M | do | $CH(CH_3)CH_2OH$ | $-NH_2$ |
| 100 | do | do | do | do | do | $-N(CH_3)_2$ |
| 101 | do | do | do | do | do | $-NH(CH_2)_3-NH(CH_3)$ |
| 102 | do | do | do | do | do | $-NH(CH_2)_3NH(C_2H_5)$ |

TABLE I (continued)

| Compound | A | B | M | $R^1$ | $R^2$ | $Y=-NR^9R^{10}$ |
|---|---|---|---|---|---|---|
| 103 | GA | GAC | M | H | $CH_2CH(OH)-CH_2OH$ | $-NH-(CH_2)_3-NH(C_2H_5)$ |
| 104 | do | do | do | do | do | $-NH_2$ |
| 105 | do | do | do | H | $CH(CH_3)CH(CH_3)OH$ | $-NH(CH_2)_3-NH(CH_3)$ |
| 106 | H | H | H | H | $CH_2CH_2-N(CH_3)_2$ | 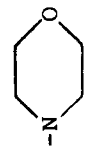 |

In Table II hereinbelow, the starting materials of the above derivatives are given.

Table II is divided in Table IIA, Table IIB, Table IIC, Table IID, Table IIE, Table IIF according to the corresponding procedures A, B, C, D, E, F described above.

TABLE IIA

Procedure A: starting from a teicoplanin amide wherein $R^1$ and $R^2$ are replaced by two hydrogen atoms and from an alkyl halide

| Compound | A | B | M | Y | alkyl halide |
|---|---|---|---|---|---|
| 14 | GA | GAC | M | $-NH-(CH_2)_3-N(CH_3)_2$ | $Cl-CH_2O(CH_2)_2OCH_3$ |
| 15 | H | H | H | do | do |
| 28 | GA | GAC | M | $-NH-(CH_2)_3-N(C_2H_5)_2$ | do |
| 70 | do | do | do | $-NH(CH_2)_3-NH(CH_2)_2OH$ | do |
| 71 | H | H | H | do | do |
| 16 | do | do | do | $-NH(CH_2)_3N(CH_3)_2$ | $Cl[(CH_2)_2O]_2(CH_2)_2OH$ |
| 30 | do | do | do | $-NH(CH_2)_3N(C_2H_5)_2$ | do |

TABLE IIB

Procedure B: starting from a teicoplanin amide wherein $R^1$ and $R^2$ are replaced by two hydrogen atoms and from a carbonyl compound

| Compound | A | B | M | Y | carbonyl compound |
|---|---|---|---|---|---|
| 1 | GA | GAC | M | $NH(CH_2)_3N(CH_3)_2$ | HCOH |
| 2 | $GA_2$ | do | do | do | do |
| 3 | H | H | H | do | do |
| 7 | GA | GAC | M | do | $CH_3-CO-CH_2OH$ |
| 8 | H | H | H | do | do |
| 10 | GA | GAC | M | do | $COH-CH(OH)-CH_2OH$ |
| 11 | H | H | H | do | do |
| 12 | GA | GAC | M | do | $CH_3-CO-CH(CH_3)-OH$ |
| 13 | H | H | H | do | do |

TABLE IIB

Procedure B: starting from a teicoplanin amide wherein $R^1$ and $R^2$ are replaced by two hydrogen atoms and from a carbonyl compound

| Compound | A | B | M | Y | carbonyl compound |
|---|---|---|---|---|---|
| 17 | GA | GAC | M | $-NH-(CH_2)_3-N(C_2H_5)_2$ | HCOH |
| 18 | $GA_2$ | do | do | do | do |
| 19 | H | H | H | do | do |
| 21 | GA | GAC | M | do | $CH_3-CO-CH_2OH$ |
| 22 | H | H | H | do | do |
| 24 | GA | GAC | M | do | $COH-CH(OH)-CH_2OH$ |
| 25 | H | H | H | do | do |
| 26 | GA | GAC | M | do | $CH_3-CO-CH(CH_3)OH$ |
| 27 | H | H | H | do | do |
| 31 | GA | GAC | M | $-NH-(CH_2)_3-N(C_4H_9)_2$ | HCOH |
| 32 | do | do | do | do | $CH_3-CO-CH_2-OH$ |
| 33 | H | H | H | do | do |

TABLE IIB

Procedure B: starting from a teicoplanin amide wherein $R^1$ and $R^2$ are replaced by two hydrogen atoms and from a carbonyl compound

| Compound | A | B | M | Y | carbonyl compound |
|---|---|---|---|---|---|
| 34 | GA | GAC | M | $-NH-(CH_2)_3-N(C_4H_9)_2$ | $COH-CH(OH)-CH_2OH$ |
| 35 | H | H | H | do | do |
| 36 | GA | GAC | M | do | $CH_3-CO-CH(CH_3)-OH$ |
| 37 | H | H | H | do | do |
| 39 | GA | GAC | M | $-NH-(CH_2)_2-N(CH_3)_2$ | $COHCH_2-N(CH_3)_2$ |
| 40 | $GA_2$ | do | do | do | do |
| 41 | GA | do | do | $NH-(CH_2)_3-N(CH_3)_2$ | do |
| 42 | $GA_2$ | do | do | do | do |
| 43 | H | H | H | do | do |
| 44 | GA | GAC | M | $-NH-(CH_2)_3-N(C_2H_5)_2$ | do |
| 45 | H | H | H | do | do |

TABLE IIB

Procedure B: starting from a teicoplanin amide wherein $R^1$ and $R^2$ are replaced by two hydrogen atoms and from a carbonyl compound

| Compound | A | B | M | Y | carbonyl compound |
|---|---|---|---|---|---|
| 46 | $GA_2$ | GAC | M | $-NH(CH_2)_3N(C_4H_9)_2$ | $COH-CH_2-N(CH_3)_2$ |
| 47 | H | H | H | do | do |
| 48 | $GA_{2-5}$ | GAC | M | $-NH(CH_2)_2-NH_2$ | do |
| 49 | H | H | H | do | do |
| 50 | GA | GAC | M | $-NH-(CH_2)_3-NH_2$ | do |
| 51 | H | H | H | do | do |
| 52 | GA | GAC | M | $-NH-(CH_2)_4-NH_2$ | do |
| 54 | H | H | H | $-NH(CH_2)_3-NH_2$ | HCOH |
| 56 | GA | GAC | M | do | $CH_3CO-CH_2OH$ |
| 58 | do | do | do | do | $COH-CH(OH)-CH_2OH$ |
| 59 | do | do | do | do | $CH_3-CO-CH(CH_3)OH$ |
| 60 | do | do | do | $-N(CH_3)-(CH_2)_2-NH(CH_3)$ | do |
| 61 | do | do | do | $-N(CH_3)-(CH_2)_3-NH(CH_3)$ | do |
| 57 | H | H | H | $-NH(CH_2)_3NH_2$ | $CH_3-CO-CH_2OH$ |

TABLE IIB

Procedure B: starting from a teicoplanin amide wherein $R^1$ and $R^2$ are replaced by two hydrogen atoms and from a carbonyl compound

| Compound | A | B | M | Y | carbonyl compound |
|---|---|---|---|---|---|
| 62 | H | H | H | $-N(CH_3)-(CH_2)_3-NH(CH_3)$ | $CH_3-CO-CH(CH_3)OH$ |
| 63 | do | do | do | do | $COH-CH(OH)-CH_2OH$ |
| 64 | GA | GAC | M | $-NH(CH_2)_3-NH-(CH_2)_2-OH$ | HCOH |
| 65 | H | H | H | do | do |
| 66 | $GA_2$ | GAC | M | do | $CH_3-CO-CH_2OH$ |
| 67 | H | H | H | do | do |
| 68 | GA | GAC | M | do | $CH_3-CO-CH(CH_3)OH$ |
| 69 | H | H | H | do | do |
| 72 | do | do | do | do | $COH-CH(OH)-CH_2OH$ |
| 73 | do | do | do | 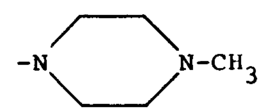 | do |
| 74 | do | do | do | do | $CH_3-CO-CH_2OH$ |
| 75 | GA | GAC | M | do | do |

TABLE IIB

Procedure B: starting from a teicoplanin amide wherein $R^1$ and $R^2$ are replaced by two hydrogen atoms and from a carbonyl compound

| Compound | A | B | M | Y | carbonyl compound |
|---|---|---|---|---|---|
| 76 | GA | GAC | M | 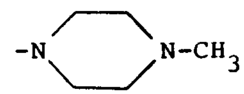 | $CH_3-CO-CH(CH_3)OH$ |
| 77 | H | H | H | do | do |
| 78 | GA | GAC | M | 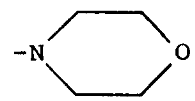 | $COH-CH_2-N(CH_3)_2$ |
| 79 | do | do | do | do | $CH_3-CO-CH_2OH$ |
| 80 | H | H | H | do | do |
| 81 | do | do | do | do | $CH_3-CO-CH(CH_3)OH$ |
| 82 | GA | GAC | M | do | do |
| 83 | do | do | do | do | $COH-CH(OH)-CH_2OH$ |
| 84 | H | H | H | do | do |
| 85 | $GA_2$ | GAC | M | $-NH-CH(COOCH_3)(CH_2)_4-NHCOOCH_2C_6H_5$ | do |
| 86 | H | H | H | do | do |
| 87 | do | do | do | do | $CH_3-CO-CH_2OH$ |

TABLE IIB

Procedure B: starting from a teicoplanin amide wherein $R^1$ and $R^2$ are replaced by two hydrogen atoms and from a carbonyl compound

| Compound | A | B | M | Y | carbonyl compound |
|---|---|---|---|---|---|
| 88 | GA | GAC | M | $NH-CH(COOCH_3)(CH_2)_4-NHCOOCH_2C_6H_5$ | $CH_3-CO-CH_2OH$ |
| 89 | $GA_2$ | do | do | do | do |
| 90 | do | do | do | do | $CH_3-CO-CH(CH_3)OH$ |
| 91 | H | H | H | do | do |
| 99 | GA | GAC | M | $-NH_2$ | $CH_3CO-CH_2OH$ |
| 100 | do | do | do | $-N(CH_3)_2$ | do |
| 101 | do | do | do | $-NH(CH_2)_3-NH(CH_3)$ | do |
| 102 | do | do | do | $-NH(CH_2)_3-NH(C_2H_5)$ | do |
| 103 | do | do | do | do | $COH-CH(OH)CH_2OH$ |
| 104 | do | do | do | $-NH_2$ | do |
| 105 | do | do | do | $-NH-(CH_2)_3-NH(CH_3)$ | $CH_3-CO-CH(CH_3)-OH$ |
| 106 | H | H | H | 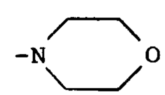 | $COH-CH_2-N(CH_3)_2$ |

TABLE IIC

Procedure C: starting from a teicoplanin compound wherein y is replaced by an -OH group and from an amine

| Compound | A | B | M | $R^1$ | $R^2$ | amine |
|---|---|---|---|---|---|---|
| 1 | GA | GAC | M | H | $CH_3$ | $NH_2(CH_2)_3-N(CH_3)_2$ |
| 20 | H | H | H | $CH_3$ | $CH_3$ | $NH_2(CH_2)_3N(C_2H_5)_2$ |
| 53 | GA | GAC | M | H | $CH_3$ | $NH_2(CH_2)_3NH_2$ |
| 55 | do | do | do | $CH_3$ | $CH_3$ | do |

TABLE IID

Procedure D: starting from a teicoplanin amide wherein $R^1$ is replaced by an hydrogen atom and from a carbonyl compound

| Compound | A | B | M | $R^2$ | Y | carbonyl compound |
|---|---|---|---|---|---|---|
| 4 | GA | GAC | M | $CH_3$ | $-NH-(CH_2)_3-N(CH_3)_2$ | HCOH |
| 5 | $GA_2$ | do | do | $CH_3$ | do | HCOH |
| 9 | GA | do | do | $-CH(CH_3)-CH_2OH$ | do | HCOH |
| 23 | do | do | do | do | $NH-(CH_2)_3-N(C_2H_5)_2$ | HCOH |
| 38 | do | do | do | H | $-NH-(CH_2)_2N(CH_3)_2$ | HCOH |

TABLE IIE

Procedure E: starting from teicoplanin derivatives prepared with other procedures followed by selective hydrolysis

| Compound | A | B | M | $R^1$ | $R^2$ | $Y=NR^1R^2$ |
|---|---|---|---|---|---|---|
| 6 | GA | GAC | M | $CH_3$ | $CH_3$ | $-NH-(CH_2)_3-N(CH_3)_2$ |
| 15 | do | do | do | H | $-CH_2O(CH_2)_2OCH_3$ | do |
| 29 | do | do | do | do | do | $-NH-(CH_2)_3-N(C_2H_5)_2$ |

TABLE IIF

Procedure F: starting from teicoplanin derivatives prepared with other procedures followed by catalytic hydrogenolysis

| Compound | A | B | M | $R^1$ | $R^2$ | $Y=NR^1R^2$ |
|---|---|---|---|---|---|---|
| 92 | $GA_2$ | GAC | M | H | $-CH_2-CH(OH)-CH_2OH$ | $NH-CH(COOCH_3)-(CH_2)_4NHCOOCH_2C_6H_5$ |
| 93 | H | H | H | do | do | do |
| 94 | do | do | do | do | $-CH(CH_3)-CH_2OH$ | do |
| 95 | GA | GAC | M | do | do | do |
| 96 | $GA_2$ | do | do | do | do | do |
| 97 | do | do | do | do | $-CH(CH_3)-CH(CH_3)OH$ | do |
| 98 | H | H | H | do | do | do |

In Table III herein below the yields calculated from the proper starting material (which is reported in Table II) together with the proper procedure of preparation are given.

TABLE III

| COMPOUND NO. | METHOD OF PREPARATION | YIELD (%) |
|---|---|---|
| 1 | B | 89 |
| 1 | C | 56 |
| 2 | B | 91 |
| 3 | B | 78 |
| 4 | B | 83 |
| 4 | D | 63 |
| 5 | D | 69 |
| 6 | E | 56 |
| 7 | B | 86 |
| 8 | B | 84 |
| 9 | D | 71 |
| 10 | B | 53 |
| 11 | B | 67 |
| 12 | B | 90 |
| 13 | B | 86 |
| 14 | A | 44 |
| 15 | A | 36 |
| 15 | E | 43 |
| 16 | A | 32 |
| 17 | B | 93 |
| 18 | B | 95 |

TABLE III

| COMPOUND NO. | METHOD OF PREPARATION | YIELD (%) |
|---|---|---|
| 19 | B | 88 |
| 20 | C | 49 |
| 21 | B | 87 |
| 22 | B | 77 |
| 23 | D | 82 |
| 24 | B | 64 |
| 25 | B | 62 |
| 26 | B | 86 |
| 27 | B | 74 |
| 28 | A | 39 |
| 29 | E | 41 |
| 30 | A | 29 |
| 31 | B | 96 |
| 32 | B | 83 |
| 33 | B | 67 |
| 34 | B | 71 |
| 35 | B | 63 |
| 36 | B | 80 |
| 37 | B | 74 |
| 38 | C | 91 |
| 39 | B | 49 |
| 40 | B | 53 |

TABLE III

| COMPOUND NO. | METHOD OF PREPARATION | YIELD (%) |
|---|---|---|
| 41 | B | 54 |
| 42 | B | 61 |
| 43 | B | 37 |
| 44 | B | 63 |
| 45 | B | 34 |
| 46 | B | 65 |
| 47 | B | 42 |
| 48 | B | 61 |
| 49 | B | 49 |
| 50 | B | 64 |
| 51 | B | 53 |
| 52 | B | 67 |
| 53 | C | 44 |
| 54 | B | 71 |
| 55 | C | 53 |
| 56 | B | 89 |
| 57 | B | 52 |
| 58 | B | 56 |
| 59 | B | 86 |
| 60 | B | 88 |
| 61 | B | 90 |
| 62 | B | 41 |
| 63 | B | 66 |

TABLE III

| COMPOUND NO. | METHOD OF PREPARATION | YIELD (%) |
|---|---|---|
| 64 | B | 79 |
| 65 | B | 61 |
| 66 | B | 76 |
| 67 | B | 75 |
| 68 | B | 70 |
| 69 | B | 53 |
| 70 | A | 23 |
| 71 | A | 21 |
| 72 | B | 46 |
| 73 | B | 47 |
| 74 | B | 58 |
| 75 | B | 88 |
| 76 | B | 85 |
| 77 | B | 44 |
| 78 | B | 51 |
| 79 | B | 92 |
| 80 | B | 74 |
| 81 | B | 65 |
| 82 | B | 83 |
| 83 | B | 87 |
| 84 | B | 53 |
| 85 | B | 68 |
| 86 | B | 35 |

TABLE III

| COMPOUND NO. | METHOD OF PREPARATION | YIELD (%) |
|---|---|---|
| 87 | B | 41 |
| 88 | B | 70 |
| 89 | B | 73 |
| 90 | B | 67 |
| 91 | B | 46 |
| 92 | F | 70 |
| 93 | F | 72 |
| 94 | F | 66 |
| 95 | F | 74 |
| 96 | F | 74 |
| 97 | F | 76 |
| 98 | F | 54 |
| 99 | B | 88 |
| 100 | B | 85 |
| 101 | B | 63 |
| 102 | B | 65 |
| 103 | B | 57 |
| 104 | B | 62 |
| 105 | B | 66 |
| 106 | B | 53 |

HPLC Analysis

The following table (table IV) reports the $t_R$ of representative examples of the compounds of the invention.

The assays were run with a VARIAN model 5000 LC pump equipped with a 20 microl loop injector Rheodyne Model 7125. Columns: pre-column (1.9 cm) Hibar LiChro Cart 25-4 MERCK pre-packed with LiChrosorb RP-8 (20-30 micrometer) followed by a column Hibar RT 250-4 MERCK pre-packed with LiChrosorb RP-8 (10 micrometer).

Eluents: A, 0.2% aq. $HCOONH_4$ and B, $CH_3CN$

Injection: 20 microl - Flow rate: 2 ml/min.

The reaction is monitored by injecting, at established times, samples of the solutions (or suspensions) diluted with the solvent mixture ($CH_3CN$ : $H_2O$, 6:4 (v/v)) enough to obtain final concentrations of either 1, 2

or 3 mg/ml.
Linear gradient: from 20 to 60% of B in A in 30 min.

TABLE IV

| Compound (No.) | HPLC ($t_R$, min) |
|---|---|
| 1 | 13.2 * |
| 4 | 14.9 * |
| 7 | 10.3 * |
| 8 | 9.9 |
| 10 | 9.6 * |
| 11 | 6.2 |
| 12 | 12.2 * |
| 13 | 11.3 |
| 14 | 12.1 * |
| 15 | 14.4 |
| 38 | 12.5 * |
| 41 | 12.5 * |
| 43 | 11.9 |
| 53 | 12.4 * |
| 55 | 13.1 * |
| 62 | 10.9 |
| 63 | 5.8 |

(*) Values referred to the component "2" of the "$A_2$" complex.

Acid base titrations

The products are dissolved in methylcellosolve (MCS):$H_2O$ 4:1 (v/v), then an excess of 0.01 N HCl in the same solvent mixture is added and the resulting solutions are titrated with 0.01 N NaOH.
The equivalent weights of representative compounds of the invention are reported in table V, below.

TABLE V

| Compound (No.) | E.W. |
|---|---|
| 1 | 1015 (x 2) |
| 4 | 1061 (x 2) |
| 7 | 988 (x 2) |
| 8 | 743 (x 2) |
| 10 | 1014(x 2) |
| 11 | 759 (x 2) |
| 12 | 1003 (x 2) |
| 13 | 712 (x 2) |
| 14 | 1024 (x 2) |
| 15 | 756 (x 2) |
| 38 | 1008 (x 2) |
| 41 | 689 (x 3) |
| 43 | 499 (x 3) |
| 53 | 981 (x 2) |
| 55 | 1002 (x 2) |
| 62 | 687 (x 2) |
| 63 | 711 (x 2) |

Table VI reports $^1$HNMR data obtained at 500 MHz with a Bruker AM-500 Spectrometer in DMSO-$d_6$ with tetramethylsilane (TMS) as the internal reference (delta = 0.00 ppm)

TABLE VI

$^{1}H$-NMR spectral data (delta, ppm) in DMSO-$d_6$

| Compound | |
|---|---|
| 1 | 2.18 [$N(CH_3)_2$]; 2.34 ($NCH_3$); 1.87 (acetyl-glucosamine); 0.87, 1.43, 2.05 (acyl chain); 3.48 (mannose); 4.13-5.65 (peptidic CH's); 6.15-8.61 (aromatic protons and peptidic NH's). |
| 4 | 2.20 [$N(CH_3)_2$]; 2.31 ($NCH_3$); 1.88 (acetyl-glucosamine); 0.83, 1.14, 1.44 (acyl chain); 4.12-5.64 (peptidic CH's); 6.28-8.62 (aromatic protons and peptidic NH's). |
| 7 | 2.22 [$NH(CH_3)_2$]; 1.04 ($CH_3^{*}$-CH); 1.62 ($CH^{*}$-$CH_3$); 1.87 (acetylglucosamine); 0.86, 1.16, 1.43, 2.02 (acyl chain); 4.12-5.13 (peptidic CH's); 6.23-8.65 (aromatic protons and peptidic NH's). |
| 8 | 2.69 ($NCH_3$); 2.96, 1.89 ($CH_2$); 1.22 ($CH_3^{*}$-CH); 3.41 ($CH_2$-OH); 4.16-5.86 (peptidic CH's); 6.18-8.41 (aromatic protons and peptidic NH's). |
| 10 | 2.32 ($NCH_3$); 3.53 (CH-OH); 1.86 (acetylglucosamine); 0.86, 1.13, 1.47, 2.03 (acyl chain); 4.13-5.62 (peptidic CH's); 6.18-8.63 (aromatic protons and peptidic NH's). |

TABLE VI (continued)

$^1$H-NMR spectral data (delta, ppm) in DMSO-$d_6$

| Compound | |
|---|---|
| 11 | 2.12 ($NCH_3$); 2.18, 3.23, 3.42 ($CH_2$); 4.14-5.59 (peptidic CH's); 6.16-8.43 (aromatic protons and peptidic NH's). |
| 12 | 2.10 ($NCH_3$); 1.08, 1.12 ($CH_3^*$-CH); 1.86 (acetylglucosamine); 0.84, 1.16, 2.01 (acyl chain); 3.48 (mannose); 4.09-5.56 (peptidic CH's); 6.23-8.62 (aromatic protons and peptidic NH's). |
| 13 | 2.66 ($NCH_3$); 3.05 ($CH_2$-N); 1.82 ($CH_2$); 0.99, 1.05 ($CH_3$); 4.16-5.60 (peptidic CH's); 6.17-8.43 (aromatic protons and peptidic NH's). |
| 14 | 2.93 ($NCH_3$); 3.45 ($CH_2^*$-O); 3.25 ($OCH_3$); 1.83 (acetylglucosamine); 0.83, 1.14, 1.46, 1.98 (acyl chain); 3.51 (mannose); 4.24-5.63 (peptidic CH's); 6.21-8.51 (aromatic protons and peptidic NH's). |
| 15 | 2.90 ($NCH_3$); 3.27 ($OCH_3$); 3.92, 3.17 ($CH_2O$); 4.18-5.65 (peptidic CH's); 5.92-8.52 (aromatic protons and peptidic NH's). |

TABLE VI (continued)

$^1$H-NMR spectral data (delta, ppm) in DMSO-$d_6$

| Compound | |
|---|---|
| 38 | 2.32, 2.18 ($NCH_3$); 1.89 (acetylglucosamine); 0.83, 1.14, 1.46 (acyl chain); 3.50 (mannose); 4.13-5.62 (peptidic CH's); 6.23-8.64 (aromatic protons and peptidic NH's). |
| 41 | 2.22, 2.19 ($NCH_3$); 3.28, 2.35 ($CH_2N$);0.83, 1.15, 1.46, 2.03 (acyl chain); 1.88 (acetyl-glucosamine); 4.09-5.68 (peptidic CH's); 6.30-8.58 (aromatic protons and peptidic NH's). |
| 53 | 2.28 ($NCH_3$); 3.22 ($CH_2N$); 0.82, 1.14, 1.47, 2.02 (acyl chain); 1.87 (acetylglucosamine); 3.48 (mannose); 4.12-5.61 (peptidic CH's); 6.28-8.56 (aromatic protons and peptidic NH's). |
| 55 | 2.28 ($NCH_3$); 3.28, 2.77 ($NCH_2$); 0.83, 1.13, 1.43, 2.00 (acyl chain); 1.85 (acetylglucosamine); 3.50 (mannose); 4.12-5.62 (peptidic CH's); 6.23-8.64 (aromatic protons and peptidic NH's). |

TABLE VI (continued)

$^{1}$H-NMR spectral data (delta, ppm) in DMSO-$d_6$

| Compound | |
|---|---|
| 62 | 2.69 ($NCH_3$); 2.95 ($CH_2N$); 1.08, 1.13 ($CH_3^*$-CH); 3.42 (CH-OH); 4.16-5.61 (peptidic CH's); 6.18-8.39 (aromatic protons and peptidic NH's). |
| 63 | 2.69 ($NCH_3$); 1.87, 2.87 ($CH_2$); 3.32-3.44 ($CH_2$, CH-OH); 4.16-5.62 (peptidic CH's); 6.18-8.42 (aromatic protons and peptidic NH's). |
| 80 | 3.55, 3.18 (morpholine); 1.26 ($CH_3$); 4.03-5.70 (peptidic CH's); 6.19-8.54 (aromatic protons and peptidic NH's). |

The symbol * indicates the protons which the reported chemical shift is attributed to.

The antibacterial activity of the compounds of the invention can be demonstrated in vitro by means of standard agar-dilution tests.
Isosensitest broth (Oxoid) and Todd-Hewitt broth (Difco) are used for growing staphylococci and streptococci, respectively. Broth cultures are diluted so that the final inoculum is about $10^4$ colony forming units/ml (CFU/ml). Minimal inhibitory concentration (MIC) is considered as the lowest concentration which shows no visible growth after 18-24 h incubation at 37°C. The results of the antibacterial testing of representative compounds of formula I are summarized in Table VII below:

TABLE VII

| Microorganism | Compound 1 | 4 | 7 | 8 | 10 |
|---|---|---|---|---|---|
| | MIC (microg/ml) | | | | |
| S.aureus Tour | 0.12 | 0.50 | 0.25 | 0.12 | 0.25 |
| S.epidermidis ATCC 12228 | 0.06 | 0.12 | 0.12 | 0.06 | 0.12 |
| S.haemolyticus L 602 | 0.12 | 1.00 | 0.25 | 0.12 | 0.50 |
| S.pyogenes C 203 | 0.06 | 0.12 | 0.06 | 0.12 | 0.12 |
| S.pneumoniae UC 41 | 0.12 | 0.50 | 0.25 | 0.25 | 0.25 |
| S.faecalis ATCC 7980 | 0.12 | 0.12 | 0.12 | 0.25 | 0.12 |
| E. coli SKF 12140 | | | | 16 | |
| Proteus vulgaris X 19H ATCC 881 | > 128 | > 128 | > 128 | > 128 | > 128 |
| Pseudomonas aeruginosa ATCC 10145 | | | | 128 | |

TABLE VII (continued)

| Microorganism | Compound 11 | 12 | 13 | 14 | 15 |
|---|---|---|---|---|---|
| | MIC (microg/ml) | | | | |
| S.aureus Tour | 0.12 | 0.25 | 0.12 | 1.00 | 0.06 |
| S.epidermidis ATCC 12228 | 0.06 | 0.12 | 0.06 | 0.12 | 0.06 |
| S.haemolyticus L 602 | 0.25 | 0.50 | 0.12 | 4.00 | 0.12 |
| S.pyogenes C 203 | 0.12 | 0.06 | 0.12 | 0.06 | 0.06 |
| S.pneumoniae UC 41 | 0.5 | 0.12 | 0.25 | 0.25 | 0.12 |
| S.faecalis ATCC 7980 | 0.25 | 0.12 | 0.25 | 0.12 | 0.12 |
| E. coli SKF 12140 | 64 | | 16 | | 8 |
| Proteus vulgaris X 19H ATCC 881 | > 128 | > 128 | > 128 | > 128 | > 128 |
| Pseudomonas aeruginosa ATCC 10145 | | | | | 128 |

TABLE VII

| Microorganism | Compound 38 | 41 | 43 | 53 | 55 |
|---|---|---|---|---|---|
| | MIC (microg/ml) | | | | |
| S.aureus Tour | 0.25 | 0.12 | 0.12 | 0.12 | 0.12 |
| S.epidermidis ATCC 12228 | 0.06 | 0.06 | 0.06 | 0.06 | 0.06 |
| S.haemolyticus L 602 | 0.12 | 0.25 | 0.12 | 0.12 | 0.12 |
| S.pyogenes C 203 | 0.06 | 0.06 | 0.12 | 0.06 | 0.06 |
| S.pneumoniae UC 41 | 0.25 | 0.12 | 0.12 | 0.12 | 0.12 |
| S.faecalis ATCC 7980 | 0.12 | 0.12 | 0.25 | 0.06 | 0.12 |
| E. coli SKF 12140 | | | 16 | | |
| Proteus vulgaris X 19H ATCC 881 | > 128 | > 128 | > 128 | > 128 | > 128 |
| Pseudomonas aeruginosa ATCC 10145 | | | 128 | | |

TABLE VII

| Microorganism | Compound | | | |
|---|---|---|---|---|
| | 62 | 63 | 80 | 106 |
| | MIC (microg/ml) | | | |
| S.aureus Tour | 0.12 | 0.06 | 0.06 | 0.06 |
| S.epidermidis ATCC 12228 | 0.06 | 0.06 | 0.06 | 0.06 |
| S.haemoliticus L 602 | 0.12 | 0.12 | 0.25 | 0.25 |
| S.pyogenes C 203 | 0.12 | 0.12 | 0.12 | 0.12 |
| S.pneumoniae UC 41 | 0.12 | 0.12 | 0.12 | 0.12 |
| S.faecalis ATCC 7980 | 0.12 | 0.12 | 0.12 | 0.12 |
| E. coli SKF 12140 | | 16 | 64 | 32 |
| Proteus vulgaris X 19H ATCC 881 | > 128 | 64 | > 128 | > 128 |
| Pseudomonas aeruginosa ATCC 10145 | | 128 | | |

The $ED_{50}$ values (mg/kg) of representative compounds of the invention in vitro tests in mice experimentally infected with S.pyogenes L 49 according to the procedure described by V. Arioli et al., Journal of Antibiotics 29; 511 (1976) are reported in Table VIII below:

TABLE VIII

| Compound | $ED_{50}$ (mg/Kg) Route of administration | |
|---|---|---|
| | os | s.c. |
| 1 | 89.5 | 0.06 |
| 7 | > 300 | 0.23 |
| 8 | > 300 | 2.18 |
| 10 | > 300 | 0.31 |
| 11 | > 300 | 2.87 |
| 12 | 215 | 0.13 |
| 13 | > 300 | 1.25 |
| 14 | 89.5 | 0.06 |
| 15 | > 300 | 0.31 |
| 38 | 300 | 0.31 |
| 41 | > 300 | 0.24 |
| 53 | 173 | 0.13 |
| 55 | 300 | 0.18 |
| 62 | > 300 | 0.31 |
| 63 | > 300 | 1.25 |

In view of the above reported antimicrobial activity, the compounds of the present invention can effectively be employed as the active ingredient of antimicrobial preparations used in human and veterinary medicine for the prevention and treatment of infectious diseases caused by pathogenic bacteria which are susceptible to said active ingredients.

In such treatments, these compounds may be employed as such or in the form of mixtures in any proportion.

The compounds of the present invention can be administered orally, topically or parenterally wherein however, the parenteral administration is preferred. Depending on the route of administration, these compounds can be formulated into various dosage forms. Preparations for oral administration may be in the form of capsules, tablets, liquid solutions or suspensions. As known in the art the capsules and tablets may contain in addition to the active ingredient, conventional excipients such as diluents, e.g. lactose, calcium phosphate, sorbitol and the like, lubricants, e.g. magnesium stearate, talc, polyethylene glycol, binding agents, e.g. polyvinylpyrrolidone, gelatin, sorbitol, tragacanth, acacia, flavoring agents, and acceptable disintegrating and wetting agents. The liquid preparations generally in the form of aqueous or oily solutions or suspensions, may contain conventional additives such as suspending agents. For topical use the compounds of the present invention may also be prepared in suitable forms for absorption through the mucous membranes of the nose and throat or bronchial tissues and may conveniently take the form of liquid sprays or inhalants, lozenges, or throat paints.

For medication of the eyes or ears, the preparation may be presented in liquid or semi-liquid form. Topical applications may be formulated in hydrophobic or hydrophilic bases as ointments, creams, lotions, paints, or powders.

For rectal administration the compounds of the invention are administered in the form of suppositories admixed with conventional vehicles, such as, for example, cocoa butter, wax, spermaceti or polyethylenglycols and their derivatives.

Compositions for injection may take such forms as suspensions, solutions, or emulsions in oily or aqueous vehicles, and may contain formulatory agents such as suspending, stabilizing and/or dispersing agents.

Alternatively, the active ingredient may be in powder form for reconstitution at the time of delivery with a suitable vehicle, such as sterile water.

The amount of active principle to be administered depends on various factors such as the size and conditions of the subject to be treated, the route and frequency of administration, and the causative agent involved.

The compounds of the invention are generally effective at a dosage comprised between about 0.5 and about 30 mg of active ingredient per Kg of body weight, preferably divided in 2 to 4 administrations per day. Particularly desirable compositions are those prepared in the form of dosage units containing from about 20 to about 300 mg per unit.

A representative example of preparation of a pharmaceutical composition is a parenteral solution which is

prepared with 100 mg of compound No 1 dissolved in 1 ml of sterile water for injection.

Besides their activity as medicaments, the compounds of the present invention can be used as animal growth promoters.

For this purpose, one or more of the compounds of the invention is administered orally in a suitable feed. The exact concentration employed is that which is required to provide for the active agent in a growth promotant effective amount when normal amounts of feed are consumed.

The addition of the active compounds of the invention to animal feed is preferably accomplished by preparing an appropriate feed premix containing the active compounds in an effective amount and incorporating the premix into the complete ration.

Alternatively, an intermediate concentrate or feed supplement containing the active ingredient can be blended into the feed.

The ways in which such feed premixes and complete rations can be prepared and administered are described in reference books (such as "Applied Animal Nutrition", W.H. Freedman and Co., S. Francisco, USA, 1969 or "Livestock Feeds and Feeding", O and B Books, Corvallis, Oregon, USA, 1977) and are incorporated herein by reference.

The following examples further illustrate the invention and must not be construed as limiting it.

Examples 1-7

(Procedure A: reaction of an amide derivative of teicoplanin with an alkyl halide)

Preparation of compounds: 14, 15, 28, 70, 71

A solution of 5 mmol of the proper starting $N^{63}$-carboxy-amide of teicoplanin $A_2$ or of deglucoteicoplanin (see Table IIA), 6 ml (about 43 mmol) of triethylamine (TEA) and 2.8 ml (24 mmol) of 2-methoxyethoxymethyl chloride (MEM) (Aldrich) in 100 ml of dimethylformamide (DMF) is stirred at room temperature for 60 min, afterwards 50 ml of absolute ethanol is added. The resulting solution is poured into 550 ml of ethyl acetate and a solid precipitated is obtained which is collected by filtration, washed with 200 ml of ethyl ether and suspended in 250 ml of a mixture acetonitrile:water 1:9 (v/v). By adding glacial acetic acid a clear solution forms (pH 3). This solution is loaded at the top of a column of 700 g of silanized Silica-gel (0.06-0.2 mm) Merck Co. in the above solvent mixture. The column is developed with a linear gradient from 10 to 80% of acetonitrile in water in 10 h at a flow rate of about 400 ml/h, while collecting 20 ml fractions which are checked by HPLC. Those fractions containing pure title compounds are pooled and enough n-butanol is added to obtain, after concentration of the resulting mixture at 45°C under reduced pressure, a final dry cloudy butanolic solution of about 100 ml. By adding 400 ml of ethyl acetate, a solid separates which is collected by filtration, washed with 200 ml of ethyl ether and dried in the air at room temperature overnight to yield the pure compounds of the title as the free bases.

Preparation of compound 16, 30.

By following the above procedure A, but using 2-[2-(2-chloroethoxy)ethoxy]ethanol (7.3 ml, about 50 mmol; Aldrich) as the reactant halide, a crude which contains compound 16 (or 30 depending on the starting material) is recovered after 24 h reaction at room temperature. The pure title compound is then obtained by reverse-phase column chromatography carried out as described for preparation of compound 14.

Examples 8-89

Procedure B: Reductive alkylation of a teicoplanin amide.

Preparation of compounds 1-3, 7, 8, 10-13, 17-19, 21, 22, 24-27, 31-37, 39-52, 54, 56-69, 72-91, 99-106

To a stirred suspension of 60 g (about 30 mmol) of the proper starting $N^{63}$ carboxyamide of teicoplanin $A_2$ or deglucoteicoplanin (see Table IIB) in 1.5 l methanol, 60 g (about 1.62 mol) of sodium borohydride is added portionwise while maintaining the temperature at about 45°C.

After about 90 min, 200 mmol of the proper carbonyl compound (see Table IIB) is added at room temperature and stirring is continued for 60 min. The reaction mixture is then cooled at 5°C and 30 g (about 0.81 mol) of sodium borohydride is added in one portion, while cooling at 10-15°C.

After about 30 min, 152 ml (about 2.64 mol) of glacial acetic acid is dropped in at room temperature and the resulting solution is evaporated at 40 °C under reduced pressure. The residue is re-dissolved in 1.5 l of water and loaded at the top of a column of 2.4 g of silanized silica-gel (particle size 0.06-0.2 mm; Merck Co.) in water. The column is developed with 3 l of water, then with 3 l of a mixture acetonitrile:water 2:8 (v/v), with 3 l of acetonitrile:water 1:1 (v/v), and finally with 7 l of a 1:1 (v/v) mixture acetonitrile: water adjusted to pH 3.0 with glacial acetic acid. Fractions (200 ml) are collected and monitored by HPLC.

Those fractions containing the pure title compound are pooled and enough n-butanol is added to obtain, after concentration of the resulting mixture at 40 °C under vacuum, a dry butanolic suspension of about 300 ml.

On adding 700 ml of ethyl ether, a solid separates which is collected by filtration, washed with 300 ml of ethyl ether and dried in vacuo at room temperature overnight to yield the title compounds, as the acetate.

Examples 90-93

Procedure C: Reaction of a $N^{15}$alkyl (or $N^{15}$,$N^{15}$dialkyl) derivative of teicoplanin with an amine

Preparation of compounds 1, 20, 53 and 55

To a stirred solution of 2 mmol of a proper $N^{15}$-alkyl or $N^{15}$-methyl-$N^{15}$-alkyl teicoplanin compound (see Table IIC) and of 5 mmol of the appropriate di-amine, di-hydrochloride in 50 ml of DMF, 13 mmol of TEA is added which is followed by 0.6 ml (2.8 mmol) of diphenylphosphorazide (DPPA). The reaction mixture is stirred at room temperature overnight, then it is poured into 500 ml of ethyl ether. The precipitate which forms is collected by filtration and re-dissolved in 150 ml of a mixture acetonitrile:water 1:9 (v/v), which is adjusted to pH 3 by glacial acetic acid. The resulting solution is then loaded on a column of 350 g of silanized Silica-gel (0.06-0.2 mm); Merck) in water. Chromatography is carried out as previously reported in Examples 1-7, to yield the title compounds, as the free bases.

Examples 94-98

Procedure D: Reductive alkylation of a $N^{63}$teicoplanin amide with a carbonyl compound in order to obtain a $N^{15}$,$N^{15}$dialkyl derivative

Preparation of compounds 4, 5, 9 and 23.

To a stirred suspension of 1 mmol of compound 1, 2, 7, 21 respectively in 100 ml of methanol, 4 ml (about 53 mmol) of 40% (by weight) aqueous formaldehyde and 0.4 ml (about 7 mmol) of anhydrous formic acid are added. The clear solution which forms is warmed to 30 °C and 0.4 g (about 15 mmol) of sodium borohydride is added portionwise, while maintaining the reaction temperature at 35-40 °C. After 60 min, 1.25 ml (about 20 mmol) of glacial acetic acid is added at room temperature and the solvents are evaporated at 40 °C under reduced pressure. The residue is re-dissolved in water (about 100 ml) and purified by reverse-phase column chromatography as described in examples 1-7 to give the title compounds.

Preparation of compound 38

By essentially following the same procedure for the preparation of compound 4 above but starting from a compound of formula I wherein A, B, M, are the sugar moieties as defined and $R^1$ and $R^2$ are replaced by two hydrogen atoms and Y represents $-NH(CH_2)_2-N(CH_3)_2$ the corresponding $N^{15}$, $N^{15}$ dimethyl derivative is obtained.

Examples 99-101

Procedure E: Selective hydrolysis

Preparation of compounds 6, 15 and 29.

A suspension of 1 mmol of the selected alkyl amide derivative of teicoplanin $A_2$ (compound 4, 14 or 28) in 50 ml of absolute trifluoroethanol is stirred at 75 °C for 18 h, while bubbling dry hydrochloric acid, then it is cooled to 10 °C and left at such a temperature overnight. By adding 20 ml of ethyl ether the crude

compounds of the title are recovered from the reaction mixture as dark yellow powders which are purified by reverse-phase column chromatography as described in Examples 1-7.

Examples 102-108

Procedure G: Catalytic hydrogenolysis

Preparation of compounds 92-98.

The respective compounds 85-91 are treated by catalytic hydrogenolysis, as follows:

1 mmol of the proper derivative (see Table IIF) is dissolved in 200 ml of a mixture methanol:0.01N HCl 7:3 (v/v). The resulting solution is hydrogenated at room temperature and 1 atm over 1 g of 5% Pd/C. As soon as 90-110 ml of $H_2$ is absorbed, the catalyst is filtered off and the filtrate is adjusted at pH 7.5 with 1N NaOH. Most of the methanol is then evaporated at 40°C under reduced pressure and the resulting cloudy solution is loaded at the top of a column of 200 g of silanized Silica gel (0.06-0.2 mm; Merck). Chromatography is then carried out under the same conditions as above described, to yield the compounds of the title, as the free bases.

Acid addition salts of the above compounds as well as of compounds of the invention in general are prepared by dissolving the proper derivative, as the free base, in a small volume (generally 5 ml per 1 mmol) of DMF containing a stoichiometric amount of the appropiate organic or mineral acid. Precipitation with ethyl ether yields the desired product.

**Claims**

**Claims for the following Contracting States : AT, BE, CH, DE, FR, GB, IT, LI, LU, NL, SE**

**1.** An amide of $N^{15}$-alkyl or $N^{15}$,$N^{15}$-dialkyl teicoplanin compound having the following formula I

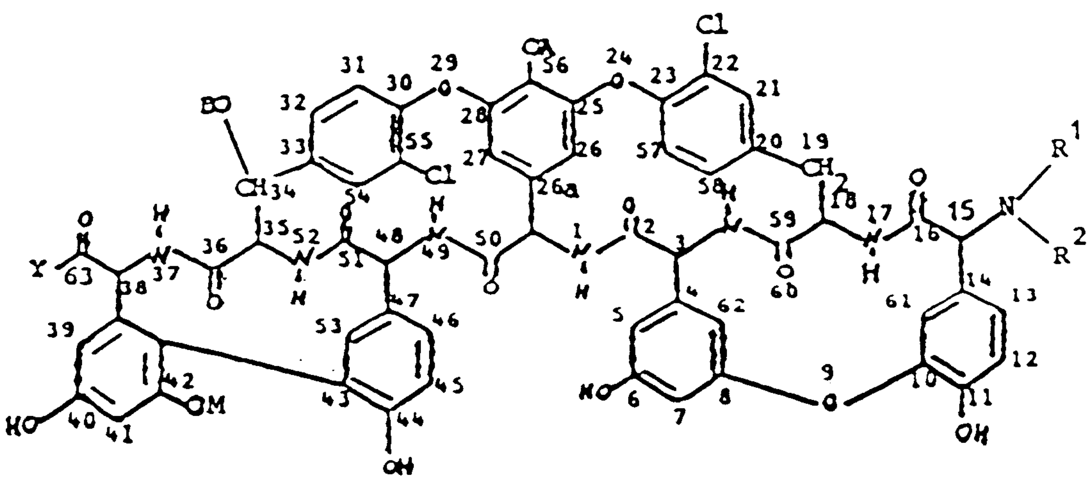

I

wherein:

$R^1$ is H or a $(C_1-C_3)$alkyl

$R^2$ represents a group $[CHR^3(CR^4R^5)_mX]_p-(CH_2)_n-R^6$

wherein:

$R^3$ and $R^4$ independently represent H or a $(C_1-C_6)$alkyl;

$R^5$ represents H, a $(C_1-C_6)$alkyl or OH;

$R^6$ represents H, a $(C_1-C_3)$alkyl, $COOR^7$, $OR^7$, $SR^7$, $NR^7R^8$ or halogen;

$R^7$ and $R^8$ independently represent H or a $(C_1-C_3)$alkyl;

m is zero or 1, n is an integer from zero to 6, p is an integer from 1 to 6,

X is O, NH or a bond with the proviso that when X is O or NH, then n is different from zero, p ranges between 1 and 3, and $R^5$ is different from OH

Y represents a group

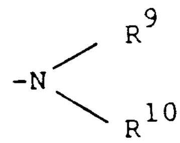

wherein:

$R^9$ represents H or a $C_1$-$C_4$ alkyl; hydroxy($C_2$-$C_4$)alkyl, amino($C_2$-$C_4$)alkyl, ($C_1$-$C_3$)alkylamino-($C_2$-$C_4$)alkyl;

$R^{10}$ represents H, $C_1$-$C_6$ alkyl, hydroxy($C_2$-$C_4$)alkyl, halogen($C_2$-$C_4$)alkyl, carboxy($C_1$-$C_3$)alkyl, carboxamido($C_1$-$C_3$)alkyl, ($C_1$-$C_3$)alkylcarboxy($C_1$-$C_3$)alkylamino, ($C_1$-$C_3$)alkoxycarbonyl($C_1$-$C_3$)alkyl, phenyl($C_1$-$C_3$)alkoxycarbonyl($C_1$-$C_3$)alkyl, a group

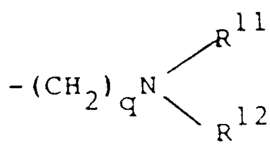

or a group

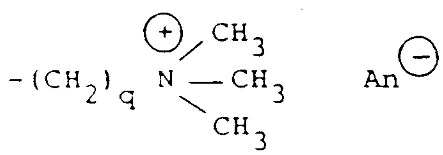

in which q is an integer ranging from 2 to 8, and one of the hydrogen atoms of the methylene unit is optionally substituted with a substituent selected from ($C_1$-$C_3$)alkyl, carboxy, ($C_1$-$C_4$)alkoxycarbonyl and carboxamido and $An^{\ominus}$ is a pharmaceutically acceptable anion,

$R^{11}$ represents H or a ($C_1$-$C_4$)alkyl, hydroxy($C_2$-$C_4$)alkyl, halogen($C_2$-$C_4$)alkyl,

$R^{12}$ represents H, ($C_1$-$C_6$)alkyl, hydroxy($C_2$-$C_4$)alkyl, halogen($C_2$-$C_4$)alkyl, ($C_1$-$C_3$)alkoxy($C_1$-$C_4$)-alkyl, phenyl($C_1$-$C_4$)alkoxycarbonyl, ($C_1$-$C_4$)alkyloxycarbonyl

or $R^{11}$ and $R^{12}$ taken together with the adjacent nitrogen atom represent a saturated 5-7 membered heterocyclic ring which may optionally bear one to two ($C_1$-$C_4$)alkyl substituents on the ring carbons and may contain a further heterogroup selected from -O-, -S-, and -$NR^{13}$-wherein $R^{13}$ is H or a ($C_1$-$C_4$)-alkyl,

or $R^9$ and $R^{10}$ taken together with the adjacent nitrogen atom represent a saturated 5-7 membered heterocyclic ring which may optionally bear one to two ($C_1$-$C_4$)alkyl substituents on the ring carbons and may contain a further heterogroup selected from -O-, -S-, and -$NR^{14}$-wherein $R^{14}$ is H or a ($C_1$-$C_4$)-alkyl

A represents hydrogen or -N[($C_9$-$C_{12}$)aliphatic acyl] -beta-D-2-deoxy-2-amino-glucopyranosyl,

B represents hydrogen or N-acetyl-beta-D-2-deoxy-2-amino-glucopyranosyl,

M represents hydrogen or alpha-D-mannopyranosyl;

with the proviso that B represents hydrogen only when A and M are simultaneously hydrogen and the addition salts thereof.

**2.** A compound as in claim 1 wherein:

$R^1$ is H or a ($C_1$-$C_3$)alkyl

$R^2$ represents a group $[CHR^3(CR^4R^5)_mX]_p$-$(CH_2)_n$-$R^6$

wherein:

$R^3$ and $R^4$ independently represent H or a ($C_1$-$C_6$)alkyl;

$R^5$ represents H, a ($C_1$-$C_6$)alkyl or OH;

$R^6$ represents H, a ($C_1$-$C_3$)alkyl, $COOR^7$, $OR^7$, $SR^7$, $NR^7R^8$ or halogen;

$R^7$ and $R^8$ independently represent H or a ($C_1$-$C_3$)alkyl;

m is zero or 1, n is an integer from zero to 6, p is an integer from 1 to 6,.

X is O, NH or a bond with the proviso that when X is O or NH, then n is different from zero, p ranges between 1 and 3, and $R^5$ is different from OH,

Y represents a group

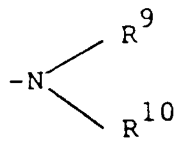

wherein:

$R^9$ represents H or a $C_1$-$C_4$ alkyl;

$R^{10}$ represents H, $C_1$-$C_6$ alkyl, hydroxy($C_2$-$C_4$)alkyl, halogen($C_2$-$C_4$)alkyl or a group

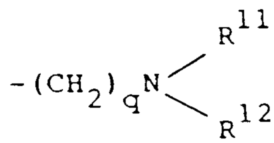

in which q is an integer ranging from 2 to 8, and one of the hydrogen atoms of a methylene unit is optionally substituted with a substituent selected from carboxy, and ($C_1$-$C_4$)-alkoxycarbonyl,

$R^{11}$ represents H or a ($C_1$-$C_4$)alkyl

$R^{12}$ represents H, ($C_1$-$C_6$)alkyl, hydroxy($C_2$-$C_4$)alkyl, halogen($C_2$-$C_4$)alkyl, ($C_1$-$C_3$)alkoxy($C_1$-$C_4$)-alkyl, phenyl($C_1$-$C_4$)alkoxycarbonyl, ($C_1$-$C_4$)alkyloxycarbonyl

or $R^{11}$ and $R^{12}$ taken together with the adjacent nitrogen atom represent a saturated 5-7 membered heterocyclic ring which may optionally bear one to two ($C_1$-$C_4$)alkyl substituents on the ring carbons and may contain a further heterogroup selected from -O-, -S-, and -$NR^{13}$-wherein $R^{13}$ is H or a ($C_1$-$C_4$)-alkyl

or $R^9$ and $R^{10}$ taken together with the adjacent nitrogen atom represent a saturated 5-7 membered heterocyclic ring which may optionally bear one to two ($C_1$-$C_4$)alkyl substituents on the ring carbons and may contain a further heterogroup selected from -O-, -S-, and -$NR^{14}$-wherein $R^{14}$ is H or a ($C_1$-$C_4$)-alkyl

A represents hydrogen or -N[($C_9$-$C_{12}$) aliphatic acyl]-beta-D-2-deoxy-2-amino-glucopyranosyl,

B represents hydrogen or N-acetyl-beta-D-2-deoxy-2-amino-glucopyranosyl,

M represents hydrogen or alpha-D-mannopyranosyl;

with the proviso that B represents hydrogen only when A and M are simultaneously hydrogen and the addition salts thereof.

**3.** A compound as in claim 1 wherein $R^1$ is hydrogen.

**4.** A compound as in claim 1 wherein $R^1$ is methyl.

**5.** A compound as in claim 1 wherein:

$R^1$ is H or a ($C_1$-$C_3$)alkyl

$R^2$ represents a group $[CHR^3(CR^4R^5)_mX]_p(CH_2)_n$-$R^6$

wherein:

$R^3$ and $R^4$ independently represent H or a ($C_1$-$C_4$)alkyl;

$R^5$ represents H, a ($C_1$-$C_4$)alkyl or OH;

$R^6$ represents H, a ($C_1$-$C_3$)alkyl, $OR^7$, $SR^7$, or $NR^7R^8$;

$R^7$ and $R^8$ independently represent H or a ($C_1$-$C_3$)alkyl,

m is zero or 1, n is an integer from zero to 3, p is an integer from 1 to 3,

X is O, NH or a bond with the proviso that when X is O or NH, then n is different from zero, p is 1 or 2, and $R^5$ is different from OH,

Y represents a group

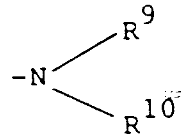

wherein:

$R^9$ represents H or a $(C_1\text{-}C_4)$alkyl

$R^{10}$ represents H, $(C_1\text{-}C_4)$alkyl, hydroxy $(C_2\text{-}C_4)$alkyl or a group

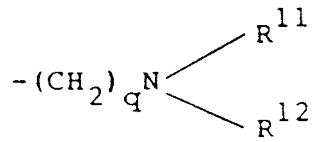

in which q is an integer ranging from 2 to 8, and one of the hydrogen atoms of a methylene unit is optionally substituted with a substituent selected from carboxy, and $(C_1\text{-}C_3)$-alkoxycarbonyl,

$R^{11}$ represents H or a $(C_1\text{-}C_4)$alkyl

$R^{12}$ represents H, $(C_1\text{-}C_6)$alkyl, hydroxy$(C_2\text{-}C_4)$alkyl, $(C_1\text{-}C_3)$alkoxy$(C_1\text{-}C_4)$alkyl, phenyl$(C_1\text{-}C_4)$-alkoxycarbonyl, $(C_1\text{-}C_4)$alkyloxycarbonyl

or $R^9$ and $R^{10}$ taken together with the adjacent nitrogen atom represent a saturated 5-7 membered heterocyclic ring which may optionally bear one to two $(C_1\text{-}C_4)$alkyl substituents on the ring carbons and may contain a further heterogroup selected from -O-, -S-, and $-NR^{14}$-wherein $R^{14}$ is H or a $C_1\text{-}C_4$ alkyl

A represents hydrogen or -N[$(C_9\text{-}C_{12})$aliphatic acyl]-beta-D-2-deoxy-2-amino-glucopyranosyl,

B represents hydrogen or N-acetyl-beta-D-2-deoxy-2-amino-glucopyranosyl,

M represents hydrogen or alpha-D-mannopyranosyl;

with the proviso that B represents hydrogen only when A and M are simultaneously hydrogen and the addition salts thereof.

6. A compound as in claim 1 wherein

$R^1$ is H or $CN_3$, $R^2$ represents a group as defined wherein

$R^3$ is H or $CH_3$, $R^4$ is H or $CH_3$; $R^5$ is H or OH; m is zero or 1, n ranges from 0 to 2, p is 1 or 2, $R^6$ is H, $OR^7$ or $NR^7R^8$ and $R^7$ and $R^8$ are independently H or $CH_3$

Y represents a group

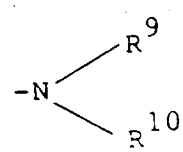

wherein:

$R^9$ represents H or a $(C_1\text{-}C_4)$alkyl

$R^{10}$ represents H, $(C_1\text{-}C_4)$alkyl, hydroxy$(C_2\text{-}C_4)$alkyl or a group

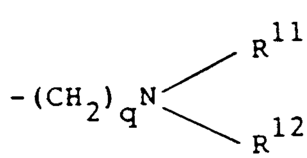

in which q is an integer ranging from 2 to 8, and one of the hydrogen atoms of a methylene unit is optionally substituted with a substituent selected from carboxy, and $(C_1\text{-}C_3)$-

alkoxycarbonyl,

$R^{11}$ represents H or a $(C_1-C_4)$alkyl

$R^{12}$ represents H, $(C_1-C_6)$alkyl, hydroxy$(C_2-C_4)$alkyl, halogeno$(C_2-C_4)$alkyl, $(C_1-C_3)$alkoxy$(C_1-C_4)$-alkyl, phenyl$(C_1-C_4)$alkoxycarbonyl, $(C_1-C_4)$alkyloxycarbonyl

or $R^{11}$ and $R^{12}$ taken together with the adjacent nitrogen atom represent a saturated 5-7 membered heterocyclic ring which may optionally bear one to two $(C_1-C_4)$alkyl substituents on the ring carbons and may contain a further heterogroup selected from -O-, -S-, and $NR^{13}$-wherein $R^{13}$ is H or a $(C_1-C_4)$-alkyl

or $R^9$ and $R^{10}$ taken together with the adjacent nitrogen atom represent a saturated 5-7 membered heterocyclic ring which may optionally bear one to two $(C_1-C_4)$alkyl substituents on the ring carbons and may contain a further heterogroup selected from -O-, -S-, and $-NR^{14}$-wherein $R^{14}$ is H or a $(C_1-C_4)$-alkyl

A represents hydrogen or -N[$(C_9-C_{12})$aliphatic acyl]-beta-D-2-deoxy-2-amino-glucopyranosyl,

B represents hydrogen or N-acetyl-beta-D-2-deoxy-2-amino-glucopyranosyl,

M represents hydrogen or alpha-D-mannopyranosyl;

with the proviso that B represents hydrogen only when A and M are simultaneously hydrogen and the addition salts thereof.

**7.** A process for preparing a compound of claim 1 which comprises reacting a compound of formula II

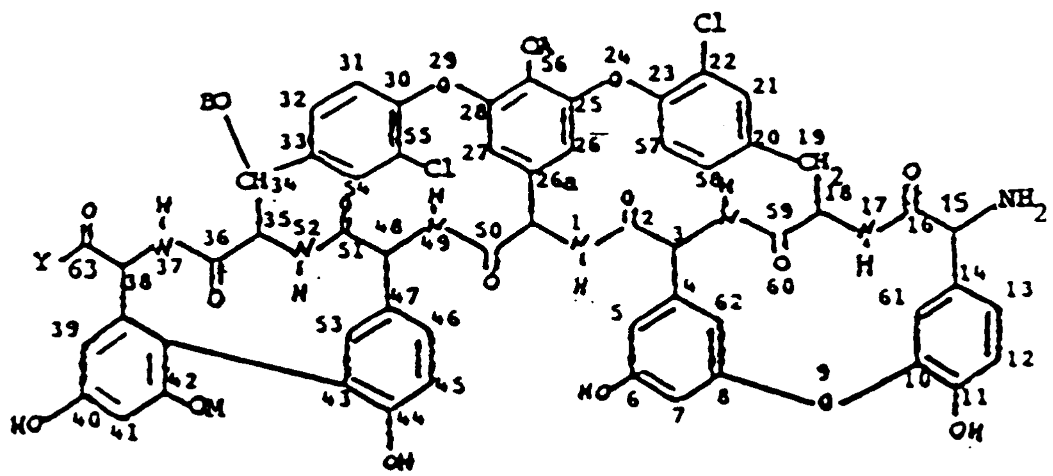

II

wherein:

Y represents a group

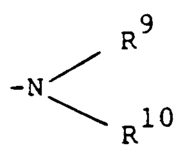

wherein:

$R^9$ represents H or a $C_1-C_4$ alkyl; hydroxy$(C_2-C_4)$alkyl, amino$(C_2-C_4)$alkyl, $(C_1-C_3)$alkylamino-$(C_2-C_4)$alkyl;

$R^{10}$ represents H, $C_1-C_6$ alkyl, hydroxy$(C_2-C_4)$alkyl, halogen$(C_2-C_4)$alkyl, carboxy$(C_1-C_3)$alkyl, carboxamido$(C_1-C_3)$alkyl, $(C_1-C_3)$alkylcarboxy$(C_1-C_3)$-alkylamido, $(C_1-C_3)$alkoxycarbonyl$(C_1-C_3)$alkyl, phenyl$(C_1-C_3)$alkoxycarbonyl$(C_1-C_3)$alkyl, a group

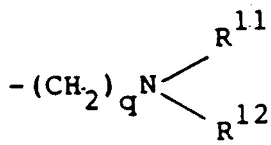

or a group

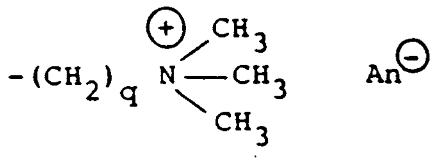

in which q is an integer ranging from 2 to 8, and one of the hydrogen atoms of the methylene unit is optionally substituted with a substituent selected from $(C_{1-3})$alkyl, carboxy, $(C_1$-$C_4)$alkoxycarbonyl, and carboxamido and $An^{\ominus}$ is a pharmaceutically acceptable anion

$R^{11}$ represents H or a $(C_1$-$C_4)$alkyl, hydroxy$(C_2$-$C_4)$alkyl, halogen$(C_2$-$C_4)$alkyl

$R^{12}$ represents H, $(C_1$-$C_6)$alkyl, hydroxy$(C_2$-$C_4)$alkyl, halogen$(C_2$-$C_4)$alkyl, $(C_1$-$C_3)$alkoxy$(C_1$-$C_4)$-alkyl, phenyl$(C_1$-$C_4)$alkoxycarbonyl, $(C_1$-$C_4)$alkyloxycarbonyl or $R^{11}$ and $R^{12}$ taken together with the adjacent nitrogen atom represent a saturated 5-7 membered heterocyclic ring which may optionally bear one to two $(C_1$-$C_4)$alkyl substituents on the ring carbons and may contain a further heterogroup selected from -O-, -S-, and -$NR^{13}$-wherein $R^{13}$ is H or a $(C_1$-$C_4)$alkyl

or $R^9$ and $R^{10}$ taken together with the adjacent nitrogen atom represent a saturated 5-7 membered heterocyclic ring which may optionally bear one to two $(C_1$-$C_4)$alkyl substituents on the ring carbons and may contain a further hetero group selected from -O-, -S-, and -$NR^{14}$-wherein $R^{14}$ is H or a $(C_1$-$C_4)$alkyl, A represents hydrogen or -N[$(C_9$-$C_{12})$aliphatic acyl]-beta-D-2-deoxy-2-amino-glucopyranosyl, B represents hydrogen or N-acetyl-beta-D-2-deoxy-2-amino-glucopyranosyl,

M represents hydrogen or alpha-D-mannopyranosyl, with the proviso that B may represent hydrogen only when A and M are simultaneously hydrogen, a salt thereof, or a mixture thereof in any proportion, with

a) an alkyl halide of formula $R^1$(halo) or $R^2$(halo), wherein:

$R^1$ is H or a $(C_1$-$C_3)$alkyl

$R^2$ represents a group $[CHR^3(CR^4R^5)_mX]_p$-$(CH_2)_n$-$R^6$

wherein:

$R^3$ and $R^4$ independently represent H or a $(C_1$-$C_6)$alkyl;

$R^5$ represents H, a $(C_1$-$C_6)$alkyl or OH;

$R^6$ represents H, a $(C_1$-$C_3)$alkyl, $COOR^7$, $OR^7$, $SR^7$, $NR^7R^8$ or halogen;

$R^7$ and $R^8$ independently represent H or a $(C_1$-$C_3)$alkyl;

m is zero or 1, n is an integer from zero to 6, p is an integer from 1 to 6,

X is O, NH or a bond with the proviso that when X is O or NH, then n is different from zero, p ranges between 1 and 3, and $R^5$ is different from OH

and (halo) represents chloro or bromo, in the presence of a base and possibly of an organic solvent

b) alternatively, a molar excess of a carbonyl compound of at least 20 times with respect to teicoplanin starting material, in an organic polar aprotic solvent with a reducing agent.

**8.** A process as in claim 7 wherein the carbonyl compound has the same skeleton of substituent $R^2$ (or $R^1$) and whose oxo group is on the carbon atom which is to link the $N^{15}$ amino group of teicoplanin moiety.

**9.** A process as in claim 7 wherein the reducing agent is an alkali metal borohydride.

**10.** A compound as in claim 1 for use as a medicine.

**11.** Use of a substance of claim 1 for the manufacture of a medicament for use as antibacterial.

12. A pharmaceutical composition comprising a compound of claim 1 in admixture with a pharmaceutically acceptable carrier.

**Claims for the following Contracting States : GR, ES**

1. A process for preparing an amide of $N^{15}$-alkyl or $N^{15},N^{15}$-dialkyl teicoplanin compound having the following formula I

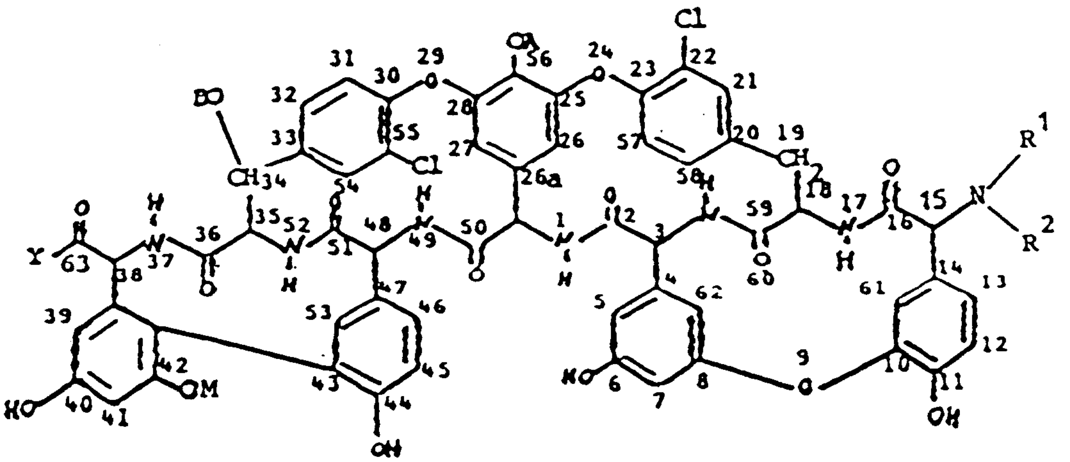

I

wherein:

$R^1$ is H or a $(C_1-C_3)$alkyl

$R^2$ represents a group $[CHR^3(CR^4R^5)_mX]_p-(CH_2)_n-R^6$

wherein:

$R^3$ and $R^4$ independently represent H or a $(C_1-C_6)$alkyl;

$R^5$ represents H, a $(C_1-C_6)$alkyl or OH;

$R^6$ represents H, a $(C_1-C_3)$alkyl, $COOR^7$, $OR^7$, $SR^7$, $NR^7R^8$ or halogen;

$R^7$ and $R^8$ independently represent H or a $(C_1-C_3)$alkyl;

m is zero or 1, n is an integer from zero to 6, p is an integer from 1 to 6,

X is O, NH or a bond with the proviso that when X is O or NH, then n is different from zero, p ranges between 1 and 3, and $R^5$ is different from OH

Y represents a group

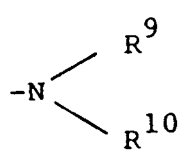

wherein:

$R^9$ represents H or a $C_1-C_4$ alkyl; hydroxy$(C_2-C_4)$alkyl, amino$(C_2-C_4)$alkyl, $(C_1-C_3)$alkylamino-$(C_2-C_4)$alkyl;

$R^{10}$ represents H, $C_1-C_6$ alkyl, hydroxy$(C_2-C_4)$alkyl, halogen$(C_2-C_4)$alkyl, carboxy$(C_1-C_3)$alkyl, carboxamido$(C_1-C_3)$alkyl, $(C_1-C_3)$alkylcarboxy$(C_1-C_3)$alkylamino, $(C_1-C_3)$alkoxycarbonyl$(C_1-C_3)$alkyl, phenyl$(C_1-C_3)$alkoxycarbonyl$(C_1-C_3)$alkyl, a group

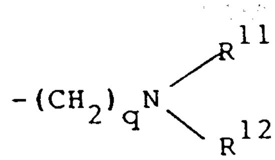

or a group

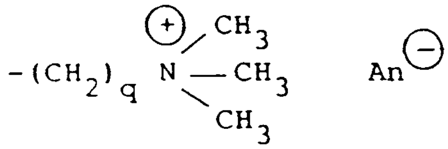

in which q is an integer ranging from 2 to 8, and one of the hydrogen atoms of the methylene unit is optionally substituted with a substituent selected from $(C_1-C_3)$alkyl, carboxy, $(C_1-C_4)$alkoxycarbonyl and carboxamido and $An^{\ominus}$ is a pharmaceutically acceptable anion,

$R^{11}$ represents H or 3 $(C_1-C_4)$alkyl, hydroxy$(C_2-C_4)$alkyl, halogen$(C_2-C_4)$alkyl,

$R^{12}$ represents H, $(C_1-C_6)$alkyl, hydroxy$(C_2-C_4)$alkyl, halogen$(C_2-C_4)$alkyl, $(C_1-C_3)$alkoxy$(C_1-C_4)$-alkyl, phenyl$(C_1-C_4)$alkoxycarbonyl, $(C_1-C_4)$alkyloxycarbonyl

or $R^{11}$ and $R^{12}$ taken together with the adjacent nitrogen atom represent a saturated 5-7 membered heterocyclic ring which may optionally bear one to two $(C_1-C_4)$alkyl substituents on the ring carbons and may contain a further heterogroup selected from -O-, -S-, and $-NR^{13}$-wherein $R^{13}$ is H or a $(C_1-C_4)$-alkyl,

or $R^9$ and $R^{10}$ taken together with the adjacent nitrogen atom represent a saturated 5-7 membered heterocyclic ring which may optionally bear one to two $(C_1-C_4)$alkyl substituents on the ring carbons and may contain a further heterogroup selected from -O-, -S-, and $-NR^{14}$-wherein $R^{14}$ is H or a $(C_1-C_4)$-alkyl

A represents hydrogen or $-N[(C_9-C_{12})$aliphatic acyl] -beta-D-deoxy-2-amino-glucopyranosyl,

B represents hydrogen or N-acetyl-beta-D-2-deoxy-2-amino-glucopyranosyl,

M represents hydrogen or alpha-D-mannopyranosyl;

with the proviso that B represents hydrogen only when A and M are simultaneously hydrogen and the addition salts thereof,

which comprises reacting a compound of formula II

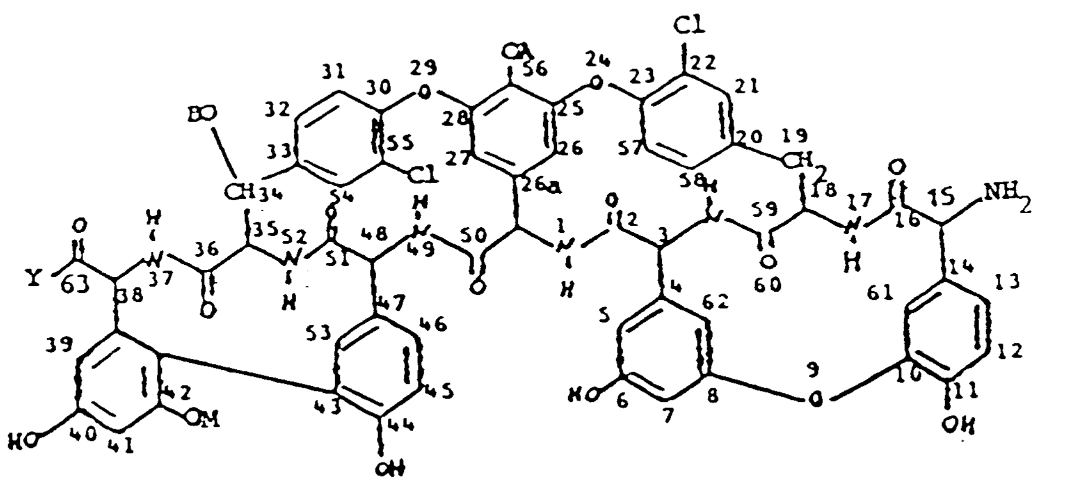

II

wherein: Y, A, B, and M are defined as above with:

a) an alkyl halide of formula $R^1$(halo) or $R^2$(halo), wherein:

$R^1$ is H or a $(C_1-C_3)$alkyl

$R^2$ represents a group $[CHR^3(CR^4R^5)_mX]_p-(CH_2)_n-R^6$

wherein:

$R^3$ and $R^4$ independently represent H or a $(C_1-C_6)$alkyl;

$R^5$ represents H, a $(C_1-C_6)$alkyl or OH;

$R^6$ represents H, a $(C_1-C_3)$alkyl, $COOR^7$, $OR^7$, $SR^7$, $NR^7R^8$ or halogen;

$R^7$ and $R^8$ independently represent H or a $(C_1-C_3)$alkyl;

m is zero or 1, n is an integer from zero to 6, p is an integer from 1 to 6,

X is O, NH or a bond with the proviso that when X is O or NH, then n is different from zero, p

ranges between 1 and 3, and $R^5$ is different from OH
and (halo) represents chloro or bromo, in the presence of a base and possibly of an organic solvent
b) alternatively, a molar excess of a carbonyl compound of at least 20 times with respect to teicoplanin starting material, in an organic polar aprotic solvent with a reducing agent, said carbonyl compound having the same carbon skeleton as the desired substituent $R^2$ (or $R^1$) and whose oxo group is on the carbon atom which is to link the $N^{15}$ amino group of teicoplanin moiety.

**2.** A process according to claim 1 for preparing a compound of formula I wherein:
$R^1$ is H or a $(C_1\text{-}C_3)$alkyl
$R^2$ represents a group $[CHR^3(CR^4R^5)_mX]_p\text{-}(CH_2)_n\text{-}R^6$
wherein:
$R^3$ and $R^4$ independently represent H or a $(C_1\text{-}C_6)$alkyl;
$R^5$ represents H, a $(C_1\text{-}C_6)$alkyl or OH;
$R^6$ represents H, a $(C_1\text{-}C_3)$alkyl, $COOR^7$, $OR^7$, $SR^7$, $NR^7R^8$ or halogen;
$R^7$ and $R^8$ independently represent H or a $(C_1\text{-}C_3)$alkyl;
m is zero or 1, n is an integer from zero to 6, p is an integer from 1 to 6,
X is O, NH or a bond with the proviso that when X is O or NH, then n is different from zero, p ranges between 1 and 3, and $R^5$ is different from OH,
Y represents a group

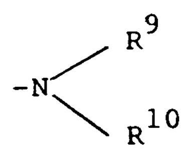

wherein:
$R^9$ represents H or a $C_1\text{-}C_4$ alkyl;
$R^{10}$ represents H, $C_1\text{-}C_6$ alkyl, hydroxy$(C_2\text{-}C_4)$alkyl, halogen$(C_2\text{-}C_4)$alkyl or a group

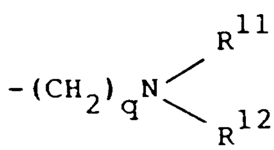

in which q is an integer ranging from 2 to 8, and one of the hydrogen atoms of a methylene unit is optionally substituted with a substituent selected from carboxy, and $(C_1\text{-}C_4)$-alkoxycarbonyl,
$R^{11}$ represents H or a $(C_1\text{-}C_4)$alkyl
$R^{12}$ represents H, $(C_1\text{-}C_6)$alkyl, hydroxy$(C_2\text{-}C_4)$alkyl, halogen$(C_2\text{-}C_4)$alkyl, $(C_1\text{-}C_3)$alkoxy$(C_1\text{-}C_4)$-alkyl, phenyl$(C_1\text{-}C_4)$alkoxycarbonyl, $(C_1\text{-}C_4)$alkyloxycarbonyl
or $R^{11}$ and $R^{12}$ taken together with the adjacent nitrogen atom represent a saturated 5-7 membered heterocyclic ring which may optionally bear one to two $(C_1\text{-}C_4)$alkyl substituents on the ring carbons and may contain a further heterogroup selected from -O-, -S-, and $\text{-}NR^{13}$-wherein $R^{13}$ is H or a $(C_1\text{-}C_4)$-alkyl
or $R^9$ and $R^{10}$ taken together with the adjacent nitrogen atom represent a saturated 5-7 membered heterocyclic ring which may optionally bear one to two $(C_1\text{-}C_4)$alkyl substituents on the ring carbons and may contain a further heterogroup selected from -O-, -S-, and $\text{-}NR^{14}$-wherein $R^{14}$ is H or a $(C_1\text{-}C_4)$-alkyl
A represents hydrogen or $\text{-}N[(C_9\text{-}C_{12})$aliphatic acyl]-beta-D-2-deoxy-2-amino-glucopyranosyl,
B represents hydrogen or N-acetyl-beta-D-2-deoxy-2-amino-glucopyranosyl,
M represents hydrogen or alpha-D-mannopyranosyl;
with the proviso that B represents hydrogen only when A and M are simultaneously hydrogen and the addition salts thereof.

**3.** A process according to claim 1 wherein $R^1$ is hydrogen.

**4.** A process according to claim 1 wherein $R^1$ is methyl.

**5.** A process according to claim 1 wherein:

$R^1$ is H or a $(C_1\text{-}C_3)$alkyl

$R^2$ represents a group $[CHR^3(CR^4R^5)_mX]_p\text{-}(CH_2)_n\text{-}R^6$

wherein:

$R^3$ and $R^4$ independently represent H or a $(C_1\text{-}C_4)$alkyl;

$R^5$ represents H, a $(C_1\text{-}C_4)$alkyl or OH;

$R^6$ represents H, a $(C_1\text{-}C_3)$alkyl, $OR^7$, $SR^7$, or $NR^7R^8$;

$R^7$ and $R^8$ independently represent H or a $(C_1\text{-}C_3)$alkyl,

m is zero or 1, n is an integer from zero to 3, p is an integer from 1 to 3,

X is O, NH or a bond with the proviso that when X is O or NH, then n is different from zero, p is 1 or 2, and $R^5$ is different from OH,

Y represents a group

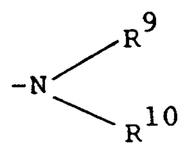

wherein:

$R^9$ represents H or a $(C_1\text{-}C_4)$ alkyl

$R^{10}$ represents H, $(C_1\text{-}C_4)$alkyl, hydroxy$(C_2\text{-}C_4)$alkyl or a group

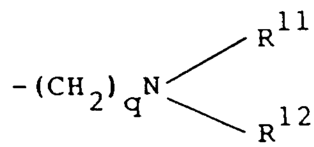

in which q is an integer ranging from 2 to 8, and one of the hydrogen atoms of a methylene unit is optionally substituted with a substituent selected from carboxy, and $(C_1\text{-}C_3)$-alkoxycarbonyl,

$R^{11}$ represents H or a $(C_1\text{-}C_4)$alkyl

$R^{12}$ represents H, $(C_1\text{-}C_6)$alkyl, hydroxy$(C_2\text{-}C_4)$alkyl, $(C_1\text{-}C_3)$alkoxy$(C_1\text{-}C_4)$alkyl, phenyl$(C_1\text{-}C_4)$-alkoxycarbonyl, $(C_1\text{-}C_4)$alkyloxycarbonyl

or $R^9$ and $R^{10}$ taken together with the adjacent nitrogen atom represent a saturated 5-7 membered heterocyclic ring which may optionally bear one to two $(C_1\text{-}C_4)$alkyl substituents on the ring carbons and may contain a further heterogroup selected from -O-, -S-, and $\text{-}NR^{14}$-wherein $R^{14}$ is H or a $C_1\text{-}C_4$ alkyl

A represents hydrogen or -N[$(C_9\text{-}C_{12})$aliphatic acyl]-beta-D-2-deoxy-2-amino-glucopyranosyl,

B represents hydrogen or N-acetyl-beta-D-2-deoxy-2-amino-glucopyranosyl,

M represents hydrogen or alpha-D-mannopyranosyl;

with the proviso that B represents hydrogen only when A and M are simultaneously hydrogen and the addition salts thereof.

**6.** A process according to claim 1 wherein

$R^1$ is H or $CH_3$, $R^2$ represents a group as defined wherein

$R^3$ is H or $CH_3$, $R^4$ is H or $CH_3$; $R^5$ is H or OH; m is zero or 1, n ranges from 0 to 2, p is 1 or 2, $R^6$ is H, $OR^7$ or $NR^7R^8$ and $R^7$ and $R^8$ are independently H or $CH_3$

Y represents a group

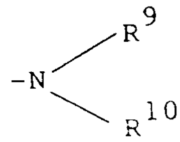

wherein:

$R^9$ represents H or a $(C_1\text{-}C_4)$alkyl

$R^{10}$ represents H, $(C_1\text{-}C_4)$alkyl, hydroxy$(C_2\text{-}C_4)$alkyl or a group

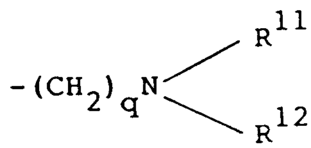

in which q is an integer ranging from 2 to 8, and one of the hydrogen atoms of a methylene unit is optionally substituted with a substituent selected from carboxy, and $(C_1\text{-}C_3)$-alkoxycarbonyl,

$R^{11}$ represents H or a $(C_1\text{-}C_4)$alkyl

$R^{12}$ represents H, $(C_1\text{-}C_6)$alkyl, hydroxy$(C_2\text{-}C_4)$alkyl, halogeno$(C_2\text{-}C_4)$alkyl, $(C_1\text{-}C_3)$alkoxy$(C_1\text{-}C_4)$-alkyl, phenyl$(C_1\text{-}C_4)$alkoxycarbonyl, $(C_1\text{-}C_4)$alkyloxycarbonyl

or $R^{11}$ and $R^{12}$ taken together with the adjacent nitrogen atom represent a saturated 5-7 membered heterocyclic ring which may optionally bear one to two $(C_1\text{-}C_4)$alkyl substituents on the ring carbons and may contain a further heterogroup selected from -O-, -S-, and $NR^{13}$-wherein $R^{13}$ is H or a $(C_1\text{-}C_4)$-alkyl

or $R^9$ and $R^{10}$ taken together with the adjacent nitrogen atom represent a saturated 5-7 membered heterocyclic ring which may optionally bear one to two $(C_1\text{-}C_4)$alkyl substituents on the ring carbons and may contain a further heterogroup selected from -O-, -S-, and $-NR^{14}$-wherein $R^{14}$ is H or a $(C_1\text{-}C_4)$-alkyl

A represents hydrogen or -N[$(C_9\text{-}C_{12})$aliphatic acyl]-beta-D-2-deoxy-2-amino-glucopyranosyl,

B represents hydrogen or N-acetyl-beta-D-2-deoxy-2-amino-glucopyranosyl,

M represents hydrogen or alpha-D-mannopyranosyl;

with the proviso that B represents hydrogen only when A and M are simultaneously hydrogen and and the addition salts thereof.

**7.** A process according to any of claims 1 to 6 wherein the reducing agent is an alkali metal borohydride.

**8.** Use of a compound of any of claims 1 to 6 for the manufacture of a medicament for use as antibacterial.

**Patentansprüche**

**Patentansprüche für folgende Vertragsstaaten : AT, BE, CH, DE, FR, GB, IT, LI, LU, NL, SE**

1. Amid einer $N^{15}$-Alkyl- oder $N^{15}$,$N^{15}$-Dialkylteicoplaninverbindung der folgenden Formel I

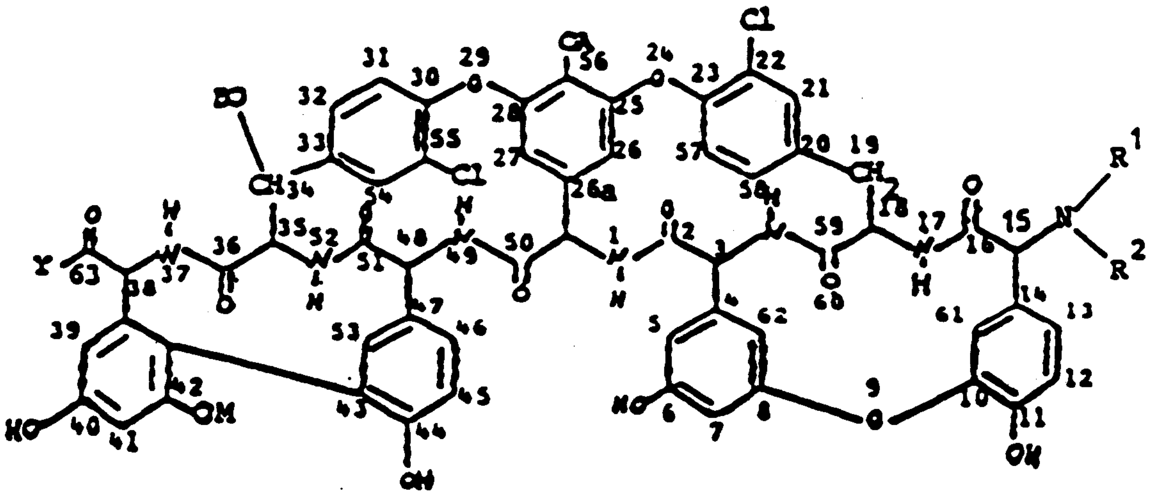

(I)

in der
$R^1$ ein Wasserstoffatom oder einen $(C_1-C_3)$Alkylrest bedeutet,
$R^2$ einen Rest der Formel $[CHR^3(CR^4R^5)_mX]_p-(CH_2)_n-R^6$ bedeutet,
in der $R^3$ und $R^4$ unabhängig voneinander ein Wasserstoffatom oder einen $(C_1-C_6)$Alkylrest bedeuten;
$R^5$ ein Wasserstoffatom, einen $(C_1-C_6)$Alkylrest oder eine Hydroxylgruppe bedeutet;
$R^6$ ein Wasserstoffatom, einen $(C_1-C_3)$Alkylrest, einen Rest $COOR^7$, $OR^7$, $SR^7$ oder $NR^7R^8$ oder ein Halogenatom bedeutet;
$R^7$ und $R^8$ unabhängig voneinander ein Wasserstoffatom oder einen $(C_1-C_3)$Alkylrest bedeuten;
m 0 oder 1 ist, n eine ganze Zahl von 0 bis 6 ist, p eine ganze Zahl von 1 bis 6 ist;
X O, NH oder eine Bindung bedeutet, mit der Maßgabe, daß, wenn X eine Gruppe O Oder NH ist, n nicht 0 ist, p zwischen 1 und 3 liegt und $R^5$ keine Hydroxylgruppe ist;
Y einen Rest

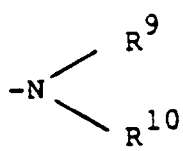

bedeutet, in der
$R^9$ ein Wasserstoffatom oder einen $C_1-C_4$-Alkyl-, Hydroxy$(C_2-C_4)$alkyl-, Amino$(C_2-C_4)$alkyl- oder $(C_1-C_3)$alkylamino$(C_2-C_4)$alkylrest bedeutet;
$R^{10}$ ein Wasserstoffatom, einen $C_1-C_6$-Alkyl-, Hydroxy$(C_2-C_4)$alkyl-, Halogen$(C_2-C_4)$alkyl-, Carboxy$(C_1-C_3)$alkyl-, Carboxamido$(C_1-C_3)$alkyl-, $(C_1-C_3)$Alkylcarboxy$(C_1-C_3)$alkylamino-, $(C_1-C_3)$Alkoxycarbonyl$(C_1-C_3)$alkyl-, Phenyl$(C_1-C_3)$alkoxycarbonyl$(C_1-C_3)$alkylrest oder einen Rest

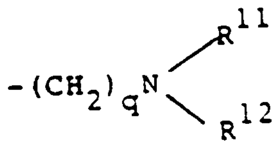

oder einen Rest

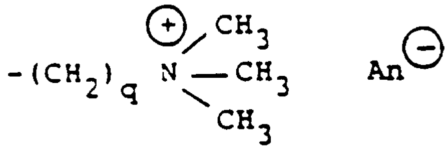

bedeutet,
in denen q eine ganze Zahl von 2 bis 8 ist, und eines der Wasserstoffatome der Methyleneinheit gegebenenfalls mit einem Substituenten aus der Gruppe $(C_1-C_3)$Alkyl-,Carboxy-, $(C_1-C_4)$-Alkoxycarbonyl- und Carboxamidorest substituiert ist und $An^{\ominus}$ ein pharmazeutisch verträgliches Anion ist;
$R^{11}$ ein Wasserstoffatom oder einen $(C_1-C_4)$Alkyl-, Hydroxy$(C_2-C_4)$alkyl- oder Halogen$(C_2-C_4)$alkylrest bedeutet;
$R^{12}$ ein Wasserstoffatom, einen $(C_1-C_6)$Alkyl-, Hydroxy$(C_2-C_4)$alkyl-, Halogen$(C_2-C_4)$alkyl-, $(C_1-C_3)$-Alkoxy$(C_1-C_4)$alkyl-, Phenyl$(C_1-C_4)$alkoxycarbonyl- oder $(C_1-C_4)$Alkyloxycarbonylrest bedeutet oder
$R^{11}$ und $R^{12}$ zusammen mit dem angrenzenden Stickstoffatom einen gesättigten 5-7-gliedrigen heterocyclischen Ring darstellen, der gegebenenfalls einen oder zwei $(C_1-C_4)$Alkylreste an den Ringkohlenstoffatomen tragen kann und ein weiteres Heteroatom aus der Gruppe -O-, -S- und $-NR^{13}-$ enthalten kann, wobei $R^{13}$ ein Wasserstoffatom oder einen $(C_1-C_4)$Alkylrest bedeutet;
oder $R^9$ und $R^{10}$ zusammen mit dem angrenzenden Stickstoffatom einen gesättigten 5-7-gliedrigen heterocyclischen Ring darstellen, der gegebenenfalls einen oder zwei $(C_1-C_4)$Alkylreste an den Ringkohlenstoffatomen tragen kann und ein weiteres Heteroatom aus der Gruppe -O-, -S- und $-NR^{14}-$ tragen kann, wobei $R^{14}$ ein Wasserstoffatom oder einen $(C_1-C_4)$Alkylrest bedeutet;
A ein Wasserstoffatom oder einen -N[$(C_9-C_{12})$aliphatisch-Acyl]-$\beta$-D-2-deoxy-2-amino-glucopyranosylrest bedeutet;
B ein Wasserstoffatom oder eine N-Acetyl-$\beta$-D-2-deoxy-2-amino-glucopyranosylgruppe bedeutet;
M ein Wasserstoffatom oder eine $\alpha$-D-Mannopyranosylgruppe bedeutet;
mit der Maßgabe, daß B nur dann ein Wasserstoffatom darstellt, wenn A und M gleichzeitig ein Wasserstoffatom bedeuten;
und die Additionssalze davon.

**2.** Verbindung gemäß Anspruch 1, in der
$R^1$ ein Wasserstoffatom oder einen $(C_1-C_3)$Alkylrest bedeutet,
$R^2$ einen Rest der Formel $[CHR^3(CR^4R^5)_mX]_p-(CH_2)_n-R^6$ bedeutet;
in der $R^3$ und $R^4$ unabhängig voneinander ein Wasserstoffatom oder einen $(C_1-C_6)$Alkylrest bedeuten;
$R^5$ ein Wasserstoffatom, einen $(C_1-C_6)$Alkylrest oder eine Hydroxylgruppe bedeutet;
$R^6$ ein Wasserstoffatom, einen $(C_1-C_3)$Alkylrest, einen Rest $COOR^7$, $OR^7$, $SR^7$ oder $NR^7R^8$ oder ein Halogenatom bedeutet;
$R^7$ und $R^8$ unabhängig voneinander ein Wasserstoffatom oder einen $(C_1-C_3)$Alkylrest bedeuten;
m 0 oder 1 ist, n eine ganze Zahl von 0 bis 6 ist, p eine ganze Zahl von 1 bis 6 ist;
X O, NH oder eine Bindung bedeutet, mit der Maßgabe, daß, wenn X eine Gruppe O oder NH ist, n nicht 0 ist, p zwischen 1 und 3 liegt und $R^5$ keine Hydroxylgruppe ist;
Y einen Rest

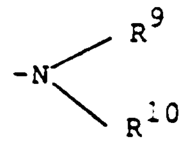

bedeutet, in der
$R^9$ ein Wasserstoffatom oder einen $C_1$-$C_4$-Alkylrest bedeutet;
$R^{10}$ ein Wasserstoffatom, einen $C_1$-$C_6$-Alkyl-, Hydroxy($C_2$-$C_4$)alkyl- oder Halogen($C_2$-$C_4$)alkylrest oder einen Rest

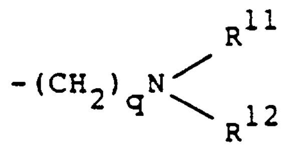

bedeutet,
in der q eine ganze Zahl von 2 bis 8 ist, und eines der Wasserstoffatome einer Methyleneinheit gegebenenfalls mit einem Substituenten aus der Gruppe Carboxy- und ($C_1$-$C_4$)Alkoxycarbonylrest substituiert ist;
$R^{11}$ ein Wasserstoffatom oder einen ($C_1$-$C_4$)Alkylrest bedeutet,
$R^{12}$ ein Wasserstoffatom, einen ($C_1$-$C_6$)Alkyl-, Hydroxy($C_2$-$C_4$)alkyl-, Halogen($C_2$-$C_4$)alkyl-, ($C_1$-$C_3$)-Alkoxy($C_1$-$C_4$)alkyl-, Phenyl($C_1$-$C_4$)alkoxycarbonyl- oder ($C_1$-$C_4$)Alkyloxycarbonylrest bedeutet oder
$R^{11}$ und $R^{12}$ zusammen mit dem benachbarten Stickstoffatom, einen gesättigten 5-7-gliedrigen heterocyclischen Ring darstellen, der gegebenenfalls ein oder zwei ($C_1$-$C_4$)-Alkylreste an den Ringkohlenstoffatomen tragen kann und weitere Heteroatome aus der Gruppe -O-, -S- und -$NR^{13}$- enthalten kann, wobei $R^{13}$ ein Wasserstoffatom oder ein ($C_1$-$C_4$)Alkylrest ist;
oder $R^9$ und $R^{10}$ zusammen mit dem benachbarten Stickstoffatom einen gesättigten 5-7-gliedrigen heterocyclischen Ring darstellen, der gegebenenfalls einen oder zwei ($C_1$-$C_4$)Alkylreste an den Ringkohlenstoffatomen tragen kann und weitere Heteroatome aus der Gruppe -O-, -S- und -$NR^{14}$- enthalten kann, wobei $R^{14}$ ein Wasserstoffatom oder einen ($C_1$-$C_4$)Alkylrest bedeutet;
A ein Wasserstoffatom oder einen -N[($C_9$-$C_{12}$)aliphatisch-Acyl]-$\beta$-D-2-deoxy-2-amino-glucopyranosylrest bedeutet;
B ein Wasserstoffatom oder eine N-Acetyl-$\beta$-D-2-deoxy-2-amino-glucopyranosylgruppe bedeutet,
M ein Wasserstoffatom oder eine $\alpha$-D-Mannopyranosylgruppe bedeutet,
mit der Maßgabe, daß B nur dann ein Wasserstoffatom bedeutet, wenn A und M gleichzeitig ein Wasserstoffatom bedeuten;
und die Additionssalze davon.

**3.** Verbindung gemäß Anspruch 1, in der $R^1$ ein Wasserstoffatom ist.

**4.** Verbindung gemäß Anspruch 1, in der $R^1$ eine Methylgruppe ist.

**5.** Verbindung gemäß Anspruch 1, in der
$R^1$ ein Wasserstoffatom oder einen ($C_1$-$C_3$)Alkylrest bedeutet,
$R^2$ einen Rest der Formel $[CHR^3(CR^4R^5)_mX]_p$-$(CH_2)_n$-$R^6$ bedeutet,
in der $R^3$ und $R^4$ unabhängig voneinander ein Wasserstoffatom oder einen ($C_1$-$C_4$)Alkylrest bedeuten;
$R^5$ ein Wasserstoffatom, einen ($C_1$-$C_4$)Alkylrest oder eine Hydroxylgruppe bedeutet;
$R^6$ ein Wasserstoffatom, einen ($C_1$-$C_3$)Alkylrest, einen Rest $OR^7$, $SR^7$ oder $NR^7R^8$ bedeutet;
$R^7$ und $R^8$ unabhängig voneinander ein Wasserstoffatom oder einen ($C_1$-$C_3$)Alkylrest bedeuten;
m 0 oder 1 ist, n eine ganze Zahl von 0 bis 3 ist, p eine ganze Zahl von 1 bis 3 ist;
X O, NH oder eine Bindung bedeutet, mit der Maßgabe, daß wenn X eine Gruppe O oder NH ist, n nicht 0 ist, p 1 oder 2 ist und $R^5$ keine Hydroxylgruppe ist;
Y einen Rest

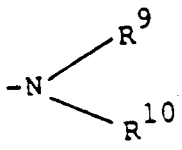

bedeutet, in der
$R^9$ ein Wasserstoffatom oder einen $(C_1$-$C_4)$Alkylrest bedeutet;
$R^{10}$ ein Wasserstoffatom, einen $(C_1$-$C_4)$Alkyl- oder Hydroxy$(C_2$-$C_4)$alkylrest oder einen Rest

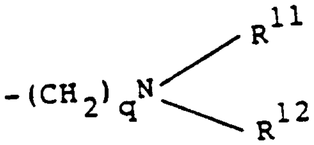

bedeutet,
in der q eine ganze Zahl von 2 bis 8 ist, und eines der Wasserstoffatome einer Methyleneinheit gegebenenfalls mit einem Substituenten aus der Gruppe Carboxy- und $(C_1$-$C_3)$Alkoxycarbonylrest substituiert ist;
$R^{11}$ ein Wasserstoffatom oder einen $(C_1$-$C_4)$Alkylrest bedeutet,
$R^{12}$ ein Wasserstoffatom, einen $(C_1$-$C_6)$Alkyl-, Hydroxy$(C_2$-$C_4)$alkyl-, $(C_1$-$C_3)$Alkoxy$(C_1$-$C_4)$alkyl-, Phenyl-$(C_1$-$C_4)$alkoxycarbonyl- oder $(C_1$-$C_4)$Alkyloxycarbonylrest bedeutet, oder
oder $R^9$ und $R^{10}$ zusammen mit dem benachbarten Stickstoffatom einen gesättigten 5-7-gliedrigen heterocyclischen Ring darstellen, der gegebenenfalls einen oder zwei $(C_1$-$C_4)$Alkylreste an den Ringkohlenstoffatomen tragen kann und weitere Heteroatome aus der Gruppe -O-, -S- und $-NR^{14}$- enthalten kann, wobei $R^{14}$ ein Wasserstoffatom oder ein $(C_1$-$C_4)$Alkylrest ist;
A ein Wasserstoffatom oder einen -N[$(C_9$-$C_{12})$aliphatisch-Acyl]-$\beta$-D-2-deoxy-2-amino-glucopyranosylrest bedeutet;
B ein Wasserstoffatom oder eine N-Acetyl-$\beta$-D-2-deoxy-2-amino-glucopyranosylgruppe bedeutet;
M ein Wasserstoffatom oder eine $\alpha$-D-Mannopyranosylgruppe bedeutet;
mit der Maßgabe, daß B nur dann ein Wasserstoffatom bedeutet, wenn A und M gleichzeitig ein Wasserstoffatom sind;
und die Additionssalze davon.

**6.** Verbindung gemäß Anspruch 1, in der
$R^1$ ein Wasserstoffatom oder eine Methylgruppe bedeutet,
$R^2$ eine Gruppe gemäß der vorstehenden Definition ist, in der $R^3$ ein Wasserstoffatom oder eine Methylgruppe bedeutet, $R^4$ ein Wasserstoffatom oder eine Methylgruppe bedeutet, $R^5$ ein Wasserstoffatom oder eine Hydroxylgruppe bedeutet, m 0 oder 1 ist, n im Bereich von 0 bis 2 liegt, p 1 oder 2 ist, $R^6$ ein Wasserstoffatom oder einen Rest $OR^7$ oder $NR^7R^8$ bedeutet, und $R^7$ und $R^8$ unabhängig voneinander ein Wasserstoffatom oder eine Methylgruppe bedeuten,
Y eine Gruppe

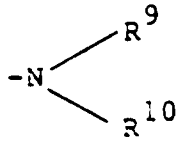

bedeutet, in der
$R^9$ ein Wasserstoffatom oder einen $(C_1$-$C_4)$Alkylrest bedeutet;
$R^{10}$ ein Wasserstoffatom, einen $(C_1$-$C_4)$Alkyl- oder Hydroxy$(C_2$-$C_4)$alkylrest bedeutet oder eine Gruppe

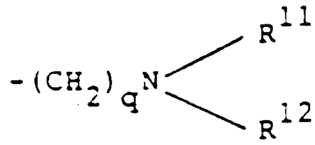

bedeutet,
in der q eine ganze Zahl von 2 bis 8 ist, und eines der Wasserstoffatome einer Methyleneinheit gegebenenfalls mit einem Rest aus der Gruppe Carboxy- und $(C_1\text{-}C_3)$Alkoxycarbonylrest substituiert ist,
$R^{11}$ ein Wasserstoffatom oder einen $(C_1\text{-}C_4)$Alkylrest bedeutet,
$R^{12}$ ein Wasserstoffatom, einen $(C_1\text{-}C_6)$Alkyl-, Hydroxy$(C_2\text{-}C_4)$alkyl-, Halogen$(C_2\text{-}C_4)$alkyl-, $(C_1\text{-}C_3)$-Alkoxy$(C_1\text{-}C_4)$alkyl-, Phenyl$(C_1\text{-}C_4)$alkoxycarbonyl- oder $(C_1\text{-}C_4)$Alkyloxycarbonylrest bedeutet, oder
$R^{11}$ und $R^{12}$ zusammen mit dem angrenzenden Stickstoffatom einen gesättigten 5-7-gliedrigen heterocyclischen Ring darstellen, der gegebenenfalls ein oder zwei $(C_1\text{-}C_4)$Alkylreste an den Ringkohlenstoffatomen tragen kann und ein weiteres Heteroatom aus der Gruppe -O-, -S- und $\text{-NR}^{13}\text{-}$ enthalten kann, wobei $R^{13}$ ein Wasserstoffatom oder ein $(C_1\text{-}C_4)$Alkylrest ist,
oder $R^9$ und $R^{10}$ zusammen mit dem angrenzenden Stickstoffatom einen gesättigten 5-7-gliedrigen heterocyclischen Ring darstellen, der gegebenenfalls einen oder zwei $(C_1\text{-}C_4)$Alkylreste an den Ringkohlenstoffatomen tragen kann und ein weiteres Heteroatom aus der Gruppe -O-, -S- und $\text{-NR}^{14}\text{-}$ enthalten kann, wobei $R^{14}$ ein Wasserstoffatom oder ein $(C_1\text{-}C_4)$Alkylrest ist,
A ein Wasserstoffatom oder einen $\text{-N[}(C_9\text{-}C_{12})$aliphatisch-Acyl]-$\beta$-D-2-deoxy-2-amino-glucopyranosylrest bedeutet,
B ein Wasserstoffatom oder eine N-Acetyl-$\beta$-D-2-deoxy-2-amino-glucopyranosylgruppe bedeutet,
M ein Wasserstoffatom oder eine $\alpha$-D-Mannopyranosylgruppe bedeutet;
mit der Maßgabe, daß B nur dann ein Wasserstoffatom bedeutet, wenn A und M gleichzeitig ein Wasserstoffatom bedeuten,
und die Additionssalze davon.

7. Verfahren zur Herstellung einer Verbindung gemäß Anspruch 1, umfassend die Umsetzung einer Verbindung der Formel II

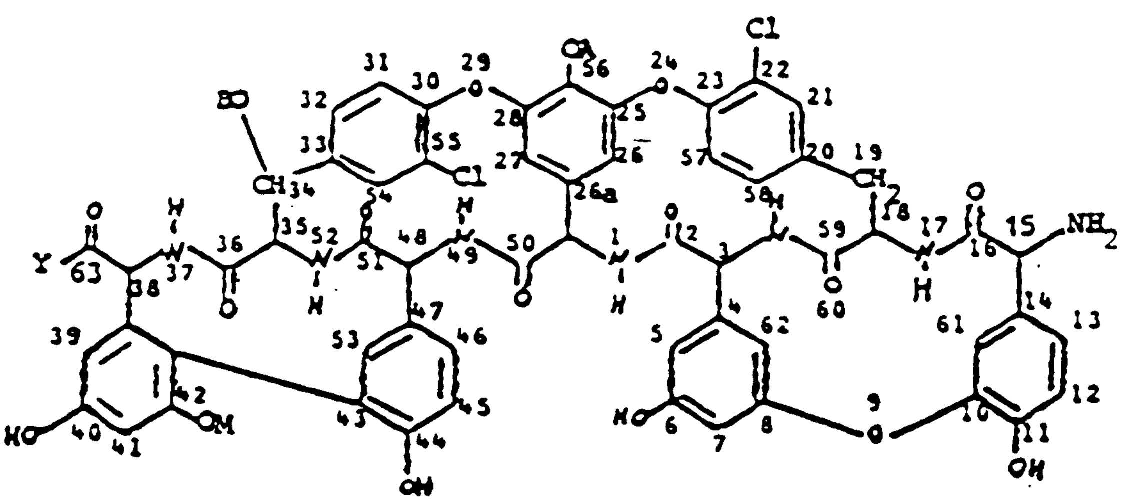

(II)

in der
Y einen Rest

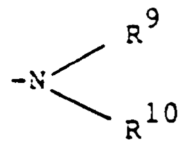

darstellt, in der
$R^9$ ein Wasserstoffatom oder einen $C_1$-$C_4$-Alkyl-, Hydroxy($C_2$-$C_4$)alkyl-, Amino($C_2$-$C_4$)alkyl- oder ($C_1$-$C_3$)Alkylamino($C_2$-$C_4$)alkylrest bedeutet;
$R^{10}$ ein Wasserstoffatom, einen $C_1$-$C_6$-Alkyl-, Hydroxy($C_2$-$C_4$)alkyl-, Halogen($C_2$-$C_4$)alkyl-, Carboxy($C_1$-$C_3$)alkyl-, Carboxamido($C_1$-$C_3$)alkyl-, ($C_1$-$C_3$)Alkylcarboxy($C_1$-$C_3$)alkylamido-, ($C_1$-$C_3$)Alkoxycarbonyl($C_1$-$C_3$)alkyl-, Phenyl($C_1$-$C_3$)alkoxycarbonyl($C_1$-$C_3$)alkylrest oder eine Gruppe

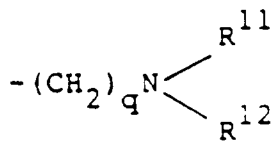

oder eine Gruppe

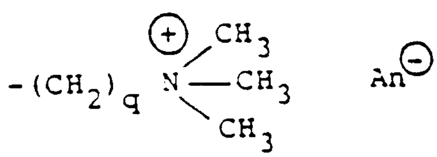

bedeutet,
in der q eine ganze Zahl von 2 bis 8 ist, und eines der Wasserstoffatome der Methyleneinheit gegebenenfalls mit einem Substituenten aus der Gruppe ($C_1$-$C_3$)Alkyl-, Carboxy-, ($C_1$-$C_4$)-Alkoxycarbonyl- und Carboxamidorest substituiert ist und $An^{\ominus}$ ein pharmazeutisch verträgliches Anion ist;
$R^{11}$ ein Wasserstoffatom oder einen ($C_1$-$C_4$)Alkyl-, Hydroxy($C_2$-$C_4$)alkyl- oder Halogen($C_2$-$C_4$)alkylrest bedeutet;
$R^{12}$ ein Wasserstoffatom, einen ($C_1$-$C_6$)Alkyl-, Hydroxy($C_2$-$C_4$)alkyl-, Halogen($C_2$-$C_4$)alkyl-, ($C_1$-$C_3$)-Alkoxy($C_1$-$C_4$)alkyl-, Phenyl($C_1$-$C_4$)alkoxycarbonyl- oder ($C_1$-$C_4$)Alkyloxycarbonylrest bedeutet, oder
$R^{11}$ und $R^{12}$ zusammen mit dem angrenzenden Stickstoffatom einen gesättigten 5-7-gliedrigen heterocyclischen Ring darstellen, der gegebenenfalls einen oder zwei ($C_1$-$C_4$)Alkylreste an den Ringkohlenstoffatomen tragen kann und ein weiteres Heteroatom aus der Gruppe -O-, -S- und -$NR^{13}$- enthalten kann, wobei $R^{13}$ ein Wasserstoffatom oder einen ($C_1$-$C_4$)Alkylrest bedeutet,
oder $R^9$ und $R^{10}$ zusammen mit dem angrenzenden Stickstoffatom einen gesättigten 5-7-gliedrigen heterocyclischen Ring bedeuten, der gegebenenfalls einen oder zwei ($C_1$-$C_4$)Alkylreste an den Ringkohlenstoffatomen tragen kann und ein weiteres Heteroatom aus der Gruppe -O-, -S- und -$NR^{14}$-enthalten kann, wobei $R^{14}$ ein Wasserstoffatom oder einen ($C_1$-$C_4$)Alkylrest bedeutet;
A ein Wasserstoffatom oder einen -N[($C_9$-$C_{12}$)aliphatisch-Acyl)-$\beta$-D-2-deoxy-2-amino-glucopyranosylrest bedeutet,
B ein Wasserstoffatom oder eine N-Acetyl-$\beta$-D-2-deoxy-2-amino-glucopyranosylgruppe bedeutet,
M ein Wasserstoffatom oder eine $\alpha$-D-Mannopyranosylgruppe bedeutet,
mit der Maßgabe, daß B nur dann ein Wasserstoffatom bedeutet, wenn A und M gleichzeitig ein Wasserstoffatom bedeuten,
eines Salzes davon oder eines Gemisches davon in beliebiger Zusammensetzung, mit

a) einem Alkylhalogenid der Formel $R^1$(halo) oder $R^2$(halo),
in der
$R^1$ ein Wasserstoffatom oder einen ($C_1$-$C_3$)Alkylrest bedeutet,
$R^2$ einen Rest der Formel Gruppe $[CHR^3(CR^4R^5)_mX]_p$-$(CH_2)_n$-$R^6$ bedeutet,
in der

$R^3$ und $R^4$ unabhängig voneinander ein Wasserstoffatom oder ein $(C_1-C_6)$Alkylrest bedeuten,
$R^5$ ein Wasserstoffatom, einen $(C_1-C_6)$Alkylrest oder eine Hydroxylgruppe bedeutet,
$R^6$ ein Wasserstoffatom, einen $(C_1-C_3)$Alkylrest, einen Rest $COOR^7$, $OR^7$, $SR^7$ oder $NR^7R^8$ oder ein Halogenatom bedeutet,
$R^7$ und $R^8$ unabhänging voneinander ein Wasserstoffatom oder einen $(C_1-C_3)$Alkylrest bedeuten, m 0 oder 1 ist, n eine ganze Zahl von 0 bis 6 ist, p eine ganze Zahl von 1 bis 6 ist,
X O, NH oder eine Bindung bedeutet, mit der Maßgabe, daß wenn X eine Gruppe O oder NH ist, n nicht 0 ist, p im Bereich zwischen 1 und 3 liegt, $R^5$ keine Hydroxygruppe ist, und (halo) ein Chlor- oder Bromatom bedeutet, in Gegenwart einer Base und möglicherweise eines organischen Lösungsmittels,
b) alternativ, einem wenigstens 20-fachen molaren Überschuß an Carbonylverbindung, bezogen auf das Teicoplaninausgangsmaterial in einem organischen, polaren, aprotischen Lösungsmittel mit einem Reduktionsmittel.

**8.** Verfahren gemäß Anspruch 7, wobei die Carbonylverbindung das gleiche Gerüst des Restes $R^2$ (oder $R^1$) aufweist und ihre Oxogruppe sich an dem Kohlenstoffatom befindet, das die Verbindung mit der $N^{15}$-Aminogruppe der Teicoplanineinheit herstellen soll.

**9.** Verfahren gemäß Anspruch 7, wobei das Reduktionsmittel ein Alkalimetallborhydrid ist.

**10.** Verbindung gemäß Anspruch 1 zur Verwendung als Arzneimittel.

**11.** Verwendung eines Stoffes gemäß Anspruch 1 zur Herstellung eines Arzneimittels zur Verwendung als antibakterielles Mittel.

**12.** Arzneimittel, umfassend eine Verbindung gemäß Anspruch 1 im Gemisch mit einem pharmazeutisch verträglichen Träger.

**Patentansprüche für folgende Vertragsstaaten : ES, GR**

**1.** Verfahren zur Herstellung eines Amids einer $N^{15}$-Alkyl- oder $N^{15}$,$N^{15}$-Dialkylteicoplaninverbindung der folgenden Formel I

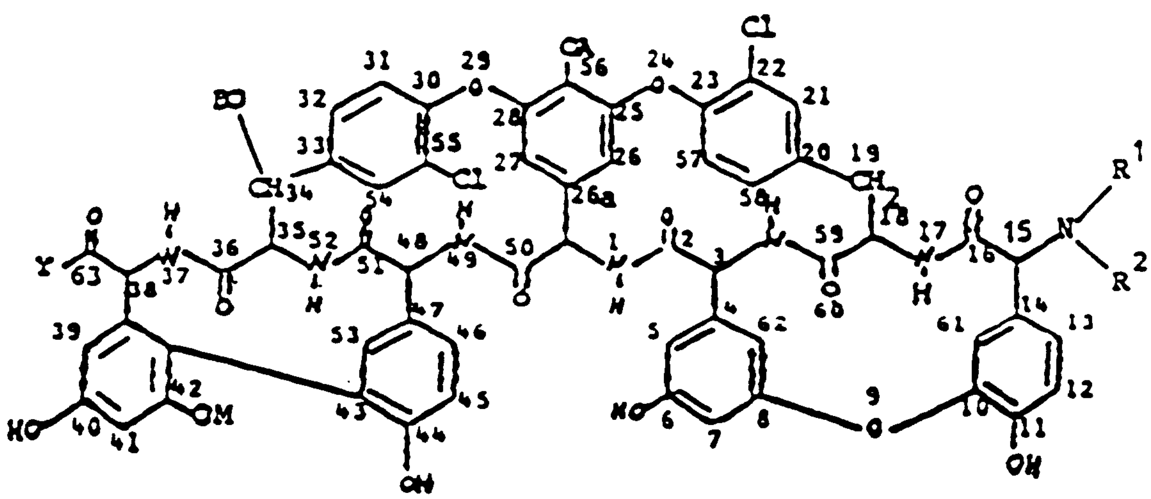

(I)

in der
$R^1$ ein Wasserstoffatom oder einen $(C_1-C_3)$Alkylrest bedeutet,
$R^2$ einen Rest der Formel $[CHR^3(CR^4R^5)_mX]_p-(CH_2)_n-R^6$ bedeutet,
in der $R^3$ und $R^4$ unabhängig voneinander ein Wasserstoffatom oder einen $(C_1-C_6)$Alkylrest bedeuten;
$R^5$ ein Wasserstoffatom, einen $(C_1-C_6)$Alkylrest oder eine Hydroxylgruppe bedeutet;

$R^6$ ein Wasserstoffatom, einen $(C_1$-$C_3)$Alkylrest, einen Rest $COOR^7$, $OR^7$, $SR^7$ oder $NR^7R^8$ oder ein Halogenatom bedeutet;
$R^7$ und $R^8$ unabhängig voneinander ein Wasserstoffatom oder einen $(C_1$-$C_3)$Alkylrest bedeuten;
m 0 oder 1 ist, n eine ganze Zahl von 0 bis 6 ist, p eine ganze Zahl von 1 bis 6 ist;
X O, NH oder eine Bindung bedeutet, mit der Maßgabe, daß, wenn X eine Gruppe O oder NH ist, n nicht 0 ist, p zwischen 1 und 3 liegt und $R^5$ keine Hydroxylgruppe ist;
Y einen Rest

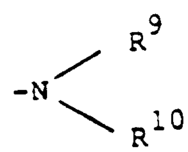

bedeutet, in der
$R^9$ ein Wasserstoffatom oder einen $C_1$-$C_4$-Alkyl-, Hydroxy$(C_2$-$C_4)$alkyl-, Amino$(C_2$-$C_4)$alkyl- oder $(C_1$-$C_3)$alkylamino$(C_2$-$C_4)$alkylrest bedeutet;
$R^{10}$ ein Wasserstoffatom, einen $C_1$-$C_6$-Alkyl-, Hydroxy$(C_2$-$C_4)$alkyl-, Halogen$(C_2$-$C_4)$alkyl-, Carboxy$(C_1$-$C_3)$alkyl-, Carboxamido$(C_1$-$C_3)$alkyl-, $(C_1$-$C_3)$Alkylcarboxy$(C_1$-$C_3)$alkylamino-, $(C_1$-$C_3)$Alkoxycarbonyl$(C_1$-$C_3)$alkyl-, Phenyl$(C_1$-$C_3)$alkoxycarbonyl$(C_1$-$C_3)$alkylrest oder einen Rest

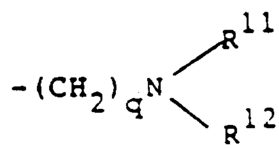

oder einen Rest

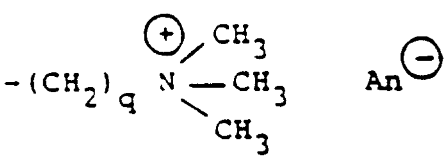

bedeutet,
in denen q eine ganze Zahl von 2 bis 8 ist, und eines der Wasserstoffatome der Methyleneinheit gegebenenfalls mit einem Substituenten aus der Gruppe $(C_1$-$C_3)$Alkyl-,Carboxy-, $(C_1$-$C_4)$-Alkoxycarbonyl- und Carboxamidorest substituiert ist und $An^{\ominus}$ ein pharmazeutisch verträgliches Anion ist;
$R^{11}$ ein Wasserstoffatom oder einen $(C_1$-$C_4)$Alkyl-, Hydroxy$(C_2$-$C_4)$alkyl- oder Halogen$(C_2$-$C_4)$alkylrest bedeutet;
$R^{12}$ ein Wasserstoffatom, einen $(C_1$-$C_6)$Alkyl-, Hydroxy$(C_2$-$C_4)$alkyl-, Halogen$(C_2$-$C_4)$alkyl-, $(C_1$-$C_3)$-Alkoxy$(C_1$-$C_4)$alkyl-, Phenyl$(C_1$-$C_4)$alkoxycarbonyl- oder $(C_1$-$C_4)$Alkyloxycarbonylrest bedeutet oder
$R^{11}$ und $R^{12}$ zusammen mit dem angrenzenden Stickstoffatom einen gesättigten 5-7-gliedrigen heterocyclischen Ring darstellen, der gegebenenfalls einen oder zwei $(C_1$-$C_4)$-Alkylreste an den Ringkohlenstoffatomen tragen kann und ein weiteres Heteroatom aus der Gruppe -O-, -S- und $-NR^{13}$- enthalten kann, wobei $R^{13}$ ein Wasserstoffatom oder einen $(C_1$-$C_4)$Alkylrest bedeutet;
oder $R^9$ und $R^{10}$ zusammen mit dem angrenzenden Stickstoffatom einen gesättigten 5-7-gliedrigen heterocyclischen Ring darstellen, der gegebenenfalls einen oder zwei $(C_1$-$C_4)$Alkylreste an den Ringkohlenstoffatomen tragen kann und ein weiteres Heteroatom aus der Gruppe -O-, -S- und $-NR^{14}$- tragen kann, wobei $R^{14}$ ein Wasserstoffatom oder einen $(C_1$-$C_4)$Alkylrest bedeutet;
A ein Wasserstoffatom oder einen -N[$(C_9$-$C_{12})$aliphatisch-Acyl]-$\beta$-D-2-deoxy-2-amino-glucopyranosylrest bedeutet;

B ein Wasserstoffatom oder eine N-Acetyl-β-D-2-deoxy-2-amino-glucopyranosylgruppe bedeutet;
M ein Wasserstoffatom oder eine α-D-Mannopyranosylgruppe bedeutet;
mit der Maßgabe, daß B nur dann ein Wasserstoffatom darstellt, wenn A und M gleichzeitig ein Wasserstoffatom bedeuten;
und die Additionssalze davon,
umfassend die Umsetzung einer Verbindung der Formel II

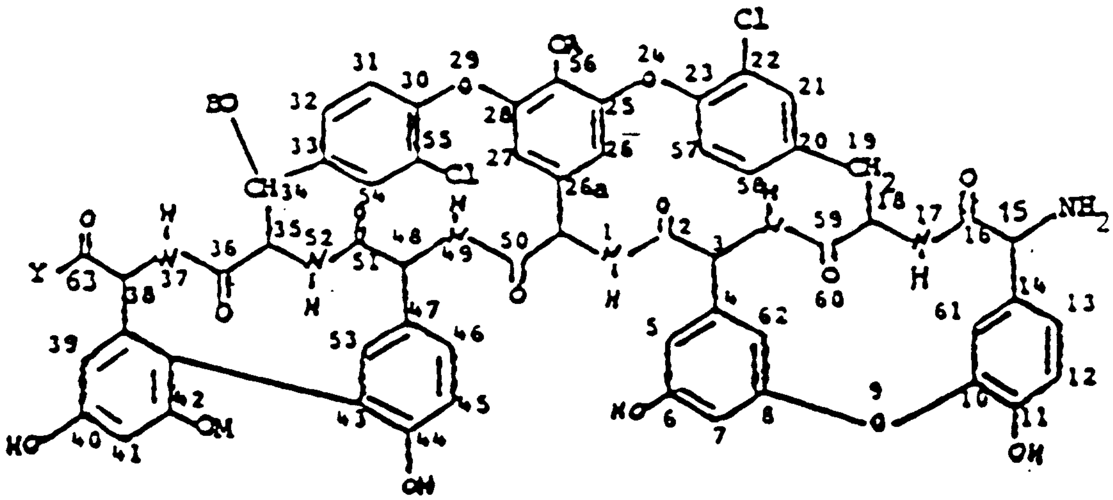

(II)

in der
Y eine Gruppe

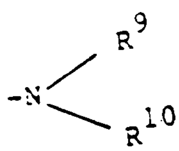

darstellt, in der Y, A, B und M die vorstehend gegebenen Bedeutungen haben, mit

a) einem Alkylhalogenid der Formel $R^1$(halo) oder
$R^2$(halo),
in der
$R^1$ ein Wasserstoffatom oder einen $(C_1\text{-}C_3)$Alkylrest bedeutet,
$R^2$ einen Rest der Formel $[CHR^3(CR^4R^5)_mX]_p\text{-}(CH_2)_n\text{-}R^6$ bedeutet,
in der
$R^3$ und $R^4$ unabhängig voneinander ein Wasserstoffatom oder ein $(C_1\text{-}C_6)$Alkylrest bedeuten,
$R^5$ ein Wasserstoffatom, einen $(C_1\text{-}C_6)$Alkylrest oder eine Hydroxylgruppe bedeutet,
$R^6$ ein Wasserstoffatom, einen $(C_1\text{-}C_3)$Alkylrest, einen Rest $COOR^7$, $OR^7$, $SR^7$ oder $NR^7R^8$ oder ein Halogenatom bedeutet,
$R^7$ und $R^8$ unabhängig voneinander ein Wasserstoffatom oder einen $(C_1\text{-}C_3)$Alkylrest bedeuten, m 0 oder 1 ist, n eine ganze Zahl von 0 bis 6 ist, p eine ganze Zahl von 1 bis 6 ist,
X O, NH oder eine Bindung bedeutet, mit der Maßgabe, daß wenn X O oder NH ist, n nicht 0 ist, p im Bereich zwischen 1 und 3 liegt, $R^5$ keine Hydroxygruppe ist, und (halo) ein Chlor- oder Bromatom bedeutet, in Gegenwart einer Base und möglicherweise eines organischen Lösungsmittels,

b) alternativ, einem wenigstens 20-fachen molaren Überschuß an Carbonylverbindung, bezogen auf das Teicoplaninausgangsmaterial in einem organischen, polaren, aprotischen Lösungsmittel mit einem Reduktionsmittel, wobei die Carbonylverbindung das gleiche Gerüst des Restes $R^2$ (oder $R^1$) aufweist und ihre Oxogruppe sich an dem Kohlenstoffatom befindet, das die Verbindung mit der $N^{15}$-Aminogruppe der Teicoplanineinheit herstellen soll.

2. Verfahren nach Anspruch 1 zur Herstellung einer Verbindung der Formel I, in der
$R^1$ ein Wasserstoffatom oder einen $(C_1-C_3)$Alkylrest bedeutet,
$R^2$ einen Rest der Formel $[CHR^3(CR^4R^5)_mX]_p-(CH_2)_n-R^6$ bedeutet;
in der $R^3$ und $R^4$ unabhängig voneinander ein Wasserstoffatom oder einen $(C_1-C_6)$Alkylrest bedeuten;
$R^5$ ein Wasserstoffatom, einen $(C_1-C_6)$Alkylrest oder eine Hydroxylgruppe bedeutet;
$R^6$ ein Wasserstoffatom, einen $(C_1-C_3)$Alkylrest, einen Rest $COOR^7$, $OR^7$, $SR^7$ oder $NR^7R^8$ oder ein Halogenatom bedeutet;
$R^7$ und $R^8$ unabhängig voneinander ein Wasserstoffatom oder einen $(C_1-C_3)$Alkylrest bedeuten;
m 0 oder 1 ist, n eine ganze Zahl von 0 bis 6 ist, p eine ganze Zahl von 1 bis 6 ist;
X O, NH oder eine Bindung bedeutet, mit der Maßgabe, daß, wenn X O oder NH ist, n nicht 0 ist, p zwischen 1 und 3 liegt und $R^5$ keine Hydroxylgruppe ist;
Y einen Rest

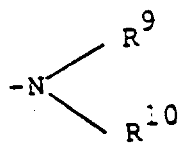

bedeutet, in der
$R^9$ ein Wasserstoffatom oder einen $C_1-C_4$-Alkylrest bedeutet;
$R^{10}$ ein Wasserstoffatom, einen $C_1-C_6$-Alkyl-, Hydroxy$(C_2-C_4)$alkyl- oder Halogen$(C_2-C_4)$alkylrest oder einen Rest

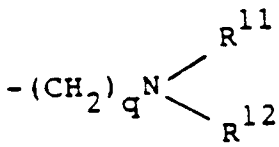

bedeutet,
in der q eine ganze Zahl von 2 bis 8 ist, und eines der Wasserstoffatome einer Methyleneinheit gegebenenfalls mit einem Substituenten aus der Gruppe Carboxy- und $(C_1-C_4)$Alkoxycarbonylrest substituiert ist;
$R^{11}$ ein Wasserstoffatom oder einen $(C_1-C_4)$Alkylrest bedeutet,
$R^{12}$ ein Wasserstoffatom, einen $(C_1-C_6)$Alkyl-, Hydroxy$(C_2-C_4)$alkyl-, Halogen$(C_2-C_4)$alkyl-, $(C_1-C_3)$-Alkoxy$(C_1-C_4)$alkyl-, Phenyl$(C_1-C_4)$alkoxycarbonyl- oder $(C_1-C_4)$Alkyloxycarbonylrest bedeutet oder
$R^{11}$ und $R^{12}$ zusammen mit dem benachbarten Stick-stoffatom, einen gesättigten 5-7-gliedrigen heterocyclischen Ring darstellen, der gegebenenfalls ein oder zwei $(C_1-C_4)$-Alkylreste an den Ringkohlenstoffatomen tragen kann und weitere Heteroatome aus der Gruppe -O-, -S- und $-NR^{13}-$ enthalten kann, wobei $R^{13}$ ein Wasser-stoffatom oder ein $(C_1-C_4)$Alkylrest ist;
oder $R^9$ und $R^{10}$ zusammen mit dem benachbarten Stickstoffatom einen gesättigten 5-7-gliedrigen hetero-cyclischen Ring darstellen, der gegebenenfalls einen oder zwei $(C_1-C_4)$Alkylreste an den Ringkohlenstoffatomen tragen kann und weitere Heteroatome aus der Gruppe -O-, -S-und $-NR^{14}-$ enthalten kann, wobei $R^{14}$ ein Wasserstoffatom oder einen $(C_1-C_4)$Alkylrest bedeutet;
A ein Wasserstoffatom oder einen $-N[(C_9-C_{12})$aliphatisch-Acyl$]$-$\beta$-D-2-deoxy-2-amino-glucopyranosylrest bedeutet;
B ein Wasserstoffatom oder eine N-Acetyl-$\beta$-D-2-deoxy-2-amino-glucopyranosylgruppe bedeutet;
M ein Wasserstoffatom oder eine $\alpha$-D-Mannopyranosylgruppe bedeutet,
mit der Maßgabe, daß B nur dann ein Wasserstoffatom bedeutet, wenn A und M gleichzeitig ein Wasserstoffatom bedeuten;
und den Additionssalzen davon.

3. Verfahren nach Anspruch 1, wobei $R^1$ ein Wasserstoffatom ist.

**4.** Verfahren nach Anspruch 1, wobei $R^1$ eine Methylgruppe ist.

**5.** Verfahren nach Anspruch 1, wobei
$R^1$ ein Wasserstoffatom oder einen $(C_1-C_3)$Alkylrest bedeutet,
$R^2$ einen Rest der Formel $[CHR^3(CR^4R^5)_mX]_p-(CH_2)_n-R^6$ bedeutet,
in der $R^3$ und $R^4$ unabhängig voneinander ein Wasserstoffatom oder einen $(C_1-C_4)$Alkylrest bedeuten;
$R^5$ ein Wasserstoffatom, einen $(C_1-C_4)$Alkylrest oder eine Hydroxylgruppe bedeutet;
$R^6$ ein Wasserstoffatom, einen $(C_1-C_3)$Alkylrest, einen Rest $OR^7$, $SR^7$ oder $NR^7R^8$ bedeutet;
$R^7$ und $R^8$ unabhängig voneinander ein Wasserstoffatom oder einen $(C_1-C_3)$Alkylrest bedeuten;
m 0 oder 1 ist, n eine ganze Zahl von 0 bis 3 ist, p eine ganze Zahl von 1 bis 3 ist;
X O, NH oder eine Bindung bedeutet, mit der Maßgabe, daß wenn X O oder NH ist, n nicht 0 ist, p 1 oder 2 ist und $R^5$ keine Hydroxylgruppe ist;
Y einen Rest

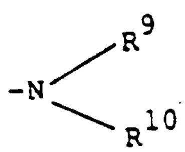

bedeutet, in der
$R^9$ ein Wasserstoffatom oder einen $(C_1-C_4)$Alkylrest bedeutet;
$R^{10}$ ein Wasserstoffatom, einen $(C_1-C_4)$Alkyl- oder Hydroxy$(C_2-C_4)$alkylrest oder einen Rest

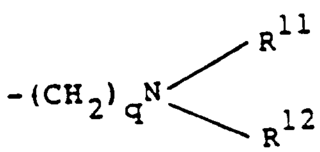

bedeutet,
in der q eine ganze Zahl von 2 bis 8 ist, und eines der Wasserstoffatome einer Methyleneinheit gegebenenfalls mit einem Substituenten aus der Gruppe Carboxy- und $(C_1-C_3)$Alkoxycarbonylrest substituiert ist;
$R^{11}$ ein Wasserstoffatom oder einen $(C_1-C_4)$Alkylrest bedeutet,
$R^{12}$ ein Wasserstoffatom, einen $(C_1-C_6)$Alkyl-, Hydroxy$(C_2-C_4)$alkyl-, $(C_1-C_3)$Alkoxy$(C_1-C_4)$alkyl-, Phenyl-$(C_1-C_4)$alkoxycarbonyl- oder $(C_1-C_4)$Alkyloxycarbonylrest bedeutet, oder
oder $R^9$ und $R^{10}$ zusammen mit dem benachbarten Stickstoffatom einen gesättigten 5-7-gliedrigen hetero-cyclischen Ring darstellen, der gegebenenfalls einen oder zwei $(C_1-C_4)$Alkylreste an den Ringkohlenstoffatomen tragen kann und weitere Heteroatome aus der Gruppe -O-, -S-und $-NR^{14}$-enthalten kann, wobei $R^{14}$ ein Wasserstoffatom oder ein $(C_1-C_4)$Alkylrest ist;
A ein Wasserstoffatom oder einen $-N[(C_9-C_{12})$aliphatisch-Acyl]-$\beta$-D-2-deoxy-2-amino-glucopyranosylrest bedeutet;
B ein Wasserstoffatom oder eine N-Acetyl-$\beta$-D-2-deoxy-2-amino-glucopyranosylgruppe bedeutet;
M ein Wasserstoffatom oder eine $\alpha$-D-Mannopyranosylgruppe bedeutet;
mit der Maßgabe, daß B nur dann ein Wasserstoffatom bedeutet, wenn A und M gleichzeitig ein Wasserstoffatom sind.

**6.** Verfahren nach Anspruch 1, wobei
$R^1$ ein Wasserstoffatom oder eine Methylgruppe bedeutet,
$R^2$ eine Gruppe gemäß der vorstehenden Definition ist, in der $R^3$ ein Wasserstoffatom oder eine Methylgruppe bedeutet, $R^4$ ein Wasserstoffatom oder eine Methylgruppe bedeutet, $R^5$ ein Wasserstoffatom oder eine Hydroxylgruppe bedeutet, m 0 oder 1 ist, n im Bereich von 0 bis 2 liegt, p 1 oder 2 ist, $R^6$ ein Wasserstoffatom oder einen Rest $OR^7$ oder $NR^7R^8$ bedeutet, und $R^7$ und $R^8$ unabhängig voneinander ein Wasserstoffatom oder eine Methylgruppe bedeuten,

Y einen Rest

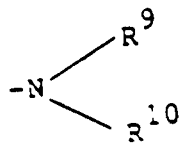

bedeutet, in der
$R^9$ ein Wasserstoffatom oder einen ($C_1$-$C_4$)Alkylrest bedeutet;
$R^{10}$ ein Wasserstoffatom, einen ($C_1$-$C_4$)Alkyl- oder Hydroxy($C_2$-$C_4$)alkylrest bedeutet oder einen Rest

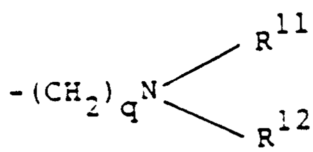

bedeutet,
in der q eine ganze Zahl von 2 bis 8 ist, und eines der Wasserstoffatome einer Methyleneinheit gegebenenfalls mit einem Rest aus der Gruppe Carboxy- und ($C_1$-$C_3$)-Alkoxycarbonylrest substituiert ist,
$R^{11}$ ein Wasserstoffatom oder einen ($C_1$-$C_4$)Alkylrest bedeutet,
$R^{12}$ ein Wasserstoffatom, einen ($C_1$-$C_6$)Alkyl-, Hydroxy($C_2$-$C_4$)alkyl-, Halogen($C_2$-$C_4$)alkyl-, ($C_1$-$C_3$)-Alkoxy($C_1$-$C_4$)alkyl-, Phenyl($C_1$-$C_4$)alkoxycarbonyl- oder ($C_1$-$C_4$)Alkyloxycarbonylrest bedeutet, oder
$R^{11}$ und $R^{12}$ zusammen mit dem angrenzenden Stickstoffatom einen gesättigten 5-7-gliedrigen heterocyclischen Ring darstellen, der gegebenenfalls ein oder zwei ($C_1$-$C_4$)-Alkylreste an den Ringkohlenstoffatomen tragen kann und ein weiteres Heteroatom aus der Gruppe -O-, -S- und -$NR^{13}$- enthalten kann, wobei $R^{13}$ ein Wasserstoffatom oder ein ($C_1$-$C_4$)Alkylrest ist,
oder $R^9$ und $R^{10}$ zusammen mit dem angrenzenden Stickstoffatom einen gesättigten 5-7-gliedrigen hetero-cyclischen Ring darstellen, der gegebenenfalls einen oder zwei ($C_1$-$C_4$)Alkylreste an den Ringkohlenstoffatomen tragen kann und ein weiteres Heteroatom aus der Gruppe -O-, -S- und -$NR^{14}$-enthalten kann, wobei $R^{14}$ ein Wasserstoffatom oder ein ($C_1$-$C_4$)Alkylrest ist,
A ein Wasserstoffatom oder einen -N(($C_9$-$C_{12}$)aliphatisch-Acyl]-β-D-2-deoxy-2-amino-glucopyranosylrest bedeutet,
B ein Wasserstoffatom oder eine N-Acetyl-β-D-2-deoxy-2-amino-glucopyranosylgruppe bedeutet,
M ein Wasserstoffatom oder eine α-D-Mannopyranosylgruppe bedeutet;
mit der Maßgabe, daß B nur dann ein Wasserstoffatom bedeutet, wenn A und M gleichzeitig ein Wasserstoffatom bedeuten.

7. Verfahren gemäß einem der Ansprüche 1 bis 6, wobei das Reduktionsmittel ein Alkalimetallborhydrid ist.

8. Verwendung eines Stoffes gemäß einem der Ansprüche 1 bis 6 zur Herstellung eines Arzneimittels zur Verwendung als antibakterielles Mittel.

## Revendications

### Revendications pour les Etats contractants suivants : AT, BE, CH, DE, FR, GB, IT, LI, LU, NL, SE

**1.** Amide du composé $N^{15}$-alkyl- ou $N^{15},N^{15}$-diakyl-téicoplanine ayant la formule I suivante

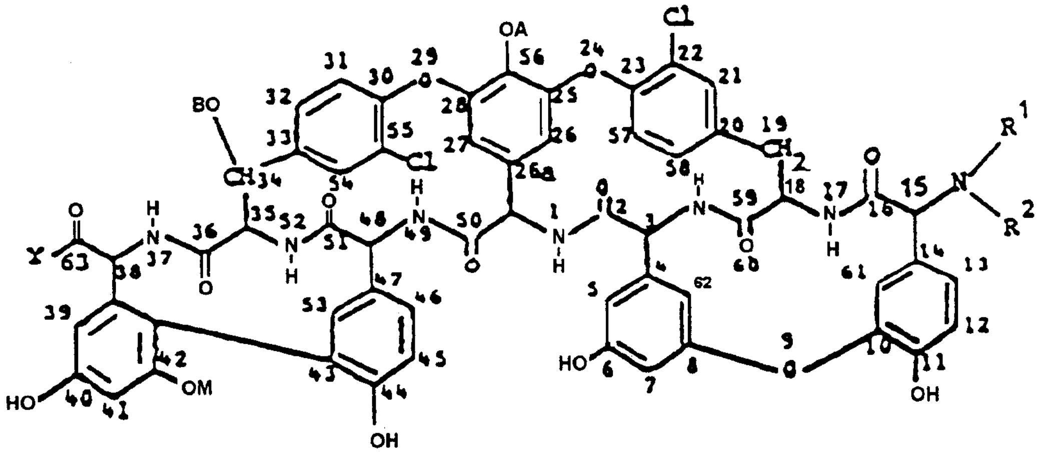

I

dans laquelle:

$R^1$ est un atome d'hydrogène ou un groupe alkyle en $C_1$ à $C_3$

$R^2$ représente un groupe $[CHR^3(CR^4R^5)_mX]_p$-$(CH_2)_n$-$R^6$

dans lequel

$R^3$ et $R^4$ représentent indépendamment un atome d'hydrogène ou un groupe alkyle en $C_1$ à $C_6$;

$R^5$ représente un atome d'hydrogène, un groupe alkyle en $C_1$ à $C_6$ ou OH;

$R^6$ représente un atome d'hydrogène, un groupe alkyle en $C_1$ à $C_3$, $COOR^7$, $OR^7$, $SR^7$, $NR^7R^8$ ou un atome d'halogène;

$R^7$ et $R^8$ représentent indépendamment un atome d'hydrogène ou un groupe alkyle en $C_1$ à $C_3$;

m vaut zéro ou 1, n est un nombre entier valant de zéro à 6, p est un nombre entier valant de 1 à 6;

X représente un atome O, un groupe NH ou une liaison à condition que lorsque X représente un atome O ou un groupe NH, alors n est différent de zéro, p vaut entre 1 et 3, et $R^5$ est différent de OH

Y représente un groupe

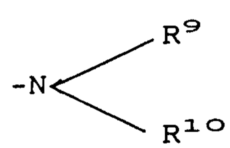

dans lequel:

$R^9$ représente un atome d'hydrogène ou un groupe alkyle en $C_1$ à $C_4$; un groupe hydroxyalkyle en $C_2$ à $C_4$, aminoalkyle en $C_2$ à $C_4$, alkyl(en $C_1$ à $C_3$)aminoalkyle(en $C_2$ à $C_4$);

$R^{10}$ représente un atome d'hydrogène, un groupe alkyle en $C_1$ à $C_6$, hydroxyalkyle en $C_2$ à $C_4$, halogénoalkyle en $C_2$ à $C_4$, carboxyalkyle en $C_1$ à $C_3$, carboxamidoalkyle en $C_1$ à $C_3$, alkyl-(en $C_1$ à $C_3$)carboxyalkyl(en $C_1$ à $C_3$)amino, alkoxy(en $C_1$ à $C_3$)carbonylalkyle(en $C_1$ à $C_3$), phénylalkoxy(en $C_1$ à $C_3$)carbonylalkyle(en $C_1$ à $C_3$), un groupe

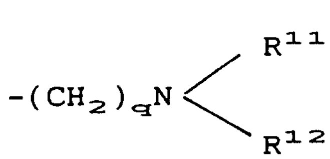

ou un groupe

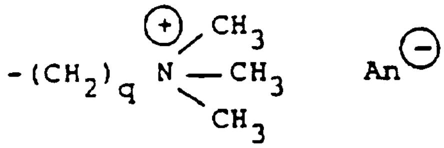

dans lequel q est un nombre entier valant de 2 à 8, et l'un des atomes d'hydrogène du motif méthylène est éventuellement substitué avec un substituant choisi parmi un groupe alkyle en $C_1$ à $C_3$, carboxy, alkoxycarbonyle en $C_1$ à $C_4$ et carboxamido et $An^{\ominus}$ représente un anion acceptable sur le plan pharmaceutique,

$R^{11}$ représente un atome d'hydrogène ou un groupe alkyle en $C_1$ à $C_4$, hydroxyalkyle en $C_2$ à $C_4$, halogénoalkyle en $C_2$ à $C_4$,

$R^{12}$ représente un atome d'hydrogène, un groupe alkyle en $C_1$ à $C_6$, hydroxyalkyle en $C_2$ à $C_4$, halogénoalkyle en $C_2$ à $C_4$, alkoxy(en $C_1$ à $C_3$)alkyle(en $C_1$ à $C_4$), phénylalkoxy(en $C_1$ à $C_4$)carbonyle, alkyloxy(en $C_1$ à $C_4$)carbonyle,

ou bien $R^{11}$ et $R^{12}$ pris ensemble avec l'atome d'azote adjacent représentent un noyau hétérocyclique saturé de 5 à 7 éléments qui peuvent éventuellement porter un à deux substituants alkyle en $C_1$ à $C_4$ sur les atomes de carbone du noyau et peuvent contenir un hétérogroupe supplémentaire choisi parmi -O-, -S-, et $-NR^{13}-$, dans lequel $R^{13}$ représente un atome d'hydrogène ou un groupe alkyle en $C_1$ à $C_4$,

ou bien $R^9$ et $R^{10}$ ensemble avec l'atome d'azote adjacent représentent un noyau hétérocyclique saturé de 5 à 7 éléments, qui peuvent éventuellement porter un à deux substituants alkyle en $C_1$ à $C_4$ sur les atomes de carbone du noyau et peuvent contenir un hétérogroupe supplémentaire choisi parmi -O-, -S-, et $-NR^{14}-$, dans lequel $R^{14}$ représente un atome d'hydrogène ou un groupe alkyle en $C_1$ à $C_4$,

A représente un atome d'hydrogène ou un groupe -N[acyle(en $C_9$ à $C_{12}$)aliphatique]-béta-D-2-désoxy-2-amino-glucopyranosyle,

B représente un atome d'hydrogène ou un groupe N-acétyl-béta-D-2-désoxy-2-amino-glucopyranosyle,

M représente un atome d'hydrogène ou un groupe alpha-D-mannopyranosyle;

à condition que B ne représente un atome d'hydrogène que lorsque A et M représentent simultanément un atome d'hydrogène et leurs sels d'addition.

**2.** Composé selon la revendication 1, dans lequel:

$R^1$ est un atome d'hydrogène ou un groupe alkyle en $C_1$ à $C_3$

$R^2$ représente un groupe $[CNR^3(CR^4R^5)_mX]_p-(CH_2)_n-R^6$

dans lequel:

$R^3$ et $R^4$ représentent indépendamment un atome d'hydrogène ou un groupe alkyle en $C_1$ à $C_6$;

$R^5$ représente un atome d'hydrogène, un groupe alkyle en $C_1$ à $C_6$ ou OH;

$R^6$ représente un atome d'hydrogène, un groupe alkyle en $C_1$ à $C_3$, $COOR^7$, $OR^7$, $SR^7$, $NR^7R^8$ ou un atome d'halogène;

$R^7$ et $R^8$ représentent indépendamment un atome d'hydrogène ou un groupe alkyle en $C_1$ à $C_3$;

m vaut zéro ou 1, n est un nombre entier valant de zéro à 6, p est un nombre entier valant de 1 à 6;

X représente un atome O, un groupe NH ou une liaison à condition que lorsque X représente un atome O ou un groupe NH, alors n est différent de zéro, p vaut entre 1 et 3, et

$R^5$ est différent de OH

Y représente un groupe

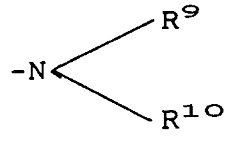

dans lequel:

$R^9$ est un atome d'hydrogène ou un groupe alkyle en $C_1$ à $C_4$;
$R^{10}$ représente un atome d'hydrogène, un groupe alkyle en $C_1$ à $C_6$, ydroxyalkyle en $C_2$ à $C_4$, halogénoalkyle en $C_2$ à $C_4$, ou un groupe

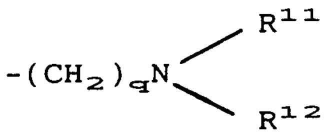

dans lequel q est un nombre entier valant de 2 à 8, et l'un des atomes d'hydrogène du motif méthylène est éventuellement substitué avec un substituant choisi parmi un groupe carboxy et alkoxycarbonyle en $C_1$ à $C_4$,

$R^{11}$ représente un atome d'hydrogène ou un groupe alkyle en $C_1$ à $C_4$,

$R^{12}$ représente un atome d'hydrogène, un groupe alkyle en $C_1$ à $C_6$, hydroxyalkyle en $C_2$ à $C_4$, halogénoalkyle en $C_2$ à $C_4$, alkoxy(en $C_1$ à $C_3$)alkyle(en $C_1$ à $C_4$), phénylalkoxy(en $C_1$ à $C_4$)-carbonyle, alkyloxy(en $C_1$ à $C_4$)carbonyle,

ou bien $R^{11}$ et $R^{12}$ pris ensemble avec l'atome d'azote adjacent représentent un noyau hétérocyclique saturé de 5 à 7 éléments qui peuvent éventuellement porter un à deux substituants alkyle en $C_1$ à $C_4$ sur les atomes de carbone du noyau et peuvent contenir un hétérogroupe supplémentaire choisi parmi -O-, -S-, et $-NR^{13}-$, dans lequel $R^{13}$ représente un atome d'hydrogène ou un groupe alkyle en $C_1$ à $C_4$,
ou bien $R^9$ et $R^{10}$ pris ensemble avec l'atome d'azote adjacent représentent un noyau hétérocyclique saturé de 5 à 7 éléments, qui peuvent éventuellement porter un à deux substituants alkyle en $C_1$ à $C_4$ sur les atomes de carbone du noyau et peuvent contenir un hétérogroupe supplémentaire choisi parmi -O-, -S-, et $-NR^{14}-$, dans lequel $R^{14}$ représente un atome d'hydrogène ou un groupe alkyle en $C_1$ à $C_4$,

A représente un atome d'hydrogène ou un groupe -N[acyle(en $C_9$ à $C_{12}$)aliphatique]-béta-D-2-désoxy-2-amino-glucopyranosyle,

B représente un atome d'hydrogène ou un groupe N-acétyl-béta-D-2-désoxy-2-amino-glucopyranosyle,

M représente un atome d'hydrogène ou un groupe alpha-D-mannopyranosyle;

à condition que B ne représente un atome d'hydrogène que lorsque A et M représentent simultanément un atome d'hydrogène et leurs sels d'addition.

**3.** Composé selon la revendication 1, dans lequel $R^1$ représente un atome d'hydrogène.

**4.** Composé selon la revendication 1, dans lequel $R^1$ représente un groupe méthyle.

**5.** Composé selon la revendication 1, dans lequel:

$R^1$ est un atome d'hydrogène ou un groupe alkyle en $C_1$ à $C_3$

$R^2$ représente un groupe $[CHR^3(CR^4R^5)_mX]_p-(CH_2)_n-R^6$

dans lequel:
$R^3$ et $R^4$ représentent indépendamment un atome d'hydrogène ou un groupe alkyle en $C_1$ à $C_6$;
$R^5$ représente un atome d'hydrogène, un groupe alkyle en $C_1$ à $C_6$ ou OH;
$R^6$ représente un atome d'hydrogène, un groupe alkyle en $C_1$ à $C_3$, $OR^7$, $SR^7$, ou $NR^7R^8$;
$R^7$ et $R^8$ représentent indépendamment un atome d'hydrogène ou un groupe alkyle en $C_1$ à $C_3$;
m vaut zéro ou 1, n est un nombre entier valant de zéro à 3, p est un nombre entier valant de 1 à 3;
X représente un atome O, un groupe NH ou une liaison à condition que lorsque X représente un atome O ou un groupe NH, alors n est différent de zéro, p vaut 1 ou 2, et $R^5$ est différent de OH,

Y représente un groupe

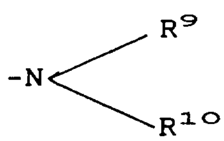

dans lequel:

$R^9$ représente un atome d'hydrogène ou un groupe alkyle en $C_1$ à $C_4$;

$R^{10}$ représente un atome d'hydrogène, un groupe alkyle en $C_1$ à $C_4$, hydroxyalkyle en $C_2$ à $C_4$ ou un groupe

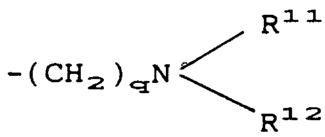

dans lequel q est un nombre entier valant de 2 à 8, et l'un des atomes d'hydrogène d'un motif méthylène est éventuellement substitué avec un substituant choisi parmi un groupe carboxy et alkoxycarbonyle en $C_1$ à $C_3$,

$R^{11}$ représente un atome d'hydrogène ou un groupe alkyle en $C_1$ à $C_4$,

$R^{12}$ représente un atome d'hydrogène, un groupe alkyle en $C_1$ à $C_6$, hydroxyalkyle en $C_2$ à $C_4$, alkoxy(en $C_1$ à $C_3$)alkyle(en $C_1$ à $C_4$), phénylalkoxy(en $C_1$ à $C_4$)carbonyle, alkyloxy(en $C_1$ à $C_4$)carbonyle,

ou bien $R^9$ et $R^{10}$ pris ensemble avec l'atome d'azote adjacent représentent un noyau hétérocyclique saturé de 5 à 7 éléments, qui peuvent éventuellement porter un à deux substituants alkyle en $C_1$ à $C_4$ sur les atomes de carbone du noyau et peuvent contenir un hétérogroupe supplémentaire choisi parmi -O-, -S-, et $-NR^{14}-$, dans lequel $R^{14}$ représente un atome d'hydrogène ou un groupe alkyle en $C_1$ à $C_4$,

A représente un atome d'hydrogène ou un groupe -N[acyle(en $C_9$ à $C_{12}$)aliphatique]-béta-D-2-désoxy-2-amino-glucopyranosyle,

B représente un atome d'hydrogène ou un groupe N-acétyl-béta-D-2-désoxy-2-amino-glucopyranosyle,

M représente un atome d'hydrogène ou un groupe alpha-D-mannopyranosyle;

à condition que B ne représente un atome d'hydrogène que lorsque A et M représentent simultanément un atome d'hydrogène et leurs sels d'addition.

**6.** Composé selon la revendication 1, dans lequel:

$R^1$ représente un atome d'hydrogène ou un groupe $CH_3$,

$R^2$ représente un groupe tel que défini dans lequel $R^3$ représente un atome d'hydrogène ou un groupe $CH_3$,

$R^4$ représente un atome d'hydrogéne ou un groupe $CH_3$;

$R^5$ représente un atome d'hydrogène ou un groupe OH;

m vaut zéro ou 1, n vaut de 0 à 2, p vaut 1 ou 2,

$R^6$ représente un atome d'hydrogène, $OR^7$ ou $NR^7R^8$ et

$R^7$ et $R^8$ représentent indépendamment un atome d'hydrogène ou un groupe $CH_3$

Y représente un groupe

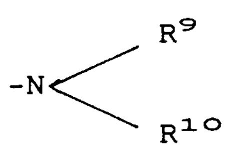

dans lequel:

$R^9$ représente un atome d'hydrogène ou un groupe alkyle en $C_1$ à $C_4$;

$R^{10}$ représente un atome d'hydrogène, un groupe alkyle en $C_1$ à $C_4$, hydroxyalkyle en $C_2$ à $C_4$ ou un groupe

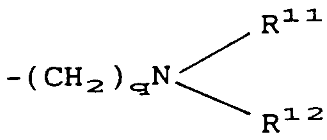

dans lequel q est un nombre entier valant de 2 à 8, et l'un des atomes d'hydrogène d'un motif méthylène est éventuellement substitué avec un substituant choisi parmi un groupe carboxy, et alkoxy-(en $C_1$ à $C_3$)carbonyle,

$R^{11}$ représente un atome d'hydrogène ou un groupe alkyle en $C_1$ à $C_4$,

$R^{12}$ représente un atome d'hydrogène, un groupe alkyle en $C_1$ à $C_6$, hydroxyalkyle en $C_2$ à $C_4$, halogénoalkyle en $C_2$ à $C_4$, alkoxy(en $C_1$ à $C_3$)alkyle(en $C_1$ à $C_4$), phénylalkoxy(en $C_1$ à $C_4$)-carbonyle, alkyloxy(en $C_1$ à $C_4$)carbonyle,

ou bien $R^{11}$ et $R^{12}$ pris ensemble avec l'atome d'azote adjacent représentent un noyau hétérocyclique saturé de 5 à 7 éléments, qui peuvent éventuellement porter un à deux substituants alkyle en $C_1$ à $C_4$ sur les atomes de carbone du noyau et peuvent contenir un hétérogroupe supplémentaire choisi parmi -O-, -S-, et $-NR^{13}-$, dans lequel $R^{13}$ représente un atome d'hydrogène ou un groupe alkyle en $C_1$ à $C_4$,

ou $R^9$ et $R^{10}$ pris ensemble avec l'atome d'azote adjacent représentent un noyau hétérocyclique saturé de 5 à 7 éléments, qui peuvent éventuellement porter un à deux substituants alkyle en $C_1$ à $C_4$ sur les atomes de carbone du noyau et peuvent contenir un hétérogroupe supplémentaire choisi parmi -O-, -S-, et $-NR^{14}-$, dans lequel $R^{14}$ représente un atome d'hydrogène ou un groupe alkyle en $C_1$ à $C_4$,

A représente un atome d'hydrogène ou un groupe -N[acyle(en $C_9$ à $C_{12}$)aliphatique]-béta-D-2-désoxy-2-amino-glucopyranosyle,

B représente un atome d'hydrogéne ou un groupe N-acétyl-béta-D-2-désoxy-2-amino-glucopyranosyle,

M représente un atome d'hydrogène ou un groupe alpha-D-mannopyranosyle;

à condition que B ne représente un atome d'hydrogène que lorsque A et M représentent simultanément un atome d'hydrogène et leurs sels d'addition.

**7.** Procédé pour préparer un composé de la revendication 1, qui comprend la réaction d'un composé de formule II

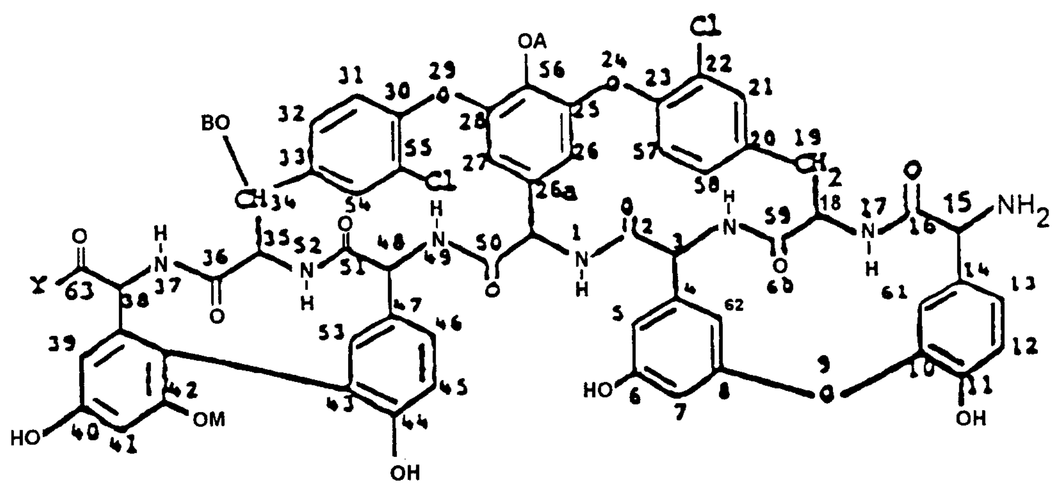

II

dans laquelle:

Y représente un groupe

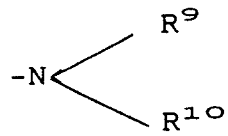

dans lequel:

$R^9$ représente un atome d'hydrogène ou un groupe alkyle en $C_1$ à $C_4$; hydroxyalkyle en $C_2$ à $C_4$, aminoalkyle en $C_2$ à $C_4$, alkylamino en $C_1$ à $C_3$ alkyle en $C_2$ à $C_4$;

$R^{10}$ représente un atome d'hydrogène, un groupe alkyle en $C_1$ à $C_6$, hydroxyalkyle en $C_2$ à $C_4$, halogénoalkyle en $C_2$ à $C_4$, carboxyalkyle en $C_1$ à $C_3$, carboxamidoalkyle en $C_1$ à $C_3$, alkyl-(en $C_1$ à $C_3$)carboxyalkyl(en $C_1$ à $C_3$)amino, alkoxy(en $C_1$ à $C_3$)carbonylalkyle(en $C_1$ à $C_3$), phénylalkoxy(en $C_1$ à $C_3$)carbonylalkyle(en $C_1$ à $C_3$), un groupe

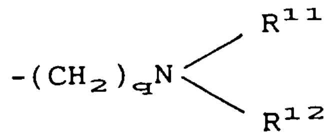

ou un groupe

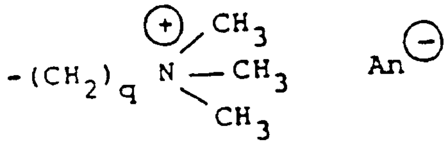

dans lequel q est un nombre entier valant de 2 à 8, et l'un des atomes d'hydrogène du motif méthylène est éventuellement substitué avec un substituant choisi parmi un groupe alkyle en $C_1$ à $C_3$, carboxy, alkoxy(en $C_1$ à $C_4$)carbonyle, carboxamido et $An^{\ominus}$ représente un anion acceptable sur le plan pharmaceutique,

$R^{11}$ représente un atome d'hydrogène ou un groupe alkyle en $C_1$ à $C_4$, hydroxyalkyle en $C_2$ à $C_4$, halogénoalkyle en $C_2$ à $C_4$,

$R^{12}$ représente un atome d'hydrogène, un groupe alkyle en $C_1$ à $C_6$, hydroxyalkyle en $C_2$ à $C_4$, halogénoalkyle en $C_2$ à $C_4$, alkoxy(en $C_1$ à $C_3$)alkyle(en $C_1$ à $C_4$), phénylalkoxy(en $C_1$ à $C_4$)-carbonyle, alkyloxy(en $C_1$ à $C_4$)carbonyle,

ou bien $R^{11}$ et $R^{12}$ pris ensemble avec l'atome d'azote adjacent représentent un noyau hétérocyclique saturé de 5 à 7 éléments, qui peuvent éventuellement porter un à deux substituants alkyle en $C_1$ à $C_4$ sur les atomes de carbone du noyau et peuvent contenir un hétérogroupe supplémentaire choisi parmi -O-, -S-, et $-NR^{13}-$, dans lequel $R^{13}$ représente un atome d'hydrogène ou un groupe alkyle en $C_1$ à $C_4$,

ou bien $R^9$ et $R^{10}$ pris ensemble avec l'atome d'azote adjacent représentent un noyau hétérocyclique saturé de 5 à 7 éléments, qui peuvent éventuellement porter un à deux substituants alkyle en $C_1$ à $C_4$ sur les atomes de carbone du noyau et peuvent contenir un hétérogroupe supplémentaire choisi parmi -O-, -S-, et $-NR^{14}-$, dans lequel $R^{14}$ représente un atome d'hydrogène ou un groupe alkyle en $C_1$ à $C_4$,

A représente un atome d'hydrogène ou un groupe -N[acyle(en $C_9$ à $C_{12}$)aliphatique]-béta-D-2-désoxy-2-amino-glucopyranosyle,

B représente un atome d'hydrogène ou un groupe N-acétyl-béta-D-2-désoxy-2-amino-glucopyranosyle,

M représente un atome d'hydrogène ou un groupe alpha-D-mannopyranosyle;

à condition que B ne représente un atome d'hydrogène que lorsque A et M représentent simultanément un atome d'hydrogène, un de leurs sels, ou un de leurs mélanges dans n'importe quelles proportions, avec

a) un halogénure d'alkyle de formule $R^1$ (halogéno) ou $R^2$ (halogéno), dans lequel:

$R^1$ est un atome d'hydrogéne ou un groupe alkyle en $C_1$ à $C_3$

$R^2$ représente un groupe $[CHR^3(CR^4R^5)_mX]_p-(CH_2)_n-R^6$

dans lequel:

$R^3$ et $R^4$ représentent indépendamment un atome d'hydrogène ou un groupe alkyle en $C_1$ à $C_6$;

$R^5$ représente un atome d'hydrogène, un groupe alkyle en $C_1$ à $C_6$ ou OH;

$R^6$ représente un atome d'hydrogène, un groupe alkyle en $C_1$ à $C_3$, $COOR^7$, $OR^7$, $SR^7$, $NR^7R^8$ ou un atome d'halogène;

$R^7$ et $R^8$ représentent indépendamment un atome d'hydrogène ou un groupe alkyle en $C_1$ à $C_3$;

m vaut zéro ou 1, n est un nombre entier valant de zéro à 6, p est un nombre entier valant de 1 à 6;

X représente un atome O, un groupe NH ou une liaison à condition que lorsque X représente un atome O ou un groupe NH, alors n est différent de zéro, p vaut entre 1 et 3, et $R^5$ est différent de OH et (halogéno) représente un atome de chlore ou de brome en présence d'une base et éventuellement d'un solvant organique,

b) en variante, avec un excès molaire d'un composé carbonyle d'au moins 20 fois par rapport à la matière de départ de téicoplanine, dans un solvant aprotique polaire organique avec un agent réducteur.

**8.** Procédé selon la revendication 7, dans lequel le composé carbonyle possède la même squelette de substituant $R^2$ (ou $R^1$) et dont le groupe oxo est sur l'atome de carbone qui doit lier le groupe amino $N^{15}$ de la fraction téicoplanine.

**9.** Procédé selon la revendication 7, dans lequel l'agent réducteur est un borohydrure de métal alcalin.

**10.** Composé selon la revendication 1 pour une utilisation en tant que médicament.

**11.** Utilisation d'une substance de la revendication 1 pour la fabrication d'un médicament en vue d'une utilisation en tant que produit antibactérien.

**12.** Composition pharmaceutique comprenant un composé delon la revendication 1 en mélange avec un porteur acceptable sur le plan pharmaceutique.

**Revendications pour les Etats contractants suivants : GR, ES**

**1.** Procédé pour préparer un amide du composé $N^{15}$-alkyl- ou $N^{15}$,$N^{15}$-diakyl téicoplanine ayant la formule I suivante

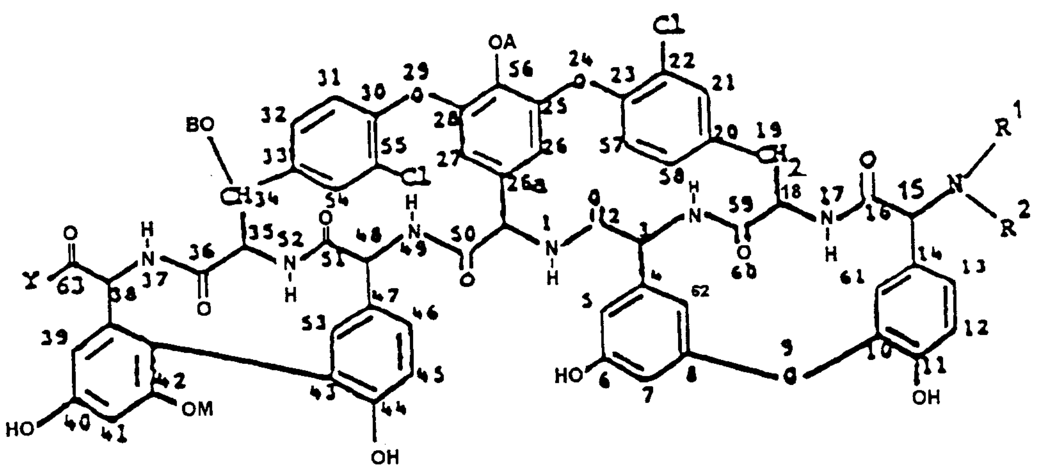

I

dans laquelle:

$R^1$ est un atome d'hydrogène ou un groupe alkyle en $C_1$ à $C_3$

$R^2$ représente un groupe $[CHR^3(CR^4R^5)_mX]_p-(CH_2)_n-R^6$

dans laquelle

$R^3$ et $R^4$ représentent indépendamment un atome d'hydrogène ou un groupe alkyle en $C_1$ à $C_6$;

$R^5$ représente un atome d'hydrogène, un groupe alkyle en $C_1$ à $C_6$ ou OH;

$R^6$ représente un atome d'hydrogène, un groupe alkyle en $C_1$ à $C_3$, $COOR^7$, $OR^7$, $SR^7$, $NR^7R^8$ ou un atome d'halogène;
$R^7$ et $R^8$ représentent indépendamment un atome d'hydrogène ou un groupe alkyle en $C_1$ à $C_3$;
m vaut zéro ou 1, n est un nombre entier valant de zéro à 6, p est un nombre entier valant de 1 à 6;
X représente un atome O, un groupe NH ou une liaison à condition que lorsque X représente un atome O ou un groupe NH, alors n est différent de zéro, p vaut entre 1 et 3, et $R^5$ est différent de OH

Y représente un groupe

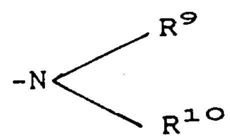

dans lequel:

$R^9$ représente un atome d'hydrogène ou un groupe alkyle en $C_1$ à $C_4$; hydroxyalkyle en $C_2$ à $C_4$, aminoalkyle en $C_2$ à $C_4$, alkyl(en $C_1$ à $C_3$)aminoalkyle(en $C_2$ à $C_4$);

$R^{10}$ représente un atome d'hydrogène, un groupe alkyle en $C_1$ à $C_6$, hydroxyalkyle en $C_2$ à $C_4$, halogénoalkyle en $C_2$ à $C_4$, carboxyalkyle en $C_1$ à $C_3$, carboxamidoalkyle en $C_1$ à $C_3$, alkyl-(en $C_1$ à $C_3$)carboxyalkyl(en $C_1$ à $C_3$)amino, alkoxy(en $C_1$ à $C_3$)carbonylalkyle(en $C_1$ à $C_3$), phénylalkoxy(en $C_1$ à $C_3$)carbonylalkyle(en $C_1$ à $C_3$), un groupe

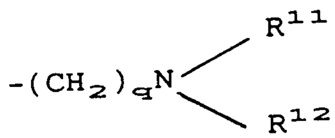

ou un groupe

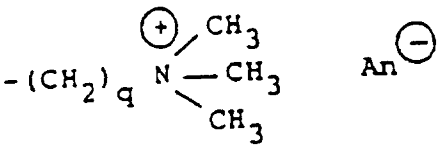

dans lequel q est un nombre entier valant de 2 à 8, et l'un des atomes d'hydrogène du motif méthylène est éventuellement substitué avec un substituant choisi parmi un groupe alkyle en $C_1$ à $C_3$, carboxy, alkoxycarbonyle en $C_1$ à $C_4$ et carboxamido et $An^{\ominus}$ représente un anion acceptable sur le plan pharmaceutique,

$R^{11}$ représente un atome d'hydrogène ou un groupe alkyle en $C_1$ à $C_4$, hydroxyalkyle en $C_2$ à $C_4$, halogénoalkyle en $C_2$ à $C_4$,

$R^{12}$ représente un atome d'hydrogène, un groupe alkyle en $C_1$ à $C_6$, hydroxyalkyle en $C_2$ à $C_4$, halogénoalkyle en $C_2$ à $C_4$, alkoxy(en $C_1$ à $C_3$)alkyle(en $C_1$ a $C_4$), phénylalkoxy(en $C_1$ à $C_4$)carbonyle, alkyloxy(en $C_1$ à $C_4$)carbonyle,

ou bien $R^{11}$ et $R^{12}$ pris ensemble avec l'atome d'azote adjacent représentent un noyau hétérocyclique saturé de 5 à 7 éléments, qui peuvent éventuellement porter un à deux substituants alkyle en $C_1$ à $C_4$ sur les atomes de carbone du noyau et peuvent contenir un hétérogroupe supplémentaire choisi parmi -O-, -S-, et $-NR^{13}-$, dans lequel $R^{13}$ représente un atome d'hydrogène ou un groupe alkyle en $C_1$ à $C_4$,

ou bien $R^9$ et $R^{10}$ pris ensemble avec l'atome d'azote adjacent représentent un noyau hétérocyclique saturé de 5 à 7 éléments, qui peuvent éventuellement porter un à deux substituants alkyle en $C_1$ à $C_4$ sur les atomes de carbone du noyau et peuvent contenir un hétérogroupe supplémentaire choisi parmi -O-, -S-, et $-NR^{14}-$, dans lequel $R^{14}$ représente un atome d'hydrogène ou un groupe alkyle en $C_1$ à $C_4$,

A représente un atome d'hydrogène ou un groupe -N[acyle(en $C_9$ à $C_{12}$)aliphatique]-béta-D-2-désoxy-2-amino-glucopyranosyle,

B représente un atome d'hydrogène ou un groupe N-acétyl-béta-D-2-désoxy-2-amino-glucopyranosyle,
M représente un atome d'hydrogène ou un groupe alpha-D-mannopyranosyle;
à condition que B ne représente un atome d'hydrogène que lorsque A et M représentent simultanément un atome d'hydrogène et leurs sels d'addition,
qui comprend la réaction d'un composé de formule II

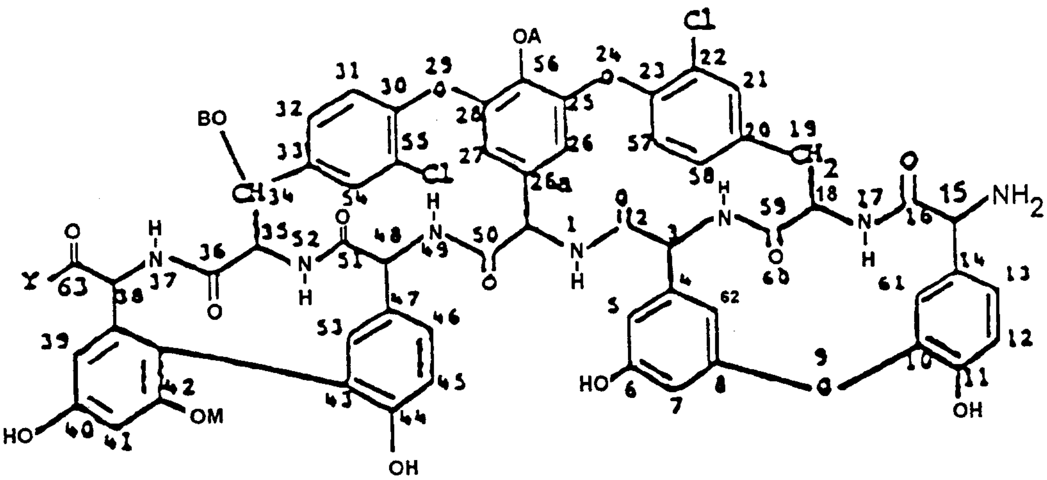

II

dans laquelle: Y, A, B et M sont définis tels que ci-dessus avec:
a) un halogénure alkyle de formule $R^1$ (hologéno) ou $R^2$ (halogéno), dans lequel:
$R^1$ est un atome d'hydrogène ou un groupe alkyle en $C_1$ à $C_3$
$R^2$ représente un groupe $[CHR^3(CR^4R^5)_mX]_p$-$(CH_2)_n$-$R^6$
dans lequel:
$R^3$ et $R^4$ représentent indépendamment un atome d'hydrogène ou un groupe alkyle en $C_1$ à $C_6$;
$R^5$ représente un atome d'hydrogène, un groupe alkyle en $C_1$ à $C_6$ ou OH;
$R^6$ représente un atome d'hydrogène, un groupe alkyle en $C_1$ à $C_3$, $COOR^7$, $OR^7$, $SR^7$, $NR^7R^8$ ou un atome d'halogène;
$R^7$ et $R^8$ représentent indépendamment un atome d'hydrogène ou un groupe alkyle en $C_1$ à $C_3$;
m vaut zéro ou 1, n est un nombre entier valant de zéro à 6, p est un nombre entier valant de 1 à 6;
X représente un atome O, un groupe NH ou une liaison à condition que lorsque X représente un atome O ou un groupe NH, alors n est différent de zéro, p vaut entre 1 et 3, et $R^5$ est différent de OH
et (halogéno) représente un atome de chlore ou de brome, en présence d'une base et éventuellement d'un solvant organique,
b) en variante, avec un excès molaire d'un composé carbonyle d'au moins 20 fois par rapport à la matière de départ de téicoplanine, dans un solvant aprotique polaire organique avec un agent de réduction, ledit composé carbonyle ayant le même squelette de carbone que le substituant souhaité $R^2$ (ou $R^1$) et dont le groupe oxo est sur l'atome de carbone qui doit lier le groupe amino $N^{15}$ de la partie de la téicoplanine.

2. Procédé selon la revendication 1 pour préparer un composé de formule I dans lequel:
$R^1$ est un atome d'hydrogène ou un groupe alkyle en $C_1$ à $C_3$
$R^2$ représente un groupe $[CHR^3(CR^4R^5)_mX]_p$-$(CH_2)_n$-$R^6$
dans lequel:
$R^3$ et $R^4$ représentent indépendamment un atome d'hydrogène ou un groupe alkyle en $C_1$ à $C_6$;
$R^5$ représente un atome d'hydrogène, un groupe alkyle en $C_1$ à $C_6$ ou OH;
$R^6$ représente un atome d'hydrogène, un groupe alkyle en $C_1$ à $C_3$, $COOR^7$, $OR^7$, $SR^7$, $NR^7R^8$ ou un atome d'halogène;
$R^7$ et $R^8$ représentent indépendamment un atome d'hydrogène ou un groupe alkyle en $C_1$ à $C_3$;
m vaut zéro ou 1, n est un nombre entier valant de zéro à 6,
p est un nombre entier valant de 1 à 6;

X représente un atome O, un groupe NH ou une liaison à condition que lorsque X représente un atome O ou un groupe NH, alors n est différent de zéro, p vaut entre 1 et 3, et $R^5$ est différent de OH

Y représente un groupe

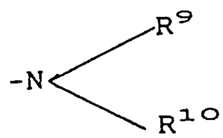

dans lequel:

$R^9$ est un atome d'hydrogène ou un groupe alkyle en $C_1$ à $C_4$;

$R^{10}$ représente un atome d'hydrogène, un groupe alkyle en $C_1$ à $C_6$, hydroxyalkyle en $C_2$ à $C_4$, halogénoalkyle en $C_2$ à $C_4$, ou un groupe

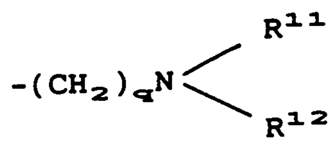

dans lequel q est un nombre entier valant de 2 à 8, et l'un des atomes d'hydrogène du motif méthylène est éventuellement substitué avec un substituant choisi parmi un groupe carboxy, alkoxy(en $C_1$ à $C_4$)carbonyle,

$R^{11}$ représente un atome d'hydrogène ou un groupe alkyle en $C_1$ à $C_4$,

$R^{12}$ représente un atome d'hydrogène, un groupe alkyle en $C_1$ à $C_6$, hydroxyalkyle en $C_2$ à $C_4$, halogénoalkyle en $C_2$ à $C_4$, alkoxy(en $C_1$ à $C_3$)alkyle(en $C_1$ à $C_4$), phénylalkoxy(en $C_1$ à $C_4$)-carbonyle, alkyloxy(en $C_1$ à $C_4$)carbonyle,

ou bien $R^{11}$ et $R^{12}$ pris ensemble avec l'atome d'azote adjacent représentent un noyau hétérocyclique saturé de 5 à 7 éléments, qui peuvent éventuellement porter un à deux substituants alkyle en $C_1$ à $C_4$ sur les atomes de carbone du noyau et peuvent contenir un hétérogroupe supplémentaire choisi parmi -O-, -S-, et -$NR^{13}$-, dans lequel $R^{13}$ représente un atome d'hydrogène ou un groupe alkyle en $C_1$ à $C_4$,

ou bien $R^9$ et $R^{10}$ pris ensemble avec l'atome d'azote adjacent représentent un noyau hétérocyclique saturé de 5 à 7 éléments, qui peuvent éventuellement porter un à deux substituants alkyle en $C_1$ à $C_4$ sur les atomes de carbone du noyau et peuvent contenir un hétérogroupe supplémentaire choisi parmi -O-, -S-, et -$NR^{14}$-, dans lequel $R^{14}$ représente un atome d'hydrogène ou un groupe alkyle en $C_1$ à $C_4$,

A représente un atome d'hydrogène ou un groupe -N[acyle(en $C_9$ à $C_{12}$)aliphatique]-béta-D-2-désoxy-2-amino-glucopyranosyle,

B représente un atome d'hydrogène ou un groupe N-acétyl-béta-D-2-désoxy-2-amino-glucopyranosyle,

M représente un atome d'hydrogène ou un groupe alpha-D-mannopyranosyle;

à condition que B ne représente un atome d'hydrogène que lorsque A et M représentent simultanément un atome d'hydrogène et leurs sels d'addition.

**3.** Procédé selon la revendication 1, dans lequel $R^1$ représente un atome d'hydrogène.

**4.** Procédé selon la revendication 1, dans lequel $R^1$ représente un groupe méthyle.

**5.** Procédé selon la revendication 1, dans lequel:

$R^1$ est un atome d'hydrogène ou un groupe alkyle en $C_1$ à $C_3$

$R^2$ représente un groupe $[CHR^3(CR^4R^5)_mX]_p\text{-}(CH_2)_n\text{-}R^6$

dans lequel:

$R^3$ et $R^4$ représentent indépendamment un atome d'hydrogène ou un groupe alkyle en $C_1$ à $C_4$;

$R^5$ représente un atome d'hydrogène, un groupe alkyle en $C_1$ à $C_4$ ou OH;

$R^6$ représente un atome d'hydrogène, un groupe alkyle en $C_1$ à $C_3$, $OR^7$, $SR^7$, ou $NR^7R^8$;

$R^7$ et $R^8$ représentent indépendamment un atome d'hydrogène ou un groupe alkyle en $C_1$ à $C_3$;

m vaut zéro ou 1, n est un nombre entier valant de zéro à 3, p est un nombre entier valant de 1 à 3; X représente un atome O, un groupe NH ou une liaison à condition que lorsque X représente un atome O ou un groupe NH, alors n est différent de zéro, p vaut 1 ou 2, et $R^5$ est différent de OH,

Y représente un groupe

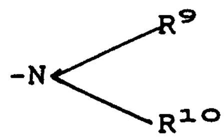

dans lequel:

$R^9$ représente un atome d'hydrogène ou un groupe alkyle en $C_1$ à $C_4$;

$R^{10}$ représente un atome d'hydrogène, un groupe alkyle en $C_1$ à $C_4$, hydroxyalkyle en $C_2$ à $C_4$ ou un groupe

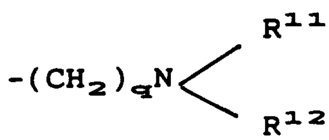

dans lequel q est un nombre entier valant de 2 à 8, et l'un des atomes d'hydrogène d'un motif méthylène est éventuellement substitué avec un substituant choisi parmi un groupe carboxy et alkoxy(en $C_1$ à $C_3$)carbonyle,

$R^{11}$ représente un atome d'hydrogène ou un groupe alkyle en $C_1$ à $C_4$,

$R^{12}$ représente un atome d'hydrogène, un groupe alkyle en $C_1$ à $C_6$, hydroxyalkyle en $C_2$ à $C_4$, halogénoalkyle en $C_2$ à $C_4$, alkoxy(en $C_1$ à $C_3$)alkyle(en $C_1$ à $C_4$), phénylalkoxy(en $C_1$ à $C_4$)-carbonyle, alkyloxy(en $C_1$ à $C_4$)carbonyle,

ou bien $R^9$ et $R^{10}$ pris ensemble avec l'atome d'azote adjacent représentent un noyau hétérocyclique saturé de 5 à 7 éléments, qui peuvent éventuellement porter un à deux substituants alkyle en $C_1$ à $C_4$ sur les atomes de carbone du noyau et peuvent contenir un hétérogroupe supplémentaire choisi parmi -O-, -S-, et $-NR^{14}-$, dans lequel $R^{14}$ représente un atome d'hydrogène ou un groupe alkyle en $C_1$ à $C_4$,

A représente un atome d'hydrogène ou un groupe -N[acyle(en $C_9$ à $C_{12}$)aliphatique]-béta-D-2-désoxy-2-amino-glucopyranosyle,

B représente un atome d'hydrogène ou un groupe N-acétyl-béta-D-2-désoxy-2-amino-glucopyranosyle,

M représente un atome d'hydrogène ou un groupe alpha-D-mannopyranosyle;

à condition que B ne représente un atome d'hydrogène que lorsque A et M représentent simultanément un atome d'hydrogène et leurs sels d'addition.

**6.** Procédé selon la revendication 1, dans lequel:

$R^1$ représente un atome d'hydrogène ou un groupe $CH_3$,

$R^2$ représente un groupe tel que défini dans lequel $R^3$ représente un atome d'hydrogène ou un groupe $CH_3$,

$R^4$ représente un atome d'hydrogène ou un groupe $CH_3$;

$R^5$ représente un atome d'hydrogène ou un groupe OH;

m vaut zéro ou 1, n vaut de 0 à 2, p vaut 1 ou 2,

$R^6$ représente un atome d'hydrogène, $OR^7$ ou $NR^7R^8$ et

$R^7$ et $R^8$ représentent indépendamment un atome d'hydrogène ou un groupe $CH_3$

Y représente un groupe

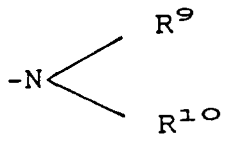

dans lequel:

$R^9$ représente un atome d'hydrogène ou un groupe alkyle en $C_1$ à $C_4$;

$R^{10}$ représente un atome d'hydrogène, un groupe alkyle en $C_1$ à $C_4$, hydroxyalkyle en $C_2$ à $C_4$ ou un groupe

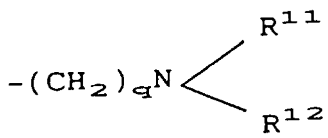

dans lequel q est un nombre entier valant de 2 à 8, et l'un des atomes d'hydrogène d'un motif méthylène est éventuellement substitué avec un substituant choisi parmi un groupe carboxy, et alkoxycarbonyle en $C_1$ à $C_3$,

$R^{11}$ représente un atome d'hydrogène ou un groupe alkyle en $C_1$ à $C_4$,

$R^{12}$ représente un atome d'hydrogène, un groupe alkyle en $C_1$ à $C_6$, hydroxyalkyle en $C_2$ à $C_4$, halogénoalkyle en $C_2$ à $C_4$, alkoxy(en $C_1$ à $C_3$)alkyle(en $C_1$ à $C_4$), phénylalkoxy(en $C_1$ à $C_4$)-carbonyle, alkyloxy(en $C_1$ à $C_4$)carbonyle,

ou bien $R^{11}$ et $R^{12}$ pris ensemble avec l'atome d'azote adjacent représentent un noyau hétérocyclique saturé de 5 à 7 éléments qui peuvent éventuellement porter un à deux substituants alkyle en $C_1$ à $C_4$ sur les atomes de carbone du noyau et peuvent contenir un hétérogroupe supplémentaire choisi parmi -O-, -S-, et $-NR^{13}-$, dans lequel $R^{13}$ représente un atome d'hydrogène ou un groupe alkyle en $C_1$ à $C_4$,

ou bien $R^9$ et $R^{10}$ pris ensemble avec l'atome d'azote adjacent représentent un noyau hétérocyclique saturé de 5 à 7 éléments, qui peuvent éventuellement porter un à deux substituants alkyle en $C_1$ à $C_4$ sur les atomes de carbone du noyau et peuvent contenir un hétérogroupe supplémentaire choisi parmi -O-, -S-, et $-NR^{14}-$, dans lequel $R^{14}$ représente un atome d'hydrogène ou un groupe alkyle en $C_1$ à $C_4$,

A représente un atome d'hydrogène ou un groupe -N[acyle(en $C_9$ à $C_{12}$)aliphatique]-béta-D-2-désoxy-2-amino-glucopyranosyle,

B représente un atome d'hydrogène ou un groupe N-acétyl-béta-D-2-désoxy-2-amino-glucopyranosyle,

M représente un atome d'hydrogène ou un groupe alpha-D-mannopyranosyle;

à condition que B ne représente un atome d'hydrogène que lorsque A et M représentent simultanément un atome d'hydrogène et leurs sels d'addition.

**7.** Procédé selon l'une quelconque des revendications 1 à 8, dans lequel l'agent de réduction est un borohydrure de métal alcalin.

**8.** Utilisation d'un composé selon l'une quelconque des revendications 1 à 6, pour fabriquer un médicament en vue d'une utilisation en tant que produit antibactérien.